(19) Europäisches Patentamt
European Patent Office
Office européen des brevets

(11) EP 4 733 307 A1

(12) EUROPEAN PATENT APPLICATION
published in accordance with Art. 153(4) EPC

(43) Date of publication:
**29.04.2026 Bulletin 2026/18**

(21) Application number: **24872534.3**

(22) Date of filing: **27.09.2024**

(51) International Patent Classification (IPC):
***C07K 1/02*** *(2006.01)* ***C07K 1/06*** *(2006.01)*
***C07K 1/10*** *(2006.01)*

(52) Cooperative Patent Classification (CPC):
**C07K 1/02; C07K 1/06; C07K 1/10**

(86) International application number:
**PCT/JP2024/034729**

(87) International publication number:
**WO 2025/070753 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.09.2023 JP 2023168751**

(71) Applicant: **CHUGAI SEIYAKU KABUSHIKI KAISHA**
**Tokyo 115-8543 (JP)**

(72) Inventor: **KOMIYA Shio**
**Tokyo 115-8543 (JP)**

(74) Representative: **Vossius & Partner**
**Patentanwälte Rechtsanwälte mbB**
**Siebertstraße 3**
**81675 München (DE)**

# (54) METHOD FOR PRODUCING PEPTIDE COMPOUND USING MIXED ANHYDRIDE PROCEDURE

(57) The present invention provides, as a method for synthesizing a peptide compound with high diastereoselectivity and in high yield even in the cases of fragment coupling, a method for producing a peptide compound or a salt thereof, comprising a step (linking step) of linking an amino group of a first amino acid or peptide and a carboxy group of a second amino acid or peptide with an amide bond in a two-layer solvent system containing water and one or more organic solvents that are not miscible with water.

EP 4 733 307 A1

## Description

[Technical Field]

The present invention relates to a method for producing a peptide compound using a mixed acid anhydride method.

[Background Art]

Recently, it has been found that the metabolic stability and membrane permeability of the peptide is improved by using non-natural amino acids such as cyclic peptides (Non Patent Literatures 1 and 2). Especially, among them, cyclic peptide compounds containing N-substituted amino acids have been suggested to have drug-likeness such as metabolic stability and membrane permeability, and to be useful for the creation of inhibitors of protein-protein interactions (Patent Literature 1, Non Patent Literature 3).

As peptide compounds have attracted attention as pharmaceuticals, the methods for producing peptide compounds have also been viewed as important (Non Patent Literature 4). Synthesis of peptide compounds is generally carried out by extending the peptide chain by repeating the step of linking the carboxy group at the C-terminus of an amino acid to the amino group at the N-terminus of another amino acid or peptide. An example of the known methods for extending the peptide chain is a method using EDC as a condensing agent in a two-layer solvent of isopropyl acetate and water (Non Patent Literature 5). Other examples of the known methods for extending the peptide chain include a method using HATU as a condensing agent, a method using T3P as a condensing agent, a method using acid chloride, and a method using an acyl halide such as pivaloyl chloride to extend by way of a mixed acid anhydride.

In the method by way of a mixed acid anhydride, the extension of the peptide chain is carried out by reacting the mixed acid anhydride obtained by activating a carboxy group at the C-terminus of a reaction substrate with an acyl halide, with an amine at the N-terminus of another reaction substrate. Examples of the known method of extending a peptide chain by way of a mixed acid anhydride include a method of reacting an unprotected amino acid with a substrate temporarily protected by silylation (Non Patent Literature 6), and a method of synthesizing a two-residue peptide from an amino acid by going through a mixed acid anhydride using pivaloyl chloride in a two-layer solvent of water and ethyl acetate (Non Patent Literature 7). All of these methods are a method of subjecting an amino acid to an amide bond formation reaction sequentially by one residue at a time.

Another known method of extending the peptide chain is a method called fragment coupling, which subjects peptide fragments each consisting of a plurality of amino acid residues to an amide bond formation reaction to each other. The fragment coupling method has the advantage of the high total yield of the final product. The greater the number of amino acid residues in the desired peptide compound, the better the advantage. However, under conventional amide bond formation conditions, the increase of impurities due to the racemization of the $\alpha$ position of the amino acid residue and the resulting decrease in the yield and purity of the target product significantly increases the difficulty of purification, resulting in an increase in the time and effort involved in production. In addition, when the N-substituted amino acid becomes the reaction point of the amide bond formation reaction in the fragment coupling, reduction in reactivity due to steric hindrance of the substituent on the nitrogen atom, which is the reaction point, has been a problem (Non Patent Literature 8). Thus, when synthesizing a peptide compound containing an N-substituted amino acid, the synthesis method has been limited to a method of subjecting the amino acids to amide bond formation reactions sequentially by one residue at a time or a method of fragment coupling with residues that do not cause epimerization.

[Citation List]

[Patent Literature]

[Patent Literature 1] International Publication No. WO 2013/100132

[Non Patent Literature]

[Non Patent Literature 1] Acc. Chem. Res. 2008, 41, 1331-1342.
[Non Patent Literature 2] Angew. Chem. Int. Ed., 2013, 52, 254-269.
[Non Patent Literature 3] Chem. Rev., 2019, 119, 10360-10391.
[Non Patent Literature 4] Amino Acids, Peptides and Proteins in Organic Chemistry: Building Blocks, Catalysis and Coupling Chemistry, Volume 3, 2011.

[Non Patent Literature 5] J. Org. Chem., 1995, 60, 3569-3570.
[Non Patent Literature 6] Org. Lett. 2020, 22, 8039-8043.
[Non Patent Literature 7] ACS Sustainable Chem. Eng., 2022, 10, 5307-5314.
[Non Patent Literature 8] J. Peptide Res., 2005, 65, 153-166.

[Summary of Invention]

[Problems to be Solved by the Invention]

The present invention has been made in view of such circumstances. In one aspect, a problem to be solved is to establish a novel synthetic method for a peptide compound. In one aspect, a problem to be solved is to establish a novel synthetic method suitable for fragment coupling of peptide compounds. In one aspect, a problem to be solved is to establish a method for synthesizing a peptide compound with high diastereo-selectivity and high yield even by fragment coupling. In one aspect, a problem to be solved is to establish a synthetic method suitable for fragment coupling a peptide compound containing an N-substituted amino acid. In one aspect, a problem to be solved is to establish a synthetic method suitable for fragment coupling a peptide compound having a bulky side chain near the reaction point of an amide bond.

[Means for Solving the Problems]

The present inventors have conducted intensive studies to solve the above problems. As a result, the present inventors have found a method for synthesizing a peptide compound with high diastereo-selectivity and high yield even by fragment coupling, by performing an amide bond formation reaction in a mixture containing water and one or more solvents that are not miscible with water. In addition, the present inventors have found a method to solve at least one of the above problems by activating the carboxy group of an amino acid or peptide with a specific acyl halide in the above mixture. In addition, the present inventors have found reaction conditions that can inhibit the production of by-products in condensation reactions.

In one non-limiting specific aspect, the present invention encompasses the following.

[A1] A method for producing a peptide compound or a salt thereof, comprising a step (linking step) of linking an amino group of a first amino acid or peptide and a carboxy group of a second amino acid or peptide with an amide bond in a two-layer solvent containing water and one or more organic solvents that are not miscible with water.
[A2] The method according to [A1], wherein the linking step is carried out in the presence of an activator.
[A3] The method according to [A1] or [A2], wherein the activator is an acyl halide.
[A4] The method according to [A1], wherein the linking step includes steps of:

(1) bringing the second amino acid or peptide into contact with an acyl halide in an organic solvent to prepare a mixed acid anhydride; and
(2) bringing the mixed acid anhydride obtained in step (1) into contact with the first amino acid or peptide in the presence of a base in the two-layer solvent containing water and one or more organic solvents that are not miscible with water.

[A5] The method according to [A4], wherein step (2) of bringing the mixed acid anhydride obtained in step (1) into contact with the first amino acid or peptide is a step of adding the first amino acid or peptide to the mixed acid anhydride obtained in step (1).
[A6] The method according to any of [A1] to [A5], wherein the second amino acid or peptide is a peptide containing two or more amino acid residues.
[A7] The method according to any of [A1] to [A6], wherein the $\alpha$-position carbon of the carboxy group of the second amino acid or peptide is optionally substituted.
[A8] The method according to any of [A1] to [A6], wherein the $\alpha$-position carbon of the carboxy group of the second amino acid or peptide is represented by formula: $-CR^1R^2-$, wherein $R^1$ and $R^2$ are identical or different and each is a hydrogen atom, a linear $C_1$-$C_6$ alkyl, an optionally substituted branched $C_3$-$C_6$ alkyl, an optionally substituted $C_3$-$C_8$ cycloalkyl-$C_1$-$C_6$ alkyl, an optionally substituted $C_3$-$C_8$ cycloalkyl, an optionally substituted phenyl-$C_1$-$C_2$ alkyl, an optionally substituted $C_1$-$C_6$ alkoxy-$C_1$-$C_2$ alkyl, an optionally substituted 5- to 6-membered heteroaryl-$C_1$-$C_2$ alkyl, or an optionally substituted phenyl-$C_1$-$C_2$ alkylthio-$C_1$-$C_2$ alkyl, or $R^1$ and $R^2$ together with the carbon atom to which they are attached form a $C_3$-$C_8$ saturated alicyclic ring.
[A9] The method according to any of [A1] to [A6], wherein the $\alpha$-position carbon of the carboxy group of the second amino acid or peptide is represented by formula: $-CR^1R^2-$, wherein $R^1$ and $R^2$ are identical or different and each is a hydrogen atom, a linear $C_1$-$C_4$ alkyl, a branched $C_3$-$C_6$ alkyl, a $C_3$-$C_6$ cycloalkyl-$C_1$-$C_6$ alkyl, a $C_1$-$C_6$ alkoxymethyl, a

1-($C_1$-$C_6$ alkoxy)ethyl, an optionally substituted benzyl, an optionally substituted phenethyl, or a phenyl $C_1$-$C_2$ alkylthiomethyl.

[A10] The method according to [A8] or [A9], wherein $R^1$ is a hydrogen atom.

[A11] The method according to any of [A1] to [A6], wherein the α-position carbon of the carboxy group of the second amino acid or peptide is represented by formula: $-CR^1R^2-$, wherein $R^1$ is a hydrogen atom, and $R^2$ is a hydrogen atom, methyl, 1-methylethyl, 1-methylpropyl, t-butoxymethyl, 1-(t-butoxy)ethyl, benzyl, 2-(4-trifluoromethyl-3,5-difluorophenyl)ethyl, or benzylthiomethyl.

[A12] The method according to any of [A1] to [A11], wherein the β-position nitrogen of the carboxy group of the second amino acid or peptide is represented by formula: $-NR^3-$, wherein $R^3$ is a hydrogen atom.

[A13] The method according to any of [A1] to [A12], wherein the first amino acid or peptide is a peptide containing two or more amino acid residues.

[A14] The method according to any of [A1] to [A13], wherein the carboxy group of the first amino acid or peptide is protected with a protecting group.

[A15] The method according to any of [A1] to [A14], wherein the amino group of the second amino acid or peptide is protected with a protecting group.

[A16] The method according to any of [A3] to [A15], wherein the acyl halide is at least one selected from the group consisting of a linear $C_1$-$C_{18}$ alkylcarbonyl chloride, a branched $C_3$-$C_{18}$ alkylcarbonyl chloride, a linear $C_1$-$C_{18}$ alkylcarbonyl bromide, a branched $C_3$-$C_{18}$ alkylcarbonyl bromide, and a monocyclic or condensed cyclic $C_3$-$C_{15}$ cycloalkylcarbonyl chloride, in which the $C_3$-$C_{15}$ cycloalkylcarbonyl chloride is optionally substituted with a $C_1$-$C_6$ alkyl.

[A17] The method according to any of [A3] to [A16], wherein the acyl halide is at least one selected from the group consisting of pivaloyl chloride, 2,2-dimethylbutyryl chloride, 1-methylcyclohexanecarbonyl chloride, 1-adamantanecarbonyl chloride, and 2-ethylbutyryl chloride.

[A18] The method according to any of [A3] to [A17], wherein the acyl halide is pivaloyl chloride or 2,2-dimethylbutyryl chloride.

[A19] The method according to any of [A3] to [A18], wherein the acyl halide is used in 0.6 to 1.0 molar equivalents relative to the second amino acid or peptide.

[A20] The method according to any of [A4] to [A19], wherein step (1) is carried out in the presence of a base.

[A21] The method according to [A20], wherein pKa of a conjugate acid of the base used in step (1) is 15.0 or less, preferably 11.5 or less.

[A22] The method according to [A20] or [A21], wherein the base used in step (1) is an organic base, preferably at least one selected from the group consisting of amines and pyridines.

[A23] The method according to any of [A20] to [A22], wherein the base used in step (1) is at least one selected from the group consisting of diisopropylethylamine, triethylamine, 2,6-lutidine, and 2,4,6-collidine.

[A24] The method according to any of [A4] to [A23], wherein the base used in step (2) is at least one selected from the group consisting of sodium carbonate, potassium carbonate, cesium carbonate, potassium phosphate, sodium hydrogen carbonate, sodium hydrogen phosphate, triethylamine, and pyridine.

[A24-0] The method according to any of [A1] to [A24], wherein the linking step is carried out in the presence of an additive.

[A24-1] The method according to any of [A4] to [A24], wherein step (1) is carried out in the presence of an additive.

[A24-2] The method according to [A24-0] or [A24-1], wherein the additive is at least one selected from the group consisting of a bromide salt, an iodide salt, and a trifluoromethanesulfonate salt.

[A24-3] The method according to [A24-0] or [A24-1], wherein the additive is at least one selected from the group consisting of sodium bromide, sodium iodide, sodium trifluoromethanesulfonate, potassium bromide, potassium iodide, potassium trifluoromethanesulfonate, magnesium bromide, magnesium iodide, magnesium trifluoromethanesulfonate, calcium bromide, calcium iodide, calcium trifluoromethanesulfonate, zinc bromide, zinc iodide, zinc trifluoromethanesulfonate, barium bromide, barium iodide, and barium trifluoromethanesulfonate.

[A24-4] The method according to [A24-0] or [A24-1], wherein the additive is at least one selected from the group consisting of sodium bromide, sodium iodide, sodium trifluoromethanesulfonate, and potassium trifluoromethanesulfonate.

[A24-5] The method according to any of [A24-0] to [A24-4], wherein the additive is used in a range from 0.2 molar equivalents to 3.0 molar equivalents relative to the second amino acid or peptide.

[A24-6] The method according to any of [A24-0] to [A24-4], wherein the additive is used in a range from 0.3 molar equivalents to 1.5 molar equivalents relative to the second amino acid or peptide.

[A25] The method according to any of [A4] to [A24], wherein the organic solvent used in step (1) is one or more organic solvents that are not miscible with water.

[A26] The method according to any of [A1] to [A25], wherein the organic solvent that is not miscible with water is a solvent having an octanol/water partition coefficient (Log Kow) of 5 or more, or a solvent having an octanol/water

partition coefficient (Log Kow) predicted value of 5 or more.

[A27] The method according to any of [A1] to [A26], wherein the organic solvent that is not miscible with water is at least one selected from the group consisting of isopropyl acetate, ethyl acetate, butyl acetate, methyl t-butyl ether, diethyl ether, dichloromethane, carbon tetrachloride, 2-methyltetrahydrofuran, toluene, and hexane.

[A28] The method according to any of [A1] to [A27], wherein the organic solvent that is not miscible with water is at least one selected from the group consisting of isopropyl acetate, methyl t-butyl ether, dichloromethane, 2-methyltetrahydrofuran, and toluene.

[A29] The method according to any of [A1] to [A28], wherein the amino group of the first amino acid or peptide is an amino group represented by formula: $-NR^4R^5$, wherein $R^4$ is a hydrogen atom, and $R^5$ is a hydrogen atom, a linear $C_1$-$C_6$ alkyl, a branched $C_3$-$C_6$ alkyl, or a $C_3$-$C_8$ cycloalkyl.

[A30] The method according to any of [A1] to [A29], wherein the amino group of the first amino acid or peptide is an amino group represented by formula: $-NR^4R^5$, wherein $R^4$ is a hydrogen atom, and $R^5$ is a hydrogen atom or a linear $C_1$-$C_4$ alkyl.

[A31] The method according to any of [A1] to [A30], wherein the amino group of the first amino acid or peptide is an amino group represented by formula: $-NR^4R^5$, wherein $R^4$ is a hydrogen atom, and $R^5$ is a hydrogen atom, methyl or ethyl.

[A32] The method according to any of [A1] to [A31], wherein the amino group of the first amino acid or peptide is an amino group represented by formula: $-NR^4R^5$, wherein $R^4$ is a hydrogen atom, and $R^5$ is a hydrogen atom or methyl.

[A33] The method according to any of [A1] to [A32], wherein the amino group of the first amino acid or peptide is an amino group represented by formula: $-NR^4R^5$, wherein $R^4$ is a hydrogen atom, and $R^5$ is methyl.

[A34] The method according to any of [A1] to [A33], wherein the peptide compound to be produced or a salt thereof contains 8 to 20 amino acid residues.

[A35] The method according to any of [A1] to [A34], wherein the peptide compound to be produced or a salt thereof contains 11 to 14 amino acid residues.

[A36] The method according to any of [A1] to [A35], wherein the peptide compound to be produced contains at least one non-natural amino acid residue.

[A37] The method according to any of [A1] to [A36], wherein the peptide compound to be produced contains at least four non-natural amino acid residues.

[A38] The method according to any of [A1] to [A37], wherein the peptide compound to be produced contains at least five non-natural amino acid residues.

[A39] The method according to any of [A36] to [A38], wherein the non-natural amino acid is an N-methylamino acid residue.

[A40] The method according to any of [A1] to [A39], wherein the peptide compound to be produced or a salt thereof contains a cyclic portion composed of 4 to 14 amino acid residues, and wherein an amide bond with which the amino group of the first amino acid or peptide and the carboxy group of the second amino acid or peptide are linked is contained in the cyclic portion at 1 to 7 locations.

[A41] The method according to any of [A1] to [A40], which is performed using a flow reaction apparatus.

[A42] A peptide compound or a salt thereof produced by the method according to any of [A1] to [A41].

[A43] A pharmaceutical composition comprising the peptide compound or a salt thereof produced by the method according to any of [A1] to [A41].

[B1] A method for producing a peptide compound or a salt thereof, comprising a step (linking step) of linking an amino group of a first amino acid or peptide and a carboxy group of a second amino acid or peptide with an amide bond in a two-layer solvent containing water and one or more organic solvents that are not miscible with water, wherein the amino group of the first amino acid or peptide is an amino group represented by formula: $-NR^4R^5$, wherein $R^4$ is a hydrogen atom, and $R^5$ is a hydrogen atom, a linear $C_1$-$C_6$ alkyl, a branched $C_3$-$C_6$ alkyl, or a $C_3$-$C_8$ cycloalkyl.

[B2] The method according to [B1], wherein the linking step includes steps of:

(1) bringing the second amino acid or peptide into contact with an acyl halide in an organic solvent to prepare a mixed acid anhydride; and

(2) bringing the mixed acid anhydride obtained in step (1) into contact with the first amino acid or peptide in the presence of a base in the two-layer solvent containing water and one or more organic solvents that are not miscible with water.

[B3] The method according to [B2], wherein step (2) of bringing the mixed acid anhydride obtained in step (1) into contact with the first amino acid or peptide is a step of adding the first amino acid or peptide to the mixed acid anhydride obtained in step (1).

[B4] The method according to any of [B1] to [B3], wherein the amino group of the first amino acid or peptide is an amino group represented by formula: $-NR^4R^5$, wherein $R^4$ is a hydrogen atom, and $R^5$ is a hydrogen atom or a linear $C_1$-$C_4$

alkyl.

[B5] The method according to any of [B1] to [B4], wherein the amino group of the first amino acid or peptide is an amino group represented by formula: $-NR^4R^5$, wherein $R^4$ is a hydrogen atom, and $R^5$ is a hydrogen atom, methyl or ethyl.

[B6] The method according to any of [B1] to [B5], wherein the amino group of the first amino acid or peptide is an amino group represented by formula: $-NR^4R^5$, wherein $R^4$ is a hydrogen atom, and $R^5$ is a hydrogen atom or methyl.

[B7] The method according to any of [B1] to [B6], wherein the amino group of the first amino acid or peptide is an amino group represented by formula: $-NR^4R^5$, wherein $R^4$ is a hydrogen atom and $R^5$ is methyl.

[B8] The method according to any of [B1] to [B7], wherein the second amino acid or peptide is a peptide containing two or more amino acid residues.

[B9] The method according to any of [B1] to [B8], wherein the carboxy group of the first amino acid or peptide is protected.

[B10] The method according to any of [B1] to [B9], wherein the amino group of the second amino acid or peptide is protected.

[B11] The method according to any of [B1] to [B10], wherein the $\alpha$-position carbon of the carboxy group of the second amino acid or peptide is optionally substituted.

[B12] The method according to any of [B1] to [B10], wherein the $\alpha$-position carbon of the carboxy group of the second amino acid or peptide is represented by formula: $-CR^1R^2-$, wherein $R^1$ and $R^2$ are identical or different and each is a hydrogen atom, a linear $C_1$-$C_6$ alkyl, an optionally substituted branched $C_3$-$C_6$ alkyl, an optionally substituted $C_3$-$C_8$ cycloalkyl-$C_1$-$C_6$ alkyl, an optionally substituted $C_3$-$C_8$ cycloalkyl, an optionally substituted phenyl-$C_1$-$C_2$ alkyl, an optionally substituted $C_1$-$C_6$ alkoxy-$C_1$-$C_2$ alkyl, an optionally substituted 5- to 6-membered heteroaryl-$C_1$-$C_2$ alkyl, or an optionally substituted phenyl-$C_1$-$C_2$ alkylthio-$C_1$-$C_2$ alkyl, or $R^1$ and $R^2$ together with the carbon atom to which they are attached form a $C_3$-$C_8$ saturated alicyclic ring.

[B13] The method according to any of [B1] to [B10], wherein the $\alpha$-position carbon of the carboxy group of the second amino acid or peptide is represented by formula: $-CR^1R^2-$, wherein $R^1$ and $R^2$ are identical or different and each is a hydrogen atom, a linear $C_1$-$C_4$ alkyl, a branched $C_3$-$C_6$ alkyl, a $C_3$-$C_6$ cycloalkyl-$C_1$-$C_6$ alkyl, a $C_1$-$C_6$ alkoxymethyl, a 1-($C_1$-$C_6$ alkoxy)ethyl, an optionally substituted benzyl, an optionally substituted phenethyl, or a phenyl $C_1$-$C_2$ alkylthiomethyl.

[B14] The method according to [B12] or [B13], wherein $R^1$ is a hydrogen atom.

[B15] The method according to any of [B1] to [B10], wherein the $\alpha$-position carbon of the carboxy group of the second amino acid or peptide is represented by formula: $-CR^1R^2-$, wherein $R^1$ is a hydrogen atom, and $R^2$ is a hydrogen atom, methyl, 1-methylethyl, 1-methylpropyl, t-butoxymethyl, 1-(t-butoxy)ethyl, benzyl, 2-(4-trifluoromethyl-3,5-difluorophenyl)ethyl, or benzylthiomethyl.

[B16] The method according to any of [B1] to [B15], wherein the $\beta$-position nitrogen of the carboxy group of the second amino acid or peptide is represented by formula: $-NR^3-$, wherein $R^3$ is a hydrogen atom.

[B17] The method according to any of [B1] to [B16], wherein the first amino acid or peptide is a peptide containing two or more amino acid residues.

[B18] The method according to any of [B1] and [B4] to [B17], wherein the linking step is carried out in the presence of an activator.

[B19] The method according to [B18], wherein the activator is an acyl halide.

[B20] The method according to any of [B2], [B3], and [B19], wherein the acyl halide is at least one selected from the group consisting of a linear $C_1$-$C_{18}$ alkylcarbonyl chloride, a branched $C_3$-$C_{18}$ alkylcarbonyl chloride, a linear $C_1$-$C_{18}$ alkylcarbonyl bromide, a branched $C_3$-$C_{18}$ alkylcarbonyl bromide, and a monocyclic or condensed cyclic $C_3$-$C_{15}$ cycloalkylcarbonyl chloride, in which the $C_3$-$C_{15}$ cycloalkylcarbonyl chloride is optionally substituted with a $C_1$-$C_6$ alkyl.

[B21] The method according to any of [B2] to [B3] and [B19] to [B20], wherein the acyl halide is at least one selected from the group consisting of pivaloyl chloride, 2,2-dimethylbutyryl chloride, 1-methylcyclohexanecarbonyl chloride, 1-adamantanecarbonyl chloride, and 2-ethylbutyryl chloride.

[B22] The method according to any of [B2] to [B3] and [B19] to [B21], wherein the acyl halide is pivaloyl chloride or 2,2-dimethylbutyryl chloride.

[B23] The method according to any of [B2] to [B3] and [B19] to [B22], wherein the acyl halide is used in 0.6 to 1.0 molar equivalents relative to the second amino acid or peptide.

[B24] The method according to any of [B2] to [B17] and [B20] to [B23], wherein step (1) is carried out in the presence of a base.

[B25] The method according to [B24], wherein pKa of a conjugate acid of the base used in step (1) is 15.0 or less, preferably 11.5 or less.

[B26] The method according to [B24] or [B25], wherein the base used in step (1) is an organic base, preferably at least one selected from the group consisting of amines and pyridines.

[B27] The method according to any of [B23] to [B26], wherein the base used in step (1) is at least one selected from the

group consisting of diisopropylethylamine, triethylamine, 2,6-lutidine, and 2,4,6-collidine.

[B28] The method according to any of [B2] to [B27], wherein the base used in step (2) is at least one selected from the group consisting of sodium carbonate, potassium carbonate, cesium carbonate, potassium phosphate, sodium hydrogen carbonate, sodium hydrogen phosphate, triethylamine, and pyridine.

[B28-0] The method according to any of [B1] to [B28], wherein the linking step is carried out in the presence of an additive.

[B28-1] The method according to any of [B2] to [B28], wherein step (1) is carried out in the presence of an additive.

[B28-2] The method according to [B28-0] or [B28-1], wherein the additive is at least one selected from the group consisting of a bromide salt, an iodide salt, and a trifluoromethanesulfonate salt.

[B28-3] The method according to [B28-0] or [B28-1], wherein the additive is at least one selected from the group consisting of sodium bromide, sodium iodide, sodium trifluoromethanesulfonate, potassium bromide, potassium iodide, potassium trifluoromethanesulfonate, magnesium bromide, magnesium iodide, magnesium trifluoromethanesulfonate, calcium bromide, calcium iodide, calcium trifluoromethanesulfonate, zinc bromide, zinc iodide, zinc trifluoromethanesulfonate, barium bromide, barium iodide, and barium trifluoromethanesulfonate.

[B28-4] The method according to [B28-0] or [B28-1], wherein the additive is at least one selected from the group consisting of sodium bromide, sodium iodide, sodium trifluoromethanesulfonate, and potassium trifluoromethanesulfonate.

[B28-5] The method according to any of [B28-0] to [B28-4], wherein the additive is used in a range from 0.2 molar equivalents to 3.0 molar equivalents relative to the second amino acid or peptide.

[B28-6] The method according to any of [B28-0] to [B28-4], wherein the additive is used in a range from 0.3 molar equivalents to 1.5 molar equivalents relative to the second amino acid or peptide.

[B29] The method according to any of [B2] to [B17] and [B20] to [B28], wherein the organic solvent used in step (1) is one or more organic solvents that are not miscible with water.

[B30] The method according to any of [B1] to [B29], wherein the organic solvent that is not miscible with water is a solvent having an octanol/water partition coefficient (Log Kow) of 5 or more, or a solvent having an octanol/water partition coefficient (Log Kow) predicted value of 5 or more.

[B31] The method according to any of [B1] to [B30], wherein the organic solvent that is not miscible with water is at least one selected from the group consisting of isopropyl acetate, ethyl acetate, butyl acetate, methyl t-butyl ether, diethyl ether, dichloromethane, carbon tetrachloride, 2-methyltetrahydrofuran, toluene, and hexane.

[B32] The method according to any of [B1] to [B31], wherein the organic solvent that is not miscible with water is at least one selected from the group consisting of isopropyl acetate, methyl t-butyl ether, dichloromethane, 2-methyltetrahydrofuran, and toluene.

[B33] The method according to any of [B1] to [B32], wherein the peptide compound to be produced or a salt thereof contains 8 to 20 amino acid residues.

[B34] The method according to any of [B1] to [B33], wherein the peptide compound to be produced or a salt thereof contains 11 to 14 amino acid residues.

[B35] The method according to any of [B1] to [B34], wherein the peptide compound to be produced contains at least one non-natural amino acid residue.

[B36] The method according to any of [B1] to [B35], wherein the peptide compound to be produced contains at least four non-natural amino acid residues.

[B37] The method according to any of [B1] to [B36], wherein the peptide compound to be produced contains at least five non-natural amino acid residues.

[B38] The method according to any of [B34] to [B37], wherein the non-natural amino acid is an N-methylamino acid residue.

[B39] The method according to any of [B1] to [B38], wherein the peptide compound to be produced or a salt thereof contains a cyclic portion composed of 4 to 14 amino acid residues, and wherein an amide bond with which the amino group of the first amino acid or peptide and the carboxy group of the second amino acid or peptide are linked is contained in the cyclic portion at 1 to 7 locations.

[B40] The method according to any of [B1] to [B39], which is performed using a flow reaction apparatus.

[B41] A peptide compound or a salt thereof produced by the method according to any of [B1] to [B40].

[B42] A pharmaceutical composition comprising the peptide compound or a salt thereof produced by the method according to any of [B1] to [B40].

[Advantageous Effects of Invention]

According to the present invention, a peptide compound can be synthesized with high diastereo-selectivity and high yield even by fragment coupling. Further according to the present invention, a synthetic method suitable for fragment coupling a peptide compound containing an N-substituted amino acid and/or a peptide compound having a bulky side

chain near the reaction point of an amide bond can be provided.

[Mode for Carrying Out the Invention]

The abbreviations used herein are listed below.

2-MeTHF: 2-methyltetrahydrofuran
IPAC: isopropyl acetate
EtOAc: ethyl acetate
MeCN: acetonitrile
THF: tetrahydrofuran
MeOH: methanol
MTBE: methyl tert-butyl ether
CPME: cyclopentyl methyl ether
DCM: dichloromethane
DIPEA: N,N-diisopropylethylamine
TEA: triethylamine
NMM: N-methylmorpholine
NMI: N-methylimidazole
T3P: propylphosphonic acid anhydride
HATU: O-(7-azabenzotriazol-1-yl)-N,N,N',N'-tetramethyluronium hexafluorophosphate
COMU: (1-cyano-2-ethoxy-2-oxoethylideneaminoxy)dimethylaminomorpholinocarbenium hexafluorophosphate
PivCl: pivaloyl chloride
$Me_2BtrCl$: 2,2-dimethylbutyryl chloride
EDC·HCl: 1-(3-dimethylaminopropyl)-3-ethylcarbodiimide hydrochloride
HOPO: 2-hydroxypyridine-N-oxide
$H_3PO_4$: phosphoric acid
NaCl: sodium chloride
$Na_2CO_3$: sodium carbonate
$K_2CO_3$: potassium carbonate
$Cs_2CO_3$: cesium carbonate
$NaHCO_3$: sodium hydrogen carbonate
$K_3PO_4$: tripotassium phosphate
$Na_2HPO_4$: disodium hydrogen phosphate
$K_2HPO_4$: dipotassium hydrogen phosphate
NaOH: sodium hydroxide
KOH: potassium hydroxide
LiOH: lithium hydroxide
$NaHSO_4$: sodium hydrogen sulfate
$Na_2SO_4$: sodium sulfate
$H_2O$: water
TFA: trifluoroacetic acid
HMDS: hexamethyldisilazane
TMSOTf: trimethylsilyl trifluoromethanesulfonate
Pd/C: palladium on carbon
Alloc: allyloxycarbonyl
Boc: t-butoxycarbonyl
Cbz: benzyloxycarbonyl
Teoc: 2-(trimethylsilyl)ethoxycarbonyl
Fmoc: 9-fluorenylmethyloxycarbonyl
TBAF: tetrabutylammonium fluoride
$LiBH_4$: lithium borohydride
EDCI: 1-ethyl-3-(3-dimethylaminopropyl)carbodiimide hydrochloride
BEP: 2-bromo-1-ethylpyridinium tetrafluoroborate
PyBOP: 1H-benzotriazol-1-yloxytripyrrolidinophosphonium hexafluorophosphate
DMT-MM: 4-(4,6-dimethoxy-1,3,5-triazin-2-yl)-4-methylmorpholinium chloride
PyOxim: (ethylcyano(hydroxyimino)acetat-O2)-tri-(1-pyrrolidinyl)-phosphonium hexafluorophosphate
NaBr: sodium bromide

NaI: sodium iodide
NaOTf: sodium trifluoromethanesulfonate
KOTf: potassium trifluoromethanesulfonate

Definitions of functional groups and the like (The terms explained below are illustrative and not intended to be particularly limiting, and are those that can be commonly understood by those skilled in the art.)

The "halogen atom" as used herein include fluorine, chlorine, bromine, and iodine. As used herein, F refers to fluorine, Cl refers to chlorine, Br refers to bromine, and I refers to iodine. Examples of the halogen include fluorine, chlorine and bromine, and preferably fluorine and chlorine.

The "alkyl" as used herein is a linear or branched monovalent saturated hydrocarbon group derived from an aliphatic saturated hydrocarbon by removing any one hydrogen atom, the group having a subset of a hydrocarbyl or hydrocarbon group structure containing hydrogen and carbon atoms without containing a heteroatom (which refers to an atom other than carbon and hydrogen atoms) or an unsaturated carbon-carbon bond in the skeleton. The alkyl includes not only a linear alkyl but also a branched alkyl. Specifically, the alkyl is an alkyl having 1 to 20 carbon atoms ($C_1$-$C_{20}$), preferably $C_1$-$C_{10}$ alkyl, more preferably $C_1$-$C_6$ alkyl. Hereinafter, "$C_p$-$C_q$" means that it has p to q carbon atoms. Specific examples of the alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl. Specific examples of the linear $C_1$-$C_6$ alkyl include methyl, ethyl, n-propyl, n-butyl, n-pentyl, and n-hexyl. Specific examples of the linear $C_1$-$C_3$ alkyl include methyl, ethyl, and n-propyl. Specific examples of the branched $C_3$-$C_6$ alkyl include i-propyl, s-butyl, t-butyl, isobutyl (2-methylpropyl), s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

The "alkenyl" as used herein is a linear or branched monovalent unsaturated hydrocarbon group having one or more carbon-carbon double bonds (bonds by two adjacent sp2 carbon atoms). Depending on the conformation of the atoms or groups of atoms attached to the sp2 carbon atoms, the geometric morphology of the double bond can assume entgegen (E) or zusammen (Z) and cis or trans conformations. The alkenyl is, for example, $C_2$-$C_{10}$ alkenyl, preferably $C_2$-$C_8$ alkenyl, more preferably $C_2$-$C_7$ alkenyl, most preferably $C_2$-$C_6$ alkenyl. Specific examples of the alkenyl include ethenyl (vinyl), 1-propenyl, 2-propenyl (allyl), isopropenyl, 1-butenyl, 2-butenyl (including cis, trans), 3-butenyl, pentenyl, and hexenyl.

The "alkynyl" as used herein is a linear or branched monovalent unsaturated hydrocarbon group having one or more carbon-carbon triple bonds (bonds by two adjacent sp carbon atoms). The alkynyl is, for example, $C_2$-$C_{10}$ alkynyl, preferably $C_2$-$C_8$ alkynyl, more preferably $C_2$-$C_7$ alkynyl, most preferably $C_2$-$C_6$ alkynyl. Specific examples of the alkynyl include ethynyl, 1-propynyl, propargyl (2-propynyl), 1-butynyl, 2-butynyl, 3-butynyl, pentynyl, and hexynyl.

The "cycloalkyl" as used herein is a saturated or partially saturated cyclic monovalent non-aromatic hydrocarbon ring group (alicyclic ring group). A carbon atom constituting the ring may be oxidated to form carbonyl. The cycloalkyl may be selected from the group consisting of a monocyclic ring, a condensed ring, and a spiro ring. As used herein, the cycloalkyl containing a monocyclic ring is referred to as monocyclic cycloalkyl or a monocyclic alicyclic ring group; the cycloalkyl containing a condensed ring is referred to as condensed cyclic cycloalkyl or a condensed cyclic alicyclic ring group; and the cycloalkyl containing a spiro ring is referred to as spirocyclic cycloalkyl or a spirocyclic alicyclic ring group. The cycloalkyl may form a condensed ring with a saturated alicyclic ring such as a cyclopentane ring or a cyclohexane ring, an unsaturated alicyclic ring such as a cyclopentene ring or a cyclohexene ring, or an aromatic hydrocarbon ring such as a benzene ring or a naphthalene ring. The cycloalkyl may form a spiro ring with a saturated alicyclic ring such as a cyclopropane ring, a cyclobutane ring, a cyclopentane ring, or a cyclohexane ring. The cycloalkyl is, for example, $C_3$-$C_{10}$ cycloalkyl, preferably $C_3$-$C_8$ cycloalkyl, more preferably $C_3$-$C_7$ cycloalkyl, most preferably $C_3$-$C_6$ cycloalkyl. Specific examples of the cycloalkyl include cyclopropyl, cyclobutyl, cyclopentyl, cyclohexyl, cycloheptyl, cyclooctyl, bicyclo[2.2.1]heptyl, spiro[3.3]heptyl, and cyclohexenyl.

The "aryl" as used herein is a monovalent aromatic hydrocarbon ring group composed of a monovalent, monocyclic or condensed ring that exhibits aromaticity. As used herein, the aryl composed of a monocyclic ring is referred to as a monocyclic aryl, and an aryl composed of a condensed ring is referred to as a condensed cyclic aryl. The aryl is, for example, $C_6$-$C_{14}$ aryl, preferably $C_6$ aryl, $C_{10}$ aryl and $C_{14}$ aryl, more preferably $C_6$ aryl and $C_{10}$ aryl, most preferably $C_6$ aryl. Specific examples of the aryl include phenyl, 1-naphthyl, 2-naphthyl, tolyl, and xylyl.

The "heterocyclyl" as used herein is a heterocyclic group that contains, as an atom constituting a ring, a hetero atom selected from the group consisting of nitrogen atoms, oxygen atoms and sulfur atoms, in addition to the carbon atom, preferably 1 to 5 hetero atoms, more preferably 1 to 3 hetero atoms, and that may have a double and/or triple bond in the ring. A carbon atom in the ring of the heterocyclyl may be oxidated to form carbonyl. As used herein, the heterocyclyl

containing a monocyclic ring is referred to as a monocyclic heterocyclyl; the heterocyclyl containing a condensed ring is referred to as a condensed cyclic heterocyclyl; and the heterocyclyl containing a spiro ring is referred to as a spirocyclic heterocyclyl. The heterocyclyl may form a condensed ring or a spiro ring with a saturated alicyclic ring such as a cyclopentane ring or a cyclohexane ring, or a saturated heterocyclic ring such as a tetrahydropyran ring, a dioxane ring, or a pyrrolidine ring. The number of atoms constituting the ring of heterocyclyl is, for example, 3 to 14 (3- to 14-membered heterocyclyl), preferably 3 to 12 (3- to 12-membered heterocyclyl), more preferably 3 to 10 (3- to 10-membered heterocyclyl), most preferably 4 to 7 (4- to 7-membered heterocyclyl). Specific examples of the heterocyclyl include azetidinyl, oxiranyl, oxetanyl, thietanyl, tetrahydrofuranyl, pyrrolidinyl, pyrazolidinyl, imidazolidinyl, oxazolidinyl, isoxazolidinyl, thiazolidinyl, isothiazolidinyl, thiadiazolidinyl, oxazolidinyl, dioxolanyl, tetrahydropyranyl, morpholinyl, thiomorpholinyl, 4-oxopyrrolidinyl, piperidinyl, 4-oxopiperidinyl, piperazinyl, and dioxanyl, and rings in which one or more single bonds in these saturated heterocycles are replaced with a double bond or a triple bond.

The "heteroaryl" as used herein is a monovalent aromatic heterocyclic group that contains at least one heteroatom in addition to the carbon atom and is composed of a monocyclic or condensed ring that exhibits aromaticity. As used herein, a heteroaryl composed of a monocyclic ring is referred to as a monocyclic heteroaryl, and a heteroaryl composed of a condensed ring is referred to as a condensed cyclic heteroaryl. The number of atoms constituting the ring of heteroaryl is, for example, 5 to 14 (5- to 14-membered heteroaryl), preferably 5 to 13 (5- to 13-membered heteroaryl), more preferably 5 to 10 (5- to 10-membered heteroaryl), most preferably 5 to 7 (5- to 7-membered heteroaryl). Specific examples of the heteroaryl include 5-membered heteroaryl such as furyl, thienyl, pyrrolyl, imidazolyl, pyrazolyl, thiazolyl, oxazolyl, triazolyl, or tetrazolyl; 6-membered heteroaryl such as pyridyl, pyrimidyl, pyridazinyl, pyrazinyl, or triazinyl; 9-membered heteroaryl such as benzofuranyl, benzothienyl, benzothiazolyl, benzimidazolyl, benzotriazolyl, indolyl, indazolyl, or pyrazolopyridyl; and 10-membered heteroaryl such as quinolyl, isoquinolyl, cinolinyl, quinazolinyl, or quinoxalinyl.

The "alkoxy" as used herein is a group (-OR, wherein R is alkyl) in which "alkyl" as defined herein is attached to an oxygen atom. The alkoxy is, for example, $C_1$-$C_{20}$ alkoxy, preferably $C_1$-$C_{10}$ alkoxy, more preferably $C_1$-$C_8$ alkoxy, most preferably $C_1$-$C_6$ alkoxy. Specific examples of the alkoxy include methoxy, ethoxy, 1-propoxy, 2-propoxy, n-butoxy, i-butoxy, s-butoxy, t-butoxy, pentyloxy, and 3-methylbutoxy.

The "alkenyloxy" as used herein is a group (-OR, wherein R is alkenyl) in which "alkenyl" as defined herein is attached to an oxygen atom. The alkenyloxy is, for example, $C_2$-$C_{10}$ alkenyloxy, preferably $C_2$-$C_8$ alkenyloxy, more preferably $C_2$-$C_7$ alkenyloxy, most preferably $C_2$-$C_6$ alkenyloxy. Specific examples of the alkenyloxy include ethenyl (vinyl) oxy, 1-propenyloxy, 2-propenyl (allyl) oxy, isopropenyloxy, 1-butenyloxy, 2-cis-butenyloxy, 2-trans-butenyloxy, 3-butenyloxy, pentenyloxy, and hexenyloxy.

The "cycloalkoxy" as used herein is a group (-OR, wherein R is cycloalkyl) in which "cycloalkyl" as defined herein is attached to an oxygen atom. The cycloalkoxy is, for example, $C_3$-$C_{10}$ cycloalkoxy, preferably $C_3$-$C_8$ cycloalkoxy, more preferably $C_3$-$C_7$ cycloalkoxy, most preferably $C_3$-$C_6$ cycloalkoxy. Specific examples of the cycloalkoxy include cyclopropoxy, cyclobutoxy, and cyclopentyloxy.

The "aryloxy" as used herein is a group (-OAr, wherein Ar is aryl) in which "aryl" as defined herein is attached to an oxygen atom. The aryloxy is, for example, $C_6$-$C_{14}$ aryloxy, preferably $C_6$ aryloxy, $C_{10}$ aryloxy and $C_{14}$ aryloxy, more preferably $C_6$ aryloxy and $C_{10}$ aryloxy, most preferably $C_6$ aryloxy. Specific examples of the aryloxy include phenoxy, 1-naphthyloxy, 2-naphthyloxy, tolyloxy, and xylyloxy.

The "amino" as used herein means -NRR' wherein N represents a nitrogen atom and R and R' are each independently selected from the group consisting of a hydrogen atom, alkyl, alkenyl, alkynyl, cycloalkyl, heterocyclyl, aryl, and heteroaryl, or R and R' form a ring together with the nitrogen atom to which they are attached. Examples of the amino include $-NH_2$, mono-$C_1$-$C_6$ alkylamino, di-$C_1$-$C_6$ alkylamino, and 4- to 8-membered cyclic amino.

The "monoalkylamino" as used herein means a group of the "amino (-NRR')" as defined herein, in which R is a hydrogen atom and R' is the "alkyl". The monoalkylamino is, for example, $C_1$-$C_{20}$ alkylamino, preferably mono-$C_1$-$C_{15}$ alkylamino, more preferably mono-$C_1$-$C_{10}$ alkylamino, most preferably mono-$C_1$-$C_6$ alkylamino. Specific examples of the monoalkylamino include methylamino, ethylamino, n-propylamino, i-propylamino, n-butylamino, s-butylamino, and t-butylamino.

The "dialkylamino" as used herein means a group of the "amino (-NRR')" as defined herein, in which R and R' are each independently the "alkyl". The dialkylamino is, for example, $C_1$-$C_{20}$ alkylamino, preferably di-$C_1$-$C_{15}$ alkylamino, more preferably di-$C_1$-$C_{10}$ alkylamino, most preferably di-$C_1$-$C_6$ alkylamino. Specific examples of the dialkylamino include dimethylamino, diethylamino, and methyl ethylamino.

The "cyclic amino" as used herein is a group of the "amino (-NRR')" as defined herein, in which R and R' form a ring together with the nitrogen atom to which they are attached. The cyclic amino is, for example, a 3- to 14-membered cyclic amino, preferably a 3- to 12-membered cyclic amino, more preferably a 3- to 10-membered cyclic amino, most preferably a 4- to 7-membered cyclic amino. Specific examples of the cyclic amino include 1-azetidyl, 1-pyrrolidyl, 1-piperidyl, 1-piperazyl, 4-morpholinyl, 3-oxazolidyl, 1,1-dioxidothiomorpholinyl-4-yl, and 3-oxa-8-azabicyclo[3.2.1]octan-8-yl.

The "protected amino" as used herein means an amino group protected with an optional protecting group.

Specific examples of the protected amino include an amino group protected by a protecting group such as Boc (tert-butoxycarbonyl), Fmoc (9-fluorenylmethyloxycarbonyl), Cbz (benzyloxycarbonyl), Troc (2,2,2-trichloroethoxycarbonyl), Alloc (allyloxycarbonyl), Teoc (2-(trimethylsilyl)ethoxycarbonyl), or trifluoroacetyl.

The "aminocarbonyl" as used herein is a group in which an "amino" as defined herein is attached to a carbon atom of a carbonyl. It is also sometimes referred to as amide. Examples of the aminocarbonyl include $-CONH_2$, mono-$C_1$-$C_6$ alkylaminocarbonyl, di-$C_1$-$C_6$ alkylaminocarbonyl, and 4- to 8-membered cyclic aminocarbonyl. Specific examples of the aminocarbonyl include $-CONH_2$, methylaminocarbonyl, ethylaminocarbonyl, dimethylaminocarbonyl, diethylaminocarbonyl, 1-azetidinylcarbonyl, 1-pyrrolidinylcarbonyl, 1-piperidinylcarbonyl, 1-piperazinylcarbonyl, 4-morpholinylcarbonyl, and 3-oxazolidinylcarbonyl.

The "alkenyloxycarbonyl" as used herein is a carbonyl group to which "alkenyloxy" as defined herein is attached. The alkenyloxycarbonyl is, for example, $C_2$-$C_{10}$ alkenyloxycarbonyl, preferably $C_2$-$C_8$ alkenyloxycarbonyl, more preferably $C_2$-$C_7$ alkenyloxycarbonyl, most preferably $C_2$-$C_6$ alkenyloxycarbonyl. Specific examples of the alkenyloxycarbonyl include ethenyl (vinyl) oxycarbonyl, 1-propenyloxycarbonyl, 2-propenyl (allyl) oxycarbonyl, isopropenyloxycarbonyl, 1-butenyloxycarbonyl, 2-cis-butenyloxycarbonyl, 2-trans-butenyloxycarbonyl, 3-butenyloxycarbonyl, pentenyloxycarbonyl, and hexenyloxycarbonyl.

The "alkylsulfonyl" as used herein is a sulfonyl group to which "alkyl" as defined herein is attached. The alkylsulfonyl is, for example, $C_1$-$C_{20}$ alkylsulfonyl, preferably $C_1$-$C_{10}$ alkylsulfonyl, more preferably $C_1$-$C_8$ alkylsulfonyl, most preferably $C_1$-$C_6$ alkylsulfonyl. Specific examples of the alkylsulfonyl include methylsulfonyl, ethylsulfonyl, 1-propylsulfonyl, 2-propylsulfonyl, n-butylsulfonyl, i-butylsulfonyl, s-butylsulfonyl, t-butylsulfonyl, pentylsulfonyl, and 3-methylbutylsulfonyl.

The "hydroxyalkyl" as used herein is a group in which one or more hydrogen atoms of the "alkyl" as defined herein are substituted with a hydroxy group. The hydroxyalkyl is preferably a group in which one hydrogen atom of the alkyl is substituted with a hydroxy group. The hydroxyalkyl is, for example, hydroxy $C_1$-$C_{20}$ alkyl, preferably hydroxy $C_1$-$C_{10}$ alkyl, more preferably hydroxy $C_1$-$C_8$ alkyl, most preferably hydroxy $C_1$-$C_6$ alkyl. Specific examples of the hydroxyalkyl include hydroxyethyl, 1-hydroxyethyl, 2-hydroxyethyl, 2-hydroxy-2-methylpropyl, and 5-hydroxypentyl.

The "haloalkyl" as used herein is a group in which one or more hydrogen atoms of the "alkyl" as defined herein are substituted with halogen. The haloalkyl is preferably a group in which one or more and six or less hydrogen atoms that are allowed to be substituted of the hydrogen atoms of the alkyl are substituted with halogen. The haloalkyl is, for example, halo-$C_1$-$C_{20}$ alkyl, preferably halo-$C_1$-$C_{10}$ alkyl, more preferably halo-$C_1$-$C_8$ alkyl, most preferably halo-$C_1$-$C_6$ alkyl. The halo-$C_1$-$C_6$ alkyl is, for example, a group in which one or more and six or less hydrogen atoms allowed to be substituted of the hydrogen atoms of the alkyl are substituted with fluorine, preferably a group in which one or more and five or less hydrogen atoms are substituted with fluorine, more preferably a group in which one or more and four or less hydrogen atoms are substituted with fluorine, most preferably a group in which one or more and three or less hydrogen atoms are substituted with fluorine. Specific examples of the haloalkyl include difluoromethyl, trifluoromethyl, 2,2-difluoroethyl, 2,2,2-trifluoroethyl, 3,3-difluoropropyl, 4,4-difluorobutyl, and 5,5-difluoropentyl.

The "cyanoalkyl" as used herein is a group in which one or more hydrogen atoms of the "alkyl" as defined herein are substituted with cyano. The cyanoalkyl is preferably a group in which one hydrogen atom of the alkyl is substituted with cyano. The cyanoalkyl is, for example, cyano $C_1$-$C_{20}$ alkyl, preferably cyano $C_1$-$C_{10}$ alkyl, more preferably cyano $C_1$-$C_8$ alkyl, most preferably cyano $C_1$-$C_6$ alkyl. Specific examples of the cyanoalkyl include cyanomethyl and 2-cyanoethyl.

The "aminoalkyl" as used herein is a group in which one or more hydrogen atoms of the "alkyl" as defined herein are substituted with amino. The aminoalkyl is preferably a group in which one hydrogen atom of the alkyl is substituted with amino. The aminoalkyl is, for example, amino $C_1$-$C_{20}$ alkyl, preferably amino $C_1$-$C_{10}$ alkyl, more preferably amino $C_1$-$C_8$ alkyl, most preferably amino $C_1$-$C_6$ alkyl. Specific examples of the aminoalkyl include aminomethyl, aminoethyl, 4-aminobutyl, methylaminomethyl, dimethylaminomethyl, methylaminoethyl, and dimethylaminoethyl.

The "carboxyalkyl" as used herein is a group in which one or more hydrogen atoms of the "alkyl" as defined herein are substituted with "carboxy". The carboxyalkyl is preferably a group in which one hydrogen atom of the alkyl is substituted with carboxy. The carboxyalkyl is, for example, carboxy $C_1$-$C_{20}$ alkyl, preferably carboxy $C_1$-$C_{15}$ alkyl, more preferably carboxy $C_1$-$C_{10}$ alkyl, most preferably carboxy $C_1$-$C_6$ alkyl. Specific examples of the carboxyalkyl include carboxymethyl and carboxyethyl.

The "alkenyloxycarbonylalkyl" as used herein is a group in which one or more hydrogen atoms of the "alkyl" as defined herein are substituted with "alkenyloxycarbonyl". The alkenyloxycarbonylalkyl is preferably a group in which one hydrogen atom of the alkyl is substituted with alkenyloxycarbonyl. The alkenyloxycarbonylalkyl is, for example, $C_2$-$C_{10}$ alkenyloxycarbonyl-$C_1$-$C_6$ alkyl, preferably $C_2$-$C_8$ alkenyloxycarbonyl-$C_1$-$C_6$ alkyl, more preferably $C_2$-$C_7$ alkenyloxycarbonyl-$C_1$-$C_6$ alkyl, most preferably $C_2$-$C_6$ alkenyloxycarbonyl-$C_1$-$C_6$ alkyl. Specific examples of the alkenyloxycarbonylalkyl include ethenyl (vinyl) oxycarbonylmethyl, 1-propenyloxycarbonylethyl, 2-propenyl (allyl) oxycarbonylmethyl, isopropenyloxycarbonylmethyl, 1-butenyloxycarbonylethyl, 2-cis-butenyloxycarbonylmethyl (including cis, trans), 2-trans-butenyloxycarbonylmethyl, 3-butenyloxycarbonylethyl, pentenyloxycarbonylmethyl, and hexenyloxycarbonylethyl.

The "alkoxyalkyl" as used herein is a group in which one or more hydrogen atoms of the "alkyl" as defined herein

are substituted with "alkoxy". The alkoxyalkyl is preferably a group in which one hydrogen atom of the alkyl is substituted with alkoxy. The alkoxyalkyl is, for example, $C_1$-$C_6$ alkoxy-$C_1$-$C_{20}$ alkyl, preferably $C_1$-$C_6$ alkoxy-$C_1$-$C_{15}$ alkyl, more preferably $C_1$-$C_6$ alkoxy-$C_1$-$C_{10}$ alkyl, most preferably $C_1$-$C_6$ alkoxy-$C_1$-$C_6$ alkyl. Specific examples of the alkoxyalkyl include methoxymethyl, ethoxymethyl, 1-propoxymethyl, 2-propoxymethyl, n-butoxymethyl, i-butoxymethyl, s-butoxymethyl, t-butoxymethyl, pentyloxymethyl, 3-methylbutoxymethyl, 1-methoxyethyl, 2-methoxyethyl, and 2-ethoxyethyl.

The "cycloalkylalkyl" as used herein is a group in which one or more hydrogen atoms of the "alkyl" as defined herein are substituted with "cycloalkyl". The cycloalkylalkyl is preferably a group in which one hydrogen atom of the alkyl is substituted with cycloalkyl. The cycloalkylalkyl is, for example, $C_3$-$C_{10}$ cycloalkyl-$C_1$-$C_{20}$ alkyl, preferably $C_3$-$C_{10}$ cycloalkyl-$C_1$-$C_6$ alkyl, more preferably $C_3$-$C_8$ cycloalkyl-$C_1$-$C_6$ alkyl, most preferably $C_3$-$C_6$ cycloalkyl-$C_1$-$C_2$ alkyl. Specific examples of the cycloalkylalkyl include cyclopropylmethyl, cyclobutylmethyl, cyclopentylmethyl, cyclohexylmethyl, cyclohexylethyl, and cyclohexylpropyl.

The "cycloalkoxyalkyl" as used herein is a group in which one or more hydrogen atoms of the "alkyl" as defined herein are substituted with "cycloalkoxy". The cycloalkoxyalkyl is preferably a group in which one hydrogen atom of the alkyl is substituted with cycloalkoxy. The cycloalkoxyalkyl is, for example, $C_3$-$C_{10}$ cycloalkoxy-$C_1$-$C_6$ alkyl, preferably $C_3$-$C_8$ cycloalkoxy-$C_1$-$C_6$ alkyl, more preferably $C_3$-$C_7$ cycloalkoxy-$C_1$-$C_6$ alkyl, most preferably $C_3$-$C_6$ cycloalkoxy-$C_1$-$C_6$ alkyl. Specific examples of the cycloalkoxyalkyl include cyclopropoxymethyl, cyclobutoxymethyl, and cyclopentyloxymethyl.

The "heterocyclylalkyl" as used herein is a group in which one or more hydrogen atoms of the "alkyl" as defined herein are substituted with "heterocyclyl". The heterocyclylalkyl is preferably a group in which one hydrogen atom of the alkyl is substituted with heterocyclyl. The heterocyclylalkyl is, for example, 3- to 14-membered heterocyclyl-$C_1$-$C_6$ alkyl, preferably 3- to 12-membered heterocyclyl-$C_1$-$C_6$ alkyl, more preferably 3- to 10-membered heterocyclyl-$C_1$-$C_4$ alkyl, most preferably 4-to 7-membered heterocyclyl-$C_1$-$C_3$ alkyl. Specific examples of the heterocyclylalkyl include azetidin-1-ylmethyl, oxetan-3-ylmethyl, 2-(tetrahydrofuran-3-yl)ethyl, (1-methylpyrrolidin-3-yl)methyl, 2-morpholinoethyl, 3-(1-piperidinyl)propyl, and 3-(4-methylpiperazin-1-yl)propyl.

The "alkylsulfonylalkyl" as used herein is a group in which one or more hydrogen atoms of the "alkyl" as defined herein are substituted with "alkylsulfonyl". The alkylsulfonylalkyl is preferably a group in which one hydrogen atom of the alkyl is substituted with alkylsulfonyl. The alkylsulfonylalkyl is, for example, $C_1$-$C_{20}$ alkylsulfonyl-$C_1$-$C_4$ alkyl, preferably $C_1$-$C_{10}$ alkylsulfonyl-$C_1$-$C_4$ alkyl, more preferably $C_1$-$C_8$ alkylsulfonyl-$C_1$-$C_4$ alkyl, most preferably $C_1$-$C_6$ alkylsulfonyl-$C_1$-$C_4$ alkyl. Specific examples of the alkylsulfonylalkyl include methylsulfonylmethyl, methylsulfonylpropyl, methylsulfonylbutyl, ethylsulfonylethyl, 1-propylsulfonylmethyl, 2-propylsulfonylethyl, n-butylsulfonylpropyl, i-butylsulfonylmethyl, s-butylsulfonylmethyl, t-butylsulfonylmethyl, pentylsulfonylmethyl, and 3-methylbutylsulfonylmethyl.

The "aminocarbonylalkyl" as used herein means a group in which one or more hydrogen atoms of the "alkyl" as defined herein are substituted with the "aminocarbonyl" as defined herein. The aminocarbonylalkyl is preferably a group in which one hydrogen atom of the alkyl is substituted with aminocarbonyl. The aminocarbonylalkyl is, for example, aminocarbonyl-$C_1$-$C_{10}$ alkyl, preferably aminocarbonyl-$C_1$-$C_6$ alkyl, more preferably aminocarbonyl-$C_1$-$C_4$ alkyl, most preferably aminocarbonyl-$C_1$-$C_2$ alkyl. Specific examples of the aminocarbonyl alkyl include $-CH_2CONH_2$, methylaminocarbonylmethyl, ethylaminocarbonylethyl, dimethylaminocarbonylpropyl, diethylaminocarbonylmethyl, 1-azetidinylcarbonylmethyl, 1-pyrrolidinylcarbonylethyl, 1-piperidinylcarbonylpropyl, 1-piperazinylcarbonylbutyl, 4-morpholinylcarbonylmethyl, and 3-oxazolidinylcarbonylethyl.

The "aryloxyalkyl" as used herein means a group in which one or more hydrogen atoms of the "alkyl" as defined herein are substituted with the "aryloxy" as defined herein. The aryloxyalkyl is preferably $C_6$-$C_{10}$ aryloxy-$C_1$-$C_6$ alkyl, more preferably $C_6$-$C_{10}$ aryloxy-$C_1$-$C_2$ alkyl. Specific examples of the aryloxyalkyl include phenoxymethyl and 2-phenoxyethyl.

The "aralkyl (arylalkyl)" as used herein is a group in which one or more hydrogen atoms of the "alkyl" as defined herein are substituted with the "aryl" as defined herein. The aralkyl is, for example, $C_7$-$C_{20}$ aralkyl, preferably $C_7$-$C_{18}$ aralkyl, more preferably $C_7$-$C_{16}$ aralkyl, most preferably $C_7$-$C_{14}$ aralkyl. The $C_7$-$C_{20}$ aralkyl is, for example, $C_6$-$C_{10}$ aryl-$C_1$-$C_{10}$ alkyl, preferably $C_6$-$C_{10}$ aryl-$C_1$-$C_8$ alkyl, more preferably $C_6$ aryl-$C_1$-$C_8$ alkyl or $C_{10}$ aryl-$C_1$-$C_8$ alkyl, most preferably $C_6$ aryl-$C_1$-$C_8$ alkyl. The $C_7$-$C_{18}$ aralkyl is, for example, $C_6$-$C_{10}$ aryl-$C_1$-$C_8$ alkyl, preferably $C_6$-$C_{10}$ aryl-$C_1$-$C_6$ alkyl, more preferably $C_6$ aryl-$C_1$-$C_6$ alkyl or $C_{10}$ aryl-$C_1$-$C_6$ alkyl, most preferably $C_6$ aryl-$C_1$-$C_6$ alkyl. The $C_7$-$C_{16}$ aralkyl is, for example, $C_6$-$C_{10}$ aryl-$C_1$-$C_6$ alkyl, preferably $C_6$-$C_{10}$ aryl-$C_1$-$C_4$ alkyl, more preferably $C_6$ aryl-$C_1$-$C_4$ alkyl or $C_{10}$ aryl-$C_1$-$C_4$ alkyl, most preferably $C_6$ aryl-$C_1$-$C_4$ alkyl. The $C_7$-$C_{14}$ aralkyl is, for example, $C_6$-$C_{10}$ aryl-$C_1$-$C_4$ alkyl, preferably $C_6$-$C_{10}$ aryl-$C_1$-$C_3$ alkyl, more preferably $C_6$ aryl-$C_1$-$C_3$ alkyl or $C_{10}$ aryl-$C_1$-$C_3$ alkyl, most preferably $C_6$ aryl-$C_1$-$C_3$ alkyl. Specific examples of the aralkyl include benzyl, phenethyl, and 3-phenylpropyl.

The "aralkoxy" as used herein is a group (-O-R-Ar, wherein R is alkylene) in which an alkyl moiety in "aralkyl" as defined herein is attached to an oxygen atom. The aralkoxy is, for example, $C_7$-$C_{20}$ aralkoxy, preferably $C_7$-$C_{18}$ aralkoxy, more preferably $C_7$-$C_{16}$ aralkoxy, most preferably $C_7$-$C_{14}$ aralkoxy. The $C_7$-$C_{20}$ aralkoxy is, for example, $C_6$-$C_{10}$ aryl-$C_1$-$C_{10}$ alkoxy, preferably $C_6$-$C_{10}$ aryl-$C_1$-$C_8$ alkoxy, more preferably $C_6$ aryl-$C_1$-$C_8$ alkoxy or $C_{10}$ aryl-$C_1$-$C_8$ alkoxy, most preferably $C_6$ aryl-$C_1$-$C_8$ alkoxy. The $C_7$-$C_{18}$ aralkoxy is, for example, $C_6$-$C_{10}$ aryl-$C_1$-$C_8$ alkoxy, preferably $C_6$-$C_{10}$ aryl-$C_1$-$C_6$ alkoxy, more preferably $C_6$ aryl-$C_1$-$C_6$ alkoxy or $C_{10}$ aryl-$C_1$-$C_6$ alkoxy, most preferably $C_6$ aryl-$C_1$-$C_6$ alkoxy. The

$C_7$-$C_{16}$ aralkoxy is, for example, $C_6$-$C_{10}$ aryl-$C_1$-$C_6$ alkoxy, preferably $C_6$-$C_{10}$ aryl-$C_1$-$C_4$ alkoxy, more preferably $C_6$ aryl-$C_1$-$C_4$ alkoxy or $C_{10}$ aryl-$C_1$-$C_4$ alkoxy, most preferably $C_6$ aryl-$C_1$-$C_4$ alkoxy. The $C_7$-$C_{14}$ aralkoxy is, for example, $C_6$-$C_{10}$ aryl-$C_1$-$C_4$ alkoxy, preferably $C_6$-$C_{10}$ aryl-$C_1$-$C_3$ alkoxy, more preferably $C_6$ aryl-$C_1$-$C_3$ alkoxy or $C_{10}$ aryl-$C_1$-$C_3$ alkoxy, most preferably $C_6$ aryl-$C_1$-$C_3$ alkoxy. Specific examples of the aralkoxy include benzyloxy, phenethyloxy, and 3-phenylpropoxy.

The "aralkoxyalkyl" as used herein means a group in which one or more hydrogen atoms of the "alkyl" as defined herein are substituted with the "aralkoxy" as defined herein. The aralkoxyalkyl is preferably $C_7$-$C_{14}$ aralkoxy-$C_1$-$C_6$ alkyl, more preferably $C_7$-$C_{14}$ aralkoxy-$C_1$-$C_2$ alkyl. Specific examples of the aralkoxyalkyl include benzyloxymethyl and 1-(benzyloxy)ethyl.

The "heteroarylalkyl" as used herein is a group in which one or more hydrogen atoms of the "alkyl" as defined herein are substituted with "heteroaryl". The heteroarylalkyl is preferably a group in which one hydrogen atom of the alkyl is substituted with heteroaryl. The heteroarylalkyl is, for example, 5- to 10-membered heteroaryl-$C_1$-$C_6$ alkyl, preferably 5- to 10-membered heteroaryl-$C_1$-$C_4$ alkyl, more preferably 5- to 10-membered heteroaryl-$C_1$-$C_3$ alkyl, most preferably 5- to 10-membered heteroaryl-$C_1$-$C_2$ alkyl. Specific examples of the heteroarylalkyl include 2-pyridylmethyl, 3-pyridylmethyl, 4-pyridylmethyl, 2-furanylmethyl, 2-thienylmethyl, 3-thienylmethyl, and 4-thiazolylmethyl.

The "heteroarylalkoxy" as used herein is a group in which an alkyl moiety in the "heteroarylalkyl" as defined herein is attached to an oxygen atom. The heteroarylalkoxy is, for example, 5- to 10-membered heteroaryl-$C_1$-$C_6$ alkoxy, preferably 5- to 10-membered heteroaryl-$C_1$-$C_4$ alkoxy, more preferably 5- to 10-membered heteroaryl-$C_1$-$C_3$ alkoxy, most preferably 5- to 10-membered heteroaryl-$C_1$-$C_2$ alkoxy. Specific examples of the heteroarylalkoxy include 2-pyridylmethoxy, 3-pyridylmethoxy, 4-pyridylmethoxy, 2-furanylmethoxy, 2-thienylmethoxy, 3-thienylmethoxy, and 4-thiazolylmethoxy.

The "heteroarylalkoxyalkyl" as used herein means a group in which one or more hydrogen atoms of the "alkyl" as defined herein are substituted with the "heteroarylalkoxy" as defined herein. The heteroarylalkoxyalkyl is preferably a group in which one hydrogen atom of the alkyl is substituted with heteroarylalkoxy. The heteroarylalkoxyalkyl is, for example, 5- to 10-membered heteroaryl-$C_1$-$C_6$ alkoxy-$C_1$-$C_6$ alkyl, preferably 5- to 10-membered heteroaryl-$C_1$-$C_4$ alkoxy-$C_1$-$C_6$ alkyl, more preferably 5- to 10-membered heteroaryl-$C_1$-$C_3$ alkoxy-$C_1$-$C_6$ alkyl, most preferably 5-to 10-membered heteroaryl-$C_1$-$C_2$ alkoxy-$C_1$-$C_6$ alkyl. Specific examples of the heteroarylalkoxyalkyl include 2-pyridylmethoxymethyl, 3-pyridylmethoxymethyl, 4-pyridylmethoxymethyl, 2-furanylmethoxymethyl, 2-thienylmethoxymethyl, 3-thienylmethoxymethyl, and 4-thiazolylmethoxymethyl.

The "heterocycloalkylidenealkyl" as used herein means a group in which one or more hydrogen atoms of the "alkyl" as defined herein are substituted with the "heterocycloalkylidene" as defined herein. The heterocycloalkylidenealkyl is preferably 4- to 7-membered heterocycloalkyliden-$C_1$-$C_6$ alkyl, more preferably 4- to 7-membered heterocycloalkyliden-$C_1$-$C_2$ alkyl. Specific examples of the heterocycloalkylidenealkyl include tetrahydro-4H-pyran-4-ylidenemethyl and azetidin-3-ylidenemethyl.

The "alkoxyalkenyl" as used herein is a group in which one or more hydrogen atoms of the "alkenyl" as defined herein are substituted with "alkoxy". The alkoxyalkenyl is preferably a group in which one hydrogen atom of the alkenyl is substituted with alkoxy. The alkoxyalkenyl is, for example, $C_1$-$C_6$ alkoxy-$C_2$-$C_{10}$ alkenyl, preferably $C_1$-$C_6$ alkoxy-$C_2$-$C_8$ alkenyl, more preferably $C_1$-$C_6$ alkoxy-$C_2$-$C_7$ alkenyl, most preferably $C_1$-$C_6$ alkoxy-$C_2$-$C_6$ alkenyl. Specific examples of the alkoxyalkenyl include (E)-4-methoxybut-2-en-1-yl.

The "aminocarbonylalkenyl" as used herein is a group in which one or more hydrogen atoms of the "alkenyl" as defined herein are substituted with "aminocarbonyl". The aminocarbonylalkenyl is preferably a group in which one hydrogen atom of the alkenyl is substituted with aminocarbonyl. The aminocarbonylalkenyl is, for example, aminocarbonyl-$C_2$-$C_{10}$ alkenyl, preferably aminocarbonyl-$C_2$-$C_8$ alkenyl, more preferably aminocarbonyl-$C_2$-$C_6$ alkenyl, most preferably aminocarbonyl-$C_2$-$C_4$ alkenyl. Specific examples of the aminocarbonylalkenyl include (E)-3-(dimethylaminocarbonyl)-prop-2-en-1-yl.

The "haloalkoxy" as used herein is a group in which one or more hydrogen atoms of the "alkoxy" as defined herein are substituted with halogen. The haloalkoxy is preferably a group in which one or more and six or less hydrogen atoms allowed to be substituted of the hydrogen atoms of the alkoxy are substituted with halogen. The haloalkoxy is, for example, halo-$C_1$-$C_{20}$ alkoxy, preferably halo-$C_1$-$C_{10}$ alkoxy, more preferably halo-$C_1$-$C_8$ alkoxy, most preferably halo-$C_1$-$C_6$ alkoxy. The halo-$C_1$-$C_6$ alkoxy is, for example, a group in which one or more and six or less hydrogen atoms allowed to be substituted of the hydrogen atoms of the alkoxy are substituted with fluorine, preferably a group in which one or more and five or less hydrogen atoms are substituted with fluorine, more preferably a group in which one or more and four or less hydrogen atoms are substituted with fluorine, most preferably a group in which one or more and three or less hydrogen atoms are substituted with fluorine. Specific examples of the haloalkoxy include difluoromethoxy, trifluoromethoxy, 2,2-difluoroethoxy, 2,2,2-trifluoroethoxy, 3-fluoropropoxy, and 2,2-difluoropropoxy.

The "alkylene" as used herein means a divalent group induced by the further removal of any one hydrogen atom from the "alkyl" described herein. The alkylene is preferably $C_1$-$C_8$ alkylene, more preferably $C_4$-$C_8$ alkylene. Examples of the alkylene specifically include $-CH_2-$, $-(CH_2)_2-$, $-(CH_2)_3-$, $-CH(CH_3)CH_2-$, $-C(CH_3)_2-$, $-(CH_2)_4-$, $-CH(CH_3)CH_2CH_2-$,

$-C(CH_3)_2CH_2-$, $-CH_2CH(CH_3)CH_2-$, $-CH_2C(CH_3)_2-$, $-CH_2CH_2CH(CH_3)-$, $-CH_2CH(CH_2CH_3)-$, $-(CH_2)_5-$, $-CH(CH_3)CH(CH_2CH_3)-$, $-(CH_2)_6-$, $-(CH_2)_7-$, and $-(CH_2)_8-$.

The "alicyclic ring" herein is a saturated or partially saturated non-aromatic hydrocarbon ring. A carbon atom constituting the ring may be oxidated to form carbonyl. The alicyclic ring may be selected from the group consisting of a monocyclic ring, a condensed ring, and a spiro ring. As used herein, an alicyclic ring containing a monocyclic ring is referred to as a monocyclic alicyclic ring; an alicyclic ring containing a condensed ring is referred to as a condensed cyclic alicyclic ring; and an alicyclic ring containing a spiro ring is referred to as a spirocyclic alicyclic ring. The alicyclic ring may form a condensed ring with a saturated alicyclic ring such as a cyclopentane ring or a cyclohexane ring; an unsaturated alicyclic ring such as a cycloheptene ring or a cyclohexene ring; or an aromatic hydrocarbon ring such as a benzene ring or a naphthalene ring. The alicyclic ring may form a spiro ring with a saturated alicyclic ring such as a cyclopropane ring, a cyclobutane ring, a cyclopentane ring, or a cyclohexane ring. The alicyclic ring is, for example, a $C_3$-$C_{10}$ alicyclic ring, preferably a $C_3$-$C_8$ alicyclic ring, more preferably a $C_3$-$C_7$ alicyclic ring, most preferably a $C_3$-$C_6$ alicyclic ring. Specific examples of the alicyclic ring include a cyclopropane ring, a cyclobutane ring, a cyclopentane ring, a cyclohexane ring, a cycloheptane ring, a cyclooctane ring, a bicyclo[2.2.1]heptane ring, and a cyclohexene ring.

The "saturated heterocycle" as used herein is a ring of the "heterocycle" that does not have an unsaturated bond in the ring. The number of atoms constituting the saturated heterocycle is from 3 to 14 (3- to 14-membered heterocycles), preferably from 3 to 12 (3- to 12-membered heterocycles), more preferably from 3 to 10 (3- to 10-membered heterocycles), most preferably from 4 to 7 (4- to 7-membered heterocycles). Specific examples of the saturated heterocycle include an azetidine ring, an epoxy ring, an oxetane ring, a thietane ring, a tetrahydrofuran ring, a pyrrolidine ring, a pyrazolidine ring, an imidazolidine ring, an oxazolidine ring, an isoxazolidine ring, a thiazolidine ring, an isothiazolidine ring, a thiadiazolidine ring, an oxazolidone ring, a dioxolan ring, a tetrahydropyran ring, a morpholine ring, a thiomorpholine ring, a 4-oxopyrrolidine rings, a piperidine ring, a 4-oxopiperidine ring, a piperazine ring, and a dioxane ring.

The "peptide compound" as used herein means a compound wherein two or more amino acids are linked by an amide bond. As long as two or more amino acids are linked by an amide bond, the peptide chain may contain another bond such as an ester bond or a thioester bond. The number of amino acid residues contained in the peptide is, for example, 5 to 30 residues, preferably 7 to 20 residues, more preferably 8 to 18 residues, most preferably 9 to 15 residues. The peptide may be linear, branched, or cyclic.

The "peptide chain" as used herein means a chain-like portion of a peptide in which two or more amino acids are linked by an amide bond. As long as two or more amino acids are linked by an amide bond, the peptide chain may contain another bond such as an ester bond or a thioester bond. The number of amino acid residues contained in the peptide chain is, for example, 5 to 30 residues, preferably 7 to 20 residues, more preferably 8 to 18 residues, most preferably 9 to 15 residues.

The "optionally substituted" as used herein means that a certain group and/or certain atom may be substituted with an optional substituent and/or optional atom. That is, either of the state in which the certain group and the certain atom are neither substituted by an optional substituent nor an optional atom, or the state in which the certain group and/or certain atom are substituted by an optional substituent and/or optional atom can be selected. Each of the certain group and/or certain atom may be further substituted with an optional substituent and/or optional atom. That is, either of the state in which the certain group and the certain atom have no substitutions of an optional substituent or an optional atom, or the state in which the certain group and/or certain atom have a substitution of an optional substituent and/or optional atom can be selected. The certain group and the optional substituent are not limited and may be, for example, freely selected from groups derived from atoms selected from the group consisting of a hydrogen atom, a halogen atom, a carbon atom, an oxygen atom, a sulfur atom, a nitrogen atom, a boron atom, a silicon atom, and a phosphorus atom. The certain atom and the optional atom are not limited and may be, for example, freely selected from a halogen atom, a carbon atom, an oxygen atom, a sulfur atom, a nitrogen atom, a boron atom, a silicon atom, and a phosphorus atom. Examples of the optional substituent include alkyl, alkoxy, fluoroalkyl, fluoroalkoxy, oxo, aminocarbonyl, alkylsulfonyl, alkylsulfonylamino, cycloalkyl, aryl, heteroaryl, heterocyclyl, arylalkyl, heteroarylalkyl, halogen, nitro, amino, monoalkylamino, dialkylamino, cyano, carboxyl, alkoxycarbonyl, and formyl.

The "optionally protected" as used herein means that a group may be protected with an optional protecting group.

The "one or more" as used herein means a number of 1 or 2 or larger. When "one or more" is used in a context related to a substituent for a certain group, this term means a number from 1 to the maximum number of substituents accepted by the group. Specific examples of the "one or more" include 1, 2, 3, 4, 5, 6, 7, 8, 9, 10, and/or larger numbers.

The compound described herein or a salt thereof can be a solvate thereof. Examples of the salt of the compound include hydrochloride; hydrobromide; hydroiodide; phosphate; phosphonate; sulfate; sulfonate such as methanesulfonate and p-toluenesulfonate; carboxylate such as acetate, citrate, malate, tartrate, succinate, and salicylate; alkali metal salts such as sodium salts and potassium salts; alkaline earth metal salts such as magnesium salt and calcium salt; and ammonium salts such as ammonium salt, alkylammonium salt, dialkylammonium salt, trialkylammonium salt, and tetraalkylammonium salt. These salts can be produced by, for example, bringing the compound into contact with an acid or a base. The solvate as used herein means one in which a compound and a solvent together form a molecular

aggregate, and is not particularly limited as long as it is a solvate formed by a solvent acceptable for ingestion along with administration of a medicament. Specific examples of the solvate include not only solvates formed with a single solvent such as water, alcohol (ethanol, methanol, 1-propanol, 2-propanol, or the like), or dimethyl sulfoxide, but also solvates formed with a plurality of solvents per one molecule of compound, or solvates formed with a plurality of types of solvents per one molecule of compound. For example, a solvate formed by a compound with water is called a hydrate. These solvates can be produced by, for example, bringing the compound into contact with a solvent. As the solvate of the compound of the present invention, the hydrate is preferred. Specifically, the hydrate is preferably 1 to 20 hydrates, more preferably 1 to 10 hydrates, further preferably 1 to 5 hydrates, most preferably 1 to 3 hydrates. The solvate of these salts can be produced by, for example, bringing the compound into contact with an acid or a base and a solvent.

The "amino acid" as used herein includes a natural amino acid and a non-natural amino acid (sometimes also referred to as an amino acid derivative). The "amino acid" as used herein may mean an amino acid residue. The "natural amino acid" as used herein is any L-type amino acid selected from Gly (glycine), L-Ala (alanine), L-Ser (serine), L-Thr (threonine), L-Val (valine), L-Leu (leucine), L-Ile (isoleucine), L-Phe (phenylalanine), L-Tyr (tyrosine), L-Trp (tryptophan), L-His (histidine), L-Glu (glutamic acid), L-Asp (aspartate), L-Gln (glutamine), L-Asn (asparagine), L-Cys (cysteine), L-Met (methionine), L-Lys (lysine), L-Arg (arginine), or L-Pro (proline). The "non-natural amino acid" as used herein is an amino acid other than the natural amino acid. Examples of the non-natural amino acid include a $\beta$-amino acid, a $\gamma$-amino acid, a D-type amino acid, an N-substituted amino acid (excluding Pro), an $\alpha,\alpha$-disubstituted amino acid, and an amino acid having a side chain different from that of natural amino acids. As the amino acid herein, amino acids having any conformation are acceptable. The side chains of the amino acids are not particularly limited, and each side chain is freely selected from groups such as alkyl, alkenyl, alkynyl, aryl, heteroaryl, aralkyl, heteroaralkyl, cycloalkyl, or spiro-bonded cycloalkyl, and/or atoms such as a hydrogen atom. Each group and/or atom is further optionally substituted. In one non-limiting aspect, the amino acid as used herein may be a compound having a carboxy group and an amino group in the same molecule. Even in this case, compounds in which the nitrogen atom of the amino group and any atom of a side chain of the amino acid together form a ring, such as proline, hydroxyproline, and azetidine-2-carboxylic acid, are also included in the amino acid.

The backbone amino group of the amino acid may be unsubstituted (i.e., an $NH_2$ group) or may be substituted (i.e., a -NHR group wherein R represents alkyl, alkenyl, alkynyl, aryl, heteroaryl, aralkyl, or cycloalkyl optionally having a substituent, and one or two non-adjacent methylene groups in any of these groups may be substituted by an oxygen atom, a carbonyl group (-CO-), or a sulfonyl group ($-SO_2-$); and a carbon chain bonded to an N atom and a carbon atom at position $\alpha$ may form a ring, as in proline). The substituent of the R is selected in the same manner as in the substituent for the amino acid side chain mentioned above. In the case of a substituted backbone amino group, the R is present in the "side chain of the amino acid" as used herein. Such an amino acid having the substituted backbone amino group is referred to herein as the "N-substituted amino acid". Examples of the "N-substituted amino acid" as used herein preferably include, but are not limited to, N-alkyl amino acids, N-$C_1$-$C_6$ alkyl amino acids, N-$C_1$-$C_4$ alkyl amino acids, and N-methyl amino acids.

The "amino acid" as used herein includes all isotopes corresponding to each. The isotope of the "amino acid" is a form in which at least one atom is replaced with an atom having the same atomic number (proton number) and a different mass number (total number of protons and neutrons) at an abundance ratio different from the natural abundance ratio. Examples of the isotope contained in the "amino acid" herein include a hydrogen atom, a carbon atom, a nitrogen atom, an oxygen atom, a phosphorus atom, a sulfur atom, a fluorine atom, and a chlorine atom, and they include $^{2}H$, $^{3}H$, $^{13}C$, $^{14}C$, $^{15}N$, $^{17}O$, $^{18}O$, $^{32}P$, $^{35}S$, $^{18}F$, $^{36}Cl$, and the like, respectively. For the compounds as used herein, all the compounds containing any proportions of radioactive or non-radioactive isotopic elements are encompassed within the scope of the present invention.

Examples of the "halogen-derived substituent" as used herein include fluoro (-F), chloro (-Cl), bromo (-Br), and iodo (-I).

Examples of the "oxygen atom-derived substituent" as used herein include hydroxy (-OH), oxy (-OR), oxo (=O), carbonyl (-C(=O)-R), carboxy ($-CO_2H$), oxycarbonyl (-C(=O)-OR), carbonyloxy (-O-C(=O)-R), thiocarbonyl (-C(=O)-SR), carbonylthio (-S-C(=O)-R), aminocarbonyl (-C(=O)-NHR), carbonylamino (-NH-C(=O)-R), oxycarbonylamino (-NH-C(=O)-OR), sulfonylamino ($-NH-SO_2-R$), aminosulfonyl ($-SO_2-NHR$), sulfamoylamino ($-NH-SO_2-NHR$), thiocarboxy (-C(=O)-SH), and carboxycarbonyl ($-C(=O)-CO_2H$).

Examples of the oxy (-OR) include alkoxy, cycloalkoxy, alkenyloxy, alkynyloxy, aryloxy, heteroaryloxy, and aralkyloxy. The alkoxy is preferably $C_1$-$C_4$ alkoxy or $C_1$-$C_2$ alkoxy, particularly preferably methoxy or ethoxy.

Examples of the carbonyl (-C(=O)-R) include formyl (-C(=O)-H), alkylcarbonyl, cycloalkylcarbonyl, alkenylcarbonyl, alkynylcarbonyl, arylcarbonyl, heteroarylcarbonyl, and aralkylcarbonyl.

Examples of the oxycarbonyl (-C(=O)-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of the carbonyloxy (-O-C(=O)-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of the thiocarbonyl (-C(=O)-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of the carbonylthio (-S-C(=O)-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of the aminocarbonyl (-C(=O)-NHR) include alkylaminocarbonyl (e.g., $C_1$-$C_6$ or $C_1$-$C_4$ alkylaminocarbonyl, specifically, ethylaminocarbonyl or methylaminocarbonyl), cycloalkylaminocarbonyl, alkenylaminocarbonyl, alkynylaminocarbonyl, arylaminocarbonyl, heteroarylaminocarbonyl, and aralkylaminocarbonyl. Additional examples thereof include groups in which the H atom bonded to the N atom in -C(=O)-NHR is further substituted with alkyl, cycloalkyl, alkenyl, alkynyl, aryl, heteroaryl, or aralkyl.

Examples of the carbonylamino (-NH-C(=O)-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Additional examples thereof include groups in which the H atom bonded to the N atom in -NH-C(=O)-R is further substituted with alkyl, cycloalkyl, alkenyl, alkynyl, aryl, heteroaryl, or aralkyl.

Examples of the oxycarbonylamino (-NH-C(=O)-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. Additional examples thereof include groups in which the H atom bonded to the N atom in -NH-C(=O)-OR is further substituted with alkyl, cycloalkyl, alkenyl, alkynyl, aryl, heteroaryl, or aralkyl.

Examples of the sulfonylamino (-NH-$SO_2$-R) include alkylsulfonylamino, cycloalkylsulfonylamino, alkenylsulfonylamino, alkynylsulfonylamino, arylsulfonylamino, heteroarylsulfonylamino, and aralkylsulfonylamino. Additional examples thereof include groups in which the H atom bonded to the N atom in -NH-$SO_2$-R is further substituted with alkyl, cycloalkyl, alkenyl, alkynyl, aryl, heteroaryl, or aralkyl.

Examples of the aminosulfonyl (-$SO_2$-NHR) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Additional examples thereof include groups in which the H atom bonded to the N atom in -$SO_2$-NHR is further substituted with alkyl, cycloalkyl, alkenyl, alkynyl, aryl, heteroaryl, or aralkyl.

Examples of the sulfamoylamino (-NH-$SO_2$-NHR) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. Further, the two H atoms bonded to the N atom in -NH-$SO_2$-NHR is optionally substituted with substituents independently selected from the group consisting of alkyl, cycloalkyl, alkenyl, alkynyl, aryl, heteroaryl, and aralkyl, and the two substituents may form a ring.

Examples of the "sulfur atom-derived substituent" as used herein include thiol (-SH), thio (-S-R), sulfinyl (-S=O-R), sulfonyl (-$S(O)_2$-R), sulfo (-$SO_3H$), pentafluorosulfanyl (-$SF_5$), and disulfanyl (-S-S-R).

Examples of the thio (-S-R) include alkylthio, cycloalkylthio, alkenylthio, alkynylthio, arylthio, heteroarylthio, and aralkylthio.

Examples of the sulfinyl (-S=O-R) include alkylsulfinyl, cycloalkylsulfinyl, alkenylsulfinyl, alkynylsulfinyl, arylsulfinyl, heteroarylsulfinyl, and aralkylsulfinyl.

Examples of the sulfonyl (-$S(O)_2$-R) include alkylsulfonyl, cycloalkylsulfonyl, alkenylsulfonyl, alkynylsulfonyl, arylsulfonyl, heteroarylsulfonyl, and aralkylsulfonyl.

Examples of the "nitrogen atom-derived substituent" as used herein include azide (-$N_3$, also referred to as "azide group"), cyano (-CN), primary amino (-$NH_2$), secondary amino (-NH-R), tertiary amino (-NR(R')), amidino (-C(=NH)-$NH_2$), substituted amidino (-C(=NR)-NR'R"), guanidino (-NH-C(=NH)-$NH_2$), substituted guanidino (-NR-C(=NR‴)-NR'R"), and aminocarbonylamino (-NR-CO-NR'R").

Examples of the secondary amino (-NH-R) include alkylamino, cycloalkylamino, alkenylamino, alkynylamino, arylamino, heteroarylamino, and aralkylamino.

Examples of the tertiary amino (-NR(R')) include an amino group having optional two substituents each independently selected from alkyl, cycloalkyl, alkenyl, alkynyl, aryl, heteroaryl, aralkyl, and the like, such as alkyl(aralkyl)amino, and the optional two substituents may form a ring.

Examples of the substituted amidino (-C(=NR)-NR'R") include groups in which three substituents R, R', and R" on the N atoms are each independently selected from alkyl, cycloalkyl, alkenyl, alkynyl, aryl, heteroaryl, and aralkyl, for example, alkyl(aralkyl)(aryl)amidino.

Examples of the substituted guanidino (-NR-C(=NR‴)-NR'R") include groups in which R,R', R", and R‴ are each independently selected from alkyl, cycloalkyl, alkenyl, alkynyl, aryl, heteroaryl, and aralkyl, and groups in which these substituents form a ring.

Examples of the aminocarbonylamino (-NR-CO-NR'R") include groups in which R, R', and R" are each independently selected from a hydrogen atom, alkyl, cycloalkyl, alkenyl, alkynyl, aryl, heteroaryl, and aralkyl, and groups in which these substituents form a ring.

As used herein, the "amino acid residue" forming a peptide compound is sometimes referred to simply as an "amino acid".

As used herein, the "to" that indicates a numerical range includes values of both ends thereof. For example, "A to B" means the numerical range of A or more and B or less.

As used herein, the term "approximately", when used in combination with a numeric value, means the value range of +10% and -10% of the numeric value.

As used herein, the term "and/or" is meant to include every combination of the terms "and" and "or" appropriately combined. Specifically, for example, the term "A, B, and/or C" includes the following seven variations: (i) A, (ii) B, (iii) C, (iv) A and B, (v) A and C, (vi) B and C, and (vii) A, B, and C.

Method for producing peptide compound

In an aspect, the present invention relates to a method for producing a peptide compound or a salt thereof, and the method includes a step (linking step) of linking an amino group of a first amino acid or peptide and a carboxy group of a second amino acid or peptide with an amide bond in a two-layer solvent containing water and one or more organic solvents that are not miscible with water.

In an aspect, the present invention relates to a method for producing a peptide compound or a salt thereof, and the method includes a step (linking step) of linking an amino group of a first amino acid or peptide and a carboxy group of a second amino acid or peptide with an amide bond in a two-layer solvent containing water and one or more organic solvents that are not miscible with water. In this aspect, the amino group of the first amino acid or peptide is an amino group substituted with a hydrogen atom, a linear $C_1$-$C_6$ alkyl, a branched $C_3$-$C_6$ alkyl, or a $C_3$-$C_8$ cycloalkyl.

In an embodiment, the linking step is carried out in the presence of an activator. Examples of the activator include acyl halide, phosphoric acid halide, phosphinic acid halide, carbodiimide, uronium, and phosphonium. The acyl halide is preferably linear $C_1$-$C_{18}$ alkylcarbonyl chloride, branched $C_3$-$C_{18}$ alkylcarbonyl chloride, linear $C_1$-$C_{18}$ alkylcarbonyl bromide, branched $C_3$-$C_{18}$ alkylcarbonyl bromide, or monocyclic or condensed cyclic $C_3$-$C_{15}$ cycloalkylcarbonyl chloride, wherein the $C_3$-$C_{15}$ cycloalkylcarbonyl chloride is optionally substituted with a $C_1$-$C_6$ alkyl, more preferably pivaloyl chloride, 2,2-dimethylbutyryl chloride, 1-methylcyclohexanecarbonyl chloride, 1-adamantanecarbonyl chloride, 2-ethylbutyryl chloride, further preferably pivaloyl chloride, or 2,2-dimethylbutyryl chloride, most preferably pivaloyl chloride. Examples of the phosphoric acid halide include diethyl phosphoryl chloride and diphenyl phosphoryl chloride. Examples of the phosphinic acid halide include diphenyl phosphine chloride. Examples of the carbodiimide include N,N-diisopropylcarbodiimide, N,N-dicyclohexylcarbodiimide, and 1-ethyl-3-(3-dimethylaminopropyl)carbodiimide hydrochloride. Examples of the uronium include HATU and COMU. Examples of the phosphonium include PyFOP.

In an embodiment, the linking step is carried out in the presence of an additive. Preferably, step (1) in the linking step is carried out in the presence of an additive. Examples of the additive include a bromide salt, an iodide salt, and a trifluoromethanesulfonate salt. Examples of the bromide salt include sodium bromide, potassium bromide, magnesium bromide, calcium bromide, and barium bromide. Examples of the iodide salt include sodium iodide, potassium iodide, magnesium iodide, calcium iodide, and barium iodide. Examples of the trifluoromethanesulfonate salt include sodium trifluoromethanesulfonate, potassium trifluoromethanesulfonate, magnesium trifluoromethanesulfonate, calcium trifluoromethanesulfonate, and barium trifluoromethanesulfonate. The additive is preferably at least one selected from the group consisting of sodium bromide, sodium iodide, sodium trifluoromethanesulfonate, and potassium trifluoromethanesulfonate. The molar equivalent of the additive used relative to the second amino acid or peptide is in a range from 0.2 molar equivalents to 3.0 molar equivalents, preferably from 0.3 molar equivalents to 1.5 molar equivalents relative to the second amino acid or peptide.

In an aspect, the linking step can be carried out, for example, by stirring the reaction mixture for 10 minutes to 24 hours at a reaction temperature of -50°C to 30°C, preferably -20°C to 10°C, further preferably -18°C to 0°C, most preferably -18°C to - 5°C.

In an embodiment, the linking step includes the steps of (1) bringing the second amino acid or peptide into contact with an acyl halide in an organic solvent to prepare a mixed acid anhydride; and (2) bringing the mixed acid anhydride obtained in step (1) into contact with the first amino acid or peptide in the presence of a base in the two-layer solvent containing water and one or more organic solvents that are not miscible with water. Preferably, (2) of the linking step includes a step of adding dropwise the first amino acid or peptide to the mixed acid anhydride obtained in step (1) in the presence of a base.

In an aspect, step (1) in the linking step can be carried out, for example, by stirring the reaction mixture for 75 minutes to 7 hours at a reaction temperature of -50°C to 30°C, preferably -20°C to 10°C, further preferably -18°C to 0°C, most preferably - 18°C to -5°C.

In an aspect, step (2) in the linking step can be carried out, for example, by stirring the reaction mixture for 10 minutes to 24 hours at a reaction temperature of - 50°C to 30°C, preferably -20°C to 10°C, further preferably -18°C to 0°C, most preferably -18°C to -5°C.

The "two-layer solvent" as used herein means a solvent in which water and an organic solvent that is not miscible with water form an interface, and the interface is visible. Depending on the organic solvent that is not miscible with water, the organic solvent may become the underlying layer, or the water may become the underlying layer.

In an embodiment, the organic solvent that is not miscible with water includes, but is not intended to be particularly

limited to, an organic solvent having low water solubility (e.g., having a solubility in water of 200 g/L or less, preferably 150 g/L or less). The organic solvent that is not miscible with water may contain a trace amount of, for example, 0.01 wt% or less, other organic solvents that are miscible with water. The solubility in water can be determined by any method known in the art or described herein. Examples of the method for determining solubility include, but are not intended to be particularly limited to, gas chromatography, and the solubility can be determined by measuring the concentration of the organic solvent in water prepared by mixing the organic solvent with the same volume of water at room temperature (e.g., 15°C to 40°C, preferably 20°C to 30°C).

For determining the miscibility of an organic solvent with water, the miscibility can also be shown by the separation of the solvent and water into two layers when the same volume of the solvent and water are mixed in a vessel, for example, at room temperature (e.g., 15°C to 40°C, preferably 20°C to 30°C). Whether the solvent and water separate into two layers can be determined, for example, by checking visually, or by collecting and checking the liquids in the upper and lower layers in the vessel. When it is confirmed that the solvent and water are separated into two layers in this way, the solvent may be referred to as a solvent that is not miscible with water. However, even a solvent that is miscible with water, depending on the solute in the solvent and the salt concentration in water, the solvent may form an interface with water and separate into two layers.

In an embodiment, the organic solvent that is not miscible with water is a solvent having an octanol/water partition coefficient (Log Kow) of 5 or more, or a solvent having an octanol/water partition coefficient (Log Kow) predicted value of 5 or more. The octanol/water partition coefficient (Log Kow) can be determined by any method known in the art or described herein. The octanol/water partition coefficient (Log Kow) prediction value may be determined by known means in separate explicit measurements by, for example, but not particularly limited to, database search or literature search. Examples of the method for measuring the octanol/water partition coefficient include, but are not limited to, the method in accordance with the Japanese Industrial Standard JIS 7260-107: 2000 Measurement of Partition coefficient (1-octanol/water) Shake flask method (https://kikakurui.com/z7/Z7260-107-2000-01.html [access date: September 9, 2024]).

In an embodiment, the organic solvent that is not miscible with water include an ester-based solvent, an ether-based solvent, a halogenated hydrocarbon-based solvent, an aromatic hydrocarbon-based solvent, and the like. Examples of the ester-based solvent include isopropyl acetate, ethyl acetate, and butyl acetate. Examples of the ether-based solvent include methyl t-butyl ether and diethyl ether. Examples of the halogenated hydrocarbon-based solvent include dichloromethane and carbon tetrachloride. Examples of the aromatic hydrocarbon-based solvent include toluene and hexane. Among these, isopropyl acetate, methyl t-butyl ether, dichloromethane, 2-methyltetrahydrofuran, and toluene are preferred.

In an embodiment, the ratio of water to the one or more organic solvents that are not miscible with water in the linking step is, for example, from 0.001 to 10,000, preferably from 0.01 to 1,000, further preferably from 0.1 to 100, most preferably from 1 to 10 by weight ratio.

In an embodiment, the organic solvent used in step (1) in the linking step is not particularly limited unless it inhibits the production of the mixed acid anhydride, but is preferably an organic solvent that is not miscible with water. Solvents used in step (1) include an ester-based solvent, an ether-based solvent, an amide-based solvent, and a nitrile-based solvent. Examples of the ester-based solvent include isopropyl acetate, ethyl acetate, and butyl acetate. Examples of the ether-based solvent include tetrahydrofuran, 2-methyltetrahydrofuran, and diethyl ether. Examples of the amide-based solvent include N,N-dimethylformamide, N,N-dimethylacetamide, and N,N-diethylformamide. Examples of the nitrile-based solvent include acetonitrile and propionitrile. Among these, isopropyl acetate, tetrahydrofuran, 2-methyltetrahydrofuran, N,N-dimethylformamide, and acetonitrile are preferred.

In an embodiment, the organic solvent used in step (1) in the linking step is used at a concentration at which the second amino acid or peptide is dissolved. The concentration of the second amino acid or peptide used is, for example, 0.1 to 300 mg/mL, preferably 1 to 200 mg/mL, further preferably 10 to 200 mg/mL, most preferably 20 to 200 mg/mL.

In certain embodiments, organic solvents that may have the potential to react with mixed acid anhydrides or peptide compounds can be excluded from the organic solvent that is not miscible with water in the present invention. Examples of the organic solvents that are not suitable for the organic solvent that is not miscible with water include an amine-based solvent (e.g., n-propylamine or diisopropylamine) and an alcohol-based solvent (e.g., methanol, ethanol, n-propanol, or phenol).

In an embodiment, in step (2) in the linking step, a mixture of the mixed acid anhydride obtained in step (1) and an organic solvent is preferably used.

The "bring into contact with" as used herein means bringing a solid phase into contact with a liquid phase in a solid phase-liquid phase synthesis method, mixing in a liquid-liquid phase synthesis method, adding, dropping, and the like.

As described herein, step (2) in the linking step of the method of the present invention is carried out in a two-layer system containing water and one or more solvents that are not miscible with water. Thus, it may be useful in terms that (a) a solvent that is not miscible with water can be used as an extraction solvent in the post-treatment of the reaction mixture, (b) an extraction solution containing a peptide compound can be used as a starting material for a subsequent step/reaction (i.e., a solution containing a starting compound for a subsequent step), and the like. In an embodiment, the method of the

present invention enables the entire reaction to be carried out and completed without isolating the intermediate from the solvent of the starting reaction.

In an embodiment, the acyl halide is a linear $C_1$-$C_{18}$ alkylcarbonyl chloride, a branched $C_3$-$C_{18}$ alkylcarbonyl chloride, a linear $C_1$-$C_{18}$ alkylcarbonyl bromide, a branched $C_3$-$C_{18}$ alkylcarbonyl bromide, or a monocyclic or condensed cyclic $C_3$-$C_{15}$ cycloalkylcarbonyl chloride, in which the $C_3$-$C_{15}$ cycloalkylcarbonyl chloride is optionally substituted with a $C_1$-$C_6$ alkyl. Among these, the acyl halide is preferably pivaloyl chloride, 2,2-dimethylbutyryl chloride, 1-methylcyclohexanecarbonyl chloride, 1-adamantanecarbonyl chloride, or 2-ethylbutyryl chloride, more preferably pivaloyl chloride or 2,2-dimethylbutyryl chloride, further preferably pivaloyl chloride or 2,2-dimethylbutyryl chloride, most preferably pivaloyl chloride.

In an embodiment, the acyl halide is used at, for example, 0.01 to 100 molar equivalents, preferably 0.1 to 10 molar equivalents, further preferably 0.5 to 1.0 molar equivalents, most preferably 0.8 to 1.0 molar equivalents, with respect to the second amino acid or peptide.

In an embodiment, step (1) in the linking step is carried out in the presence of a base.

In an embodiment, the base used in step (1) in the linking step has pKa of the conjugate acid of 15.0 or less, preferably 13.0 or less, further preferably 11.5 or less. In an embodiment, the base used in step (1) in the linking step has pKa of the conjugate acid of 0 or more, preferably 3 or more, further preferably 5 or more. In an embodiment, the base used in step (1) in the linking step has pKa of the conjugate acid of 0 to 15.0, preferably 0 to 11.5, more preferably 3 to 11.5, further preferably 5 to 11.5, most preferably 6 to 11.5. The "pKa" as used herein can be measured using water as a solvent. As the pKa used herein, measured values that have already been reported as pKa when using water as a solvent may be employed. When the measured value of pKa is not available, pKa may be calculated with ADMETPredictor (Simulations Plus Inc., ver8.0) and employed as pKa herein. Listed below are pKa of representative reagents:

Conjugate acid of DBU (pKa = 11.9; R. Srivastava, J. Mol. Catal. A: Chem. 264 (2007) 146-152);
Conjugate acid of piperidine (pKa = 11.22; Hall, H.K., Jr. J. A.m. Chem. Soc. 1957, 79, 5441);
Conjugate acid of triethylamine (pKa = 10.65; Hall, H.K., Jr. J. A.m. Chem. Soc. 1957, 79, 5441);
Conjugate acid of diisopropylethylamine (pKa = 11.44; Chemical and Pharmaceutical Bulletin, 1995, 43, 1872-1877);
Conjugate acid of 2,4,6-collidine (pKa = 7.48; Clarke, K., Rothwell, K. J. Chem. Soc. 1960, 1885);
Conjugate acid of 2,6-lutidine (pKa = 6.77; Clarke, K., Rothwell, K. J. Chem. Soc. 1960, 1885);
Conjugate acid of pyridine (pKa = 5.21; D.H. Ripin, D.A. Evans, pKa's of Nitrogen Acids,[online], [searched on 22 September 2023], internet <URL:http://evans.rc.fas.harvard.edu/pdf/evans_pKa_table.pdf>)

In an embodiment, the base used in step (1) in the linking step is an organic base, preferably at least one selected from the group consisting of amines and pyridines. Examples of the amines include diisopropylethylamine and triethylamine. Examples of the pyridines include 2,6-lutidine and 2,4,6-collidine. The base used in step (1) in the linking step is preferably one selected from the group consisting of diisopropylethylamine, triethylamine, 2,6-lutidine, and 2,4,6-collidine, more preferably one selected from the group consisting of triethylamine, 2,6-lutidine, and 2,4,6-collidine, most preferably 2,6-lutidine.

In an embodiment, the base used in step (1) in the linking step is used at, for example, 0.5 to 100 molar equivalents, preferably 0.8 to 10 molar equivalents, further preferably 0.9 to 5 molar equivalents, most preferably 1.0 to 1.3 molar equivalents relative to the second amino acid or peptide.

In an embodiment, the base used in step (2) in the linking step is not particularly limited, but is preferably an organic base or an inorganic base, more preferably at least one selected from the group consisting of sodium carbonate, potassium carbonate, cesium carbonate, potassium phosphate, sodium hydrogen carbonate, sodium hydrogen phosphate, triethylamine, and pyridine, further preferably one selected from the group consisting of sodium carbonate, potassium carbonate, cesium carbonate, and potassium phosphate, most preferably one selected from the group consisting of sodium carbonate and potassium carbonate.

The "first amino acid" and "second amino acid" as used herein are terms used only to distinguish reaction points between amino acids, which are the substrates in the linking step. Specifically, the "first amino acid" means an amino acid in which the amino group is the reaction point, and the "second amino acid" means an amino acid in which the carboxy group is the reaction point.

In an embodiment, the first amino acid or peptide is a peptide containing two or more amino acid residues.

In an embodiment, the amino group of the first amino acid or peptide is an amino group represented by formula: $-NR^4R^5$, wherein $R^4$ is a hydrogen atom, and $R^5$ is a linear $C_1$-$C_6$ alkyl, a branched $C_3$-$C_6$ alkyl, or a $C_3$-$C_8$ cycloalkyl. In certain embodiments, the amino group of the first amino acid or peptide is an amino group represented by formula: $-NR^4R^5$, wherein $R^4$ is a hydrogen atom, and $R^5$ is a hydrogen atom or a linear $C_1$-$C_4$ alkyl. In certain embodiments, the amino group of the first amino acid or peptide is an amino group represented by formula: $-NR^4R^5$, wherein $R^4$ is a hydrogen atom, and $R^5$ is a hydrogen atom, methyl or ethyl. In certain embodiments, the amino group of the first amino acid or peptide is an amino group represented by formula: $-NR^4R^5$, wherein $R^4$ is a hydrogen atom, and $R^5$ is a hydrogen atom or methyl. In certain

embodiments, the amino group of the first amino acid or peptide is an amino group represented by formula: $-NR^4R^5$, wherein $R^4$ is a hydrogen atom, and $R^5$ is methyl.

In an embodiment, the carboxy group of the first amino acid or peptide is optionally protected with a protecting group. Any protecting group known in the art can be used as the protecting group for the carboxy group as long as it does not reduce the solubility of the peptide in the solvent. Specific examples of such a protecting group for the carboxy group include a methyl group, an ethyl group, a t-butyl group, a trityl group, and a cumyl group, and among these, a t-butyl group is preferred.

In an embodiment, the second amino acid or peptide is a peptide containing two or more amino acid residues.

In an embodiment, the $\alpha$-position carbon of the carboxy group of the second amino acid or peptide is optionally substituted. In an embodiment, the $\alpha$-position carbon of the carboxy group of the second amino acid or peptide is represented by formula: $-CR^1R^2-$, wherein $R^1$ and $R^2$ are identical or different and each is a hydrogen atom, a linear $C_1$-$C_6$ alkyl, an optionally substituted branched $C_3$-$C_6$ alkyl, an optionally substituted $C_3$-$C_8$ cycloalkyl-$C_1$-$C_6$ alkyl, an optionally substituted $C_3$-$C_8$ cycloalkyl, an optionally substituted phenyl-$C_1$-$C_2$ alkyl, an optionally substituted $C_1$-$C_6$ alkoxy-$C_1$-$C_2$ alkyl, an optionally substituted 5- to 6-membered heteroaryl-$C_1$-$C_2$ alkyl, or an optionally substituted phenyl-$C_1$-$C_2$ alkylthio-$C_1$-$C_2$ alkyl, or $R^1$ and $R^2$ together with the carbon atom to which they are attached form a $C_3$-$C_8$ saturated alicyclic ring.

When $R^1$ or $R^2$ is a branched $C_1$-$C_6$ alkyl, the branched $C_1$-$C_6$ alkyl is optionally substituted with one or more halogens.

When $R^1$ or $R^2$ is $C_3$-$C_8$ cycloalkyl-$C_1$-$C_6$ alkyl, the $C_3$-$C_8$ cycloalkyl-$C_1$-$C_6$ alkyl is optionally substituted with one or more selected from the group consisting of methyl, ethyl, propyl, halogen, methoxy, ethoxy, and trifluoromethyl.

When $R^1$ or $R^2$ is $C_3$-$C_8$ cycloalkyl, the $C_3$-$C_8$ cycloalkyl is optionally substituted with one or more selected from the group consisting of methyl, ethyl, propyl, halogen, methoxy, ethoxy and trifluoromethyl.

When $R^1$ or $R^2$ is phenyl-$C_1$-$C_2$ alkyl, the phenyl-$C_1$-$C_2$ alkyl is optionally substituted with one or more selected from the group consisting of methyl, ethyl, propyl, halogen, methoxy, ethoxy and trifluoromethyl.

When $R^1$ or $R^2$ is $C_1$-$C_6$ alkoxy-$C_1$-$C_2$ alkyl, the $C_1$-$C_6$ alkoxy-$C_1$-$C_2$ alkyl is optionally substituted with one or more selected from the group consisting of methyl, ethyl, propyl, halogen, methoxy, ethoxy and trifluoromethyl.

When $R^1$ or $R^2$ is 5- to 6-membered heteroaryl-$C_1$-$C_2$ alkyl, the 5- to 6-membered heteroaryl-$C_1$-$C_2$ alkyl is optionally substituted with one or more selected from the group consisting of methyl, ethyl, propyl, halogen, methoxy, ethoxy and trifluoromethyl.

In certain embodiments, the $\alpha$-position carbon of the carboxy group of the second amino acid or peptide is represented by formula: $-CR^1R^2-$, wherein $R^1$ and $R^2$ are identical or different and each is a hydrogen atom, a linear $C_1$-$C_3$ alkyl, a branched $C_3$-$C_6$ alkyl, a $C_3$-$C_6$ cycloalkyl-$C_1$-$C_6$ alkyl, a $C_1$-$C_6$ alkoxymethyl, a 1-($C_1$-$C_6$ alkoxy)ethyl, an optionally substituted benzyl, an optionally substituted phenethyl, or a phenyl-$C_1$-$C_2$ alkylthiomethyl.

When $R^1$ or $R^2$ is benzyl or phenethyl, the benzyl or phenethyl is optionally substituted with one or more selected from the group consisting of fluorine, methyl, ethyl, methoxy, ethoxy, and trifluoromethyl.

In certain embodiments, the $\alpha$-position carbon of the carboxy group of the second amino acid or peptide is represented by formula: $-CR^1R^2-$, wherein $R^1$ is a hydrogen atom, and $R^2$ is a hydrogen atom, methyl, 1-methylethyl, 1-methylpropyl, t-butoxymethyl, 1-(t-butoxy)ethyl, benzyl, 2-(4-trifluoromethyl-3,5-difluorophenyl)ethyl, or benzylthiomethyl.

In an embodiment, the $\beta$-position nitrogen of the carboxy group of the second amino acid or peptide is represented by formula: $-NR^3-$, wherein $R^3$ is a hydrogen atom.

In an embodiment, the amino group of the second amino acid or peptide is optionally protected with a protecting group. Any protecting group known in the art can be used as the protecting group for the amino group as long as it does not reduce the solubility of the peptide in the solvent. Specific examples of such a protecting group for the amino group include Cbz, p-nitrobenzyloxycarbonyl, 2-naphthylmethyloxycarbonyl, diphenylmethyloxycarbonyl, 9-anthrylmethyloxycarbonyl, Teoc, Fmoc, Boc, Alloc, and trifluoroacetyl, and among these, Cbz, Teoc, and trifluoroacetyl are preferred.

In an embodiment, a peptide compound or a salt thereof produced by the method of the present invention contains amino acid residues of 8 to 20 residues, preferably 11 to 14 residues, more preferably 11 to 13 residues, most preferably 11 residues.

In an aspect, a peptide compound or a salt thereof produced by the method of the present invention can contain at least one, at least two, at least three, at least four, or at least five non-natural amino acid residues. In certain embodiments, the non-natural amino acid residues contained in the peptide compound or a salt thereof produced by the method of the present invention are N-methylamino acid residues.

In an aspect, the peptide compound produced by the method of the present invention may be a linear peptide compound. In another aspect, the peptide compound produced by the method of the present invention may be a cyclic peptide compound. In an aspect, the linear or cyclic peptide compound may comprise a cyclic structure (cyclic portion) as its moiety structure. Specific examples of the cyclic structure include those in which the side chain of one amino acid residue is linked to a side chain of another amino acid residue, those in which the N-substituent of one amino acid residue is

linked to the side chain of another amino acid residue, and those in which the N-substituent of one amino acid residue is linked to the N-substituent of another amino acid residue. The two amino acid residues involved in the linkage for the cyclic structure may be adjacent, or any number of amino acid residues, for example, any of amino acid residues selected from the group consisting of 1, 2, 3, 4, 5, 6, 7, 8, 9, 10, 11, 12, 13, 14, 15, 16, 17, 18, and 19 amino acid residues may be present therebetween. Examples of the size of the ring formed by the cyclic structure include, but are not intended to be particularly limited to, a 4-membered ring, a 5-membered ring, a 6-membered ring, a 7-membered ring, an 8-membered ring, a 9-membered ring, a 10-membered ring, an 11-membered ring, a 12-membered ring, a 13-membered ring, a 14-membered ring, a 15-membered ring, a 16-membered ring, a 17-membered ring, an 18-membered ring, a 19-membered ring, a 20-membered ring, a 21-membered ring, a 22-membered ring, a 23-membered ring, a 24-membered ring, a 25-membered ring, a 26-membered ring, a 27-membered ring, a 28-membered ring, a 29-membered ring, a 30-membered ring, a 31-membered ring, a 32-membered ring, a 33-membered ring, a 34-membered ring, and a 35-membered ring. Preferably, the ring formed by the cyclic structure is a 5-membered ring, a 6-membered ring, a 7-membered ring, an 8-membered ring, a 9-membered ring, a 10-membered ring, an 11-membered ring, a 12-membered ring, a 13-membered ring, a 14-membered ring, a 15-membered ring, a 16-membered ring, a 17-membered ring, an 18-membered ring, a 19-membered ring, or a 20-membered ring, more preferably a 10-membered ring, an 11-membered ring, a 12-membered ring, a 13-membered ring, a 14-membered ring, a 15-membered ring, a 16-membered ring, a 17-membered ring, or an 18-membered ring, most preferably an 11-membered ring, a 12-membered ring, a 13-membered ring, or a 14-membered ring. When a cyclic structure is present in the peptide compound, the number of cyclic structures is not limited, but it is preferable that one, two, three, four, or five cyclic structures be present.

In an embodiment, the peptide compound or a salt thereof produced by the method of the present invention can contain a cyclic portion composed of 4 or more, 6 or more, 8 or more, or 11 or more amino acid residues. In an embodiment, the peptide compound or a salt thereof produced by the method of the present invention is a cyclic peptide compound or a salt thereof, consisting of a cyclic portion composed of 4 to 14, preferably 6 to 14, further preferably 8 to 14, most preferably 11 to 14 amino acid residues. In an embodiment, the peptide compound or a salt thereof produced by the method of the present invention is a cyclic peptide compound or a salt thereof, consisting of a cyclic portion composed of 11 amino acid residues. In these aspects, the amide bond with which the amino group of the first amino acid or peptide and the carboxy group of the second amino acid or peptide are linked is contained in the cyclic portion at 1, 2, 3, 4, 5, 6, or 7 locations.

In an embodiment, the method of the present invention may further include a step (deprotection step) of removing/deprotecting a protecting group of an amino group or carboxy group of the second amino acid or peptide and/or a protecting group of an amino group or carboxy group of the first amino acid or peptide in the peptide compound obtained in the linking step, immediately before or after and/or during the linking step. The deprotection step can be performed using methods known in the art, for example, using the reagents and conditions described in "Greene's Protective Groups in Organic Synthesis, Fifth Edition, 2014".

In an aspect, the deprotection step of the protecting group of the amino group can be performed by contact hydrogenation, for example, when the protecting group is Cbz, p-nitrobenzyloxycarbonyl, 2-naphthylmethyloxycarbonyl, diphenylmethyloxycarbonyl, or 9-anthrylmethyloxycarbonyl. For the contact hydrogenation, any catalyst known in the art can be used. Specific examples of the catalyst include Pd/C, $Pd(OH)_2/C$, and $PtO_2$, and Pd/C is preferred.

In an aspect, the deprotection step of the protecting group of the amino group can be carried out using a reagent such as TBAF, $LiBH_4$, piperidine, trifluoroacetic acid, or methanesulfonic acid, for example, when the protecting group is Teoc, Fmoc, Boc, Alloc, or trifluoroacetyl.

In an aspect, the deprotection step of the protecting group of the carboxy group can be carried out under acidic conditions in the presence of a deprotection reagent, for example, when the protecting group is a methyl group, an ethyl group, a t-butyl group, a trityl group, or a cumyl group.

In an embodiment, the method of the present invention may further include a step (linking step A) of linking/-condensing a third amino acid or peptide to a peptide compound obtained in the linking step or the deprotection step.

In an embodiment, the method of the present invention may further include a step (linking step B) of linking/-condensing an amino group and a carboxy group in a peptide compound obtained in the linking step or the deprotection step. In this case, the peptide compound obtained in the linking step B is a cyclic peptide compound.

In an aspect, the linking steps A and B can be performed, for example, in the presence or absence of a condensation reagent. Examples of the condensation reagent include T3P, EDCI, HATU, COMU, BEP, PyBOP, DMT-MM, and PyOxim.

In an embodiment, the method of the present invention is performed using a flow reaction apparatus. The "flow reaction apparatus" as used herein refers to an apparatus that continuously supplies raw materials to a tubular reaction vessel, and mixes and reacts the raw materials to produce a target compound. The flow reaction apparatus may be a commercially available flow reaction apparatus (e.g., Africa flow chemistry system or Asia flow chemistry system manufactured by Syrris), or may be an apparatus that combines a tubular reaction vessel and a syringe pump.

Peptide compound

In an aspect, the present invention is a peptide compound or a salt thereof produced by the "Method for producing peptide compound" described above. In an aspect, the present invention is a pharmaceutical composition containing a peptide compound or a salt thereof produced by the "Method for producing peptide compound" described above.

All of the prior art cited herein are incorporated herein by reference. The present application claims priority to Japanese Patent Application No. 2023-168751, filed on September 28, 2023, the contents of which are hereby incorporated by reference in their entirety. All references cited herein, including patent applications and publications, are incorporated herein by reference in their entirety, including the following literatures: International Publication No. WO 2013/100132, International Publication No. WO 2018/225851, International Publication No. WO 2018/225864, International Publication No. WO 2019/ 117274; International Publication No. WO 2020/111238; International Publication No. WO 2020/122182; International Publication No. WO 2021/075478; International Publication No. WO 2021/090856; International Publication No. WO 2021/132545; International Publication No. WO 2021/246471; International Publication No. WO 2022/097540; International Publication No. WO 2022/138891; International Publication No. WO 2022/145444; International Publication No. WO 2022/234864; International Publication No. WO 2023/127869; International Publication No. WO 2023/219152; International Publication No. 2024/096023; and International Publication No. 2024/143514.

[Examples]

The contents of the present invention will be further described by the following Examples, but the present invention is not limited to their contents. Except those as specifically described, starting substances, starting raw materials, solvents, and reagents were obtained from commercial suppliers, or synthesized using known methods.

The $^1$H-NMR spectrum was measured using a nuclear magnetic resonance device JNM-ECZ 500 (manufactured by JEOL Ltd.), the chemical shift of tetramethylsilane used as an internal standard substance was set to 0 ppm, and a deuterium lock signal from a sample solvent was consulted. The chemical shift of a signal from a compound to be analyzed was expressed in ppm. Abbreviations for splitting of a signal were expressed as follows: s = singlet, brs = broad singlet, d = doublet, t = triplet, q = quartet, dd = double doublet, and m = multiplet. The splitting width of a signal was expressed as a J value (Hz). The integral value of signal was calculated on the basis of a ratio of the area intensity of each signal.

HPLC analysis was performed using H-Class system manufactured by Waters Corporation, by measuring at a wavelength of 210 nm with a PDA detector. The reactivity, selectivity, and purity of each substrate used in Examples were evaluated by the analytical method shown in Table 1 below. LCMS analysis was performed using a SQD2 or QDa detector.

[Table 1]

| Measurement method | Column | Measurement conditions |
|---|---|---|
| HPLC Method A | Waters Acquity CSH C18 (15 cm x 2.1 mm, 1.7 um) | Eluent A: 0.05% TFA in $H_2O$<br>Eluent B: 0.05% TFA in MeCN<br>Gradient (%B): 10% (0 min) → 100% (19.0 min) → 100% (21.0 min) → 10%(21.01 min) → 10% (25.0 min)<br>Flow rate: 0.3 mL/min<br>Column temperature: 50°C<br>Detection wavelength: 210 nm |
| HPLC Method B | Sigma Aldrich Ascentis Express C18 (5 cm x 4.6 mm, 2.7 um) | Eluent A: 0.05% TFA in $H_2O$<br>Eluent B: 0.05% TFA in MeCN<br>Gradient (%B): 5% (0 min) → 100% (6.0 min) → 100% (7.0 min) → 5% (7.01 min) → 5% (9.0 min)<br>Flow rate: 0.8 mL/min<br>Column temperature: 35°C<br>Detection wavelength: 210 nm |

Unless otherwise noted in the experiment section, HPLC samples were prepared as follows.

Activation step: 2-3 μL of the reaction mixture was dissolved in MeCN (1.0 mL) and azetidine (0.1 mL).
Condensation step: 5 μL of the reaction mixture was dissolved in MeCN (1.0 mL) and azetidine (0.1 mL).

The conversion of the condensation reaction and the diastereomeric ratio were calculated by the following formula using the HPLC area value of each component in the reaction mixture. The substrate in the formula refers to the one used in smaller number of molar equivalents among the two substrates.

Conversion = [(desired product + epimer)/(substrate + desired product + epimer)] x 100 Diastereomeric ratio = desired product/epimer

(Example 1) Condensation reaction of Cbz-MeLeu-Ile-OH and H-MeAla-Aze-EtPhe(4-Me)-Sar-OtBu: two-layer conditions of organic solvent and water

[Formula 1]

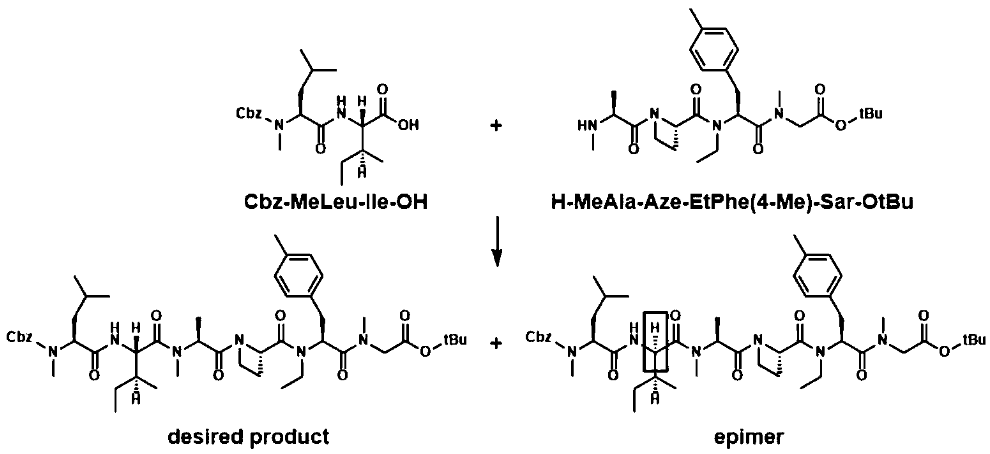

Cbz-MeLeu-Ile-OH

H-MeAla-Aze-EtPhe(4-Me)-Sar-OtBu

desired product

epimer

Activation and condensation reaction steps

A solution of Cbz-MeLeu-Ile-OH in IPAC (concentration 157 mg/mL, 2.00 mL, 314.0 mg, 0.800 mmol) and 2,6-lutidine (104.0 μL, 0.895 mmol) were added to a test tube (hereinafter referred to as "test tube A") with a stirrer, and the reaction mixture was cooled to -10°C while stirring. To the reaction mixture, PivCl (91.8 μL, 0.746 mmol) was added, and the mixture was stirred for 3 hours. The reaction mixture was analyzed by HPLC to confirm that activation had progressed. H-MeAla-Aze-EtPhe(4-Me)-Sar-OtBu (300.0 mg, 0.597 mmol), toluene (2.00 mL), $Na_2CO_3$ (190.0 mg, 1.790 mmol), and water (1.90 mL) were added to another test tube (hereinafter referred to as "test tube B") with a stirrer, and the reaction mixture was cooled to 0°C while stirring. The mixture in test tube A was added dropwise to test tube B, and the obtained mixture was stirred at 0°C continuously for 22 hours.

Reaction conversion: 98.8%
Diastereomeric ratio: 99.6/0.4
Piv-MeAla-Aze-EtPhe(4-Me)-Sar-OtBu: 1.8%

Workup step

The aqueous layer of the reaction mixture was removed, and N,N-dimethyl ethylenediamine (65.0 μL, 0.597 mmol) was added to the organic layer, and the temperature was raised to 25°C while stirring the organic layer. To the organic layer, 2.5% aqueous ammonia solution (2.00 mL) was added. The mixture was stirred and allowed to stand still, then the aqueous layer was removed. To the organic layer, 5% aqueous sulfuric acid solution (2.00 mL) was added. The mixture was stirred and allowed to stand still, then the aqueous layer was removed. To the organic layer, 5% aqueous $Na_2CO_3$ solution was added. The mixture was stirred and allowed to stand still, then the aqueous layer was removed. The resulting organic layer was concentrated to dryness to obtain 513.4 mg of the target.

Yield: 98%
Purity: 96.8%

Measurement method: HPLC Method A
Retention time: H-MeAla-Aze-EtPhe(4-Me)-Sar-OtBu: 8.0 min, Piv-MeAla-Aze-EtPhe(4-Me)-Sar-OtBu: 13.1 min, desired product: 16.4 min, epimer: 16.8 min
Mass spectrometry: H-MeAla-Aze-EtPhe(4-Me)-Sar-OtBu: m/z 503.68 ($[M+H]^+$), Piv-MeAla-Aze-EtPhe(4-Me)-Sar-OtBu: m/z 587.81 ($[M+H]^+$), desired product: m/z 732.98 ($[M\text{-}Sar\text{-}OtBu]^+$)

(Example 2) Condensation reaction of Cbz-MeLeu-Ile-OH and H-MeAla-Aze-EtPhe(4-Me)-Sar-OtBu

The condensation reaction was carried out under the following conditions A to conditions D.

[Formula 2]

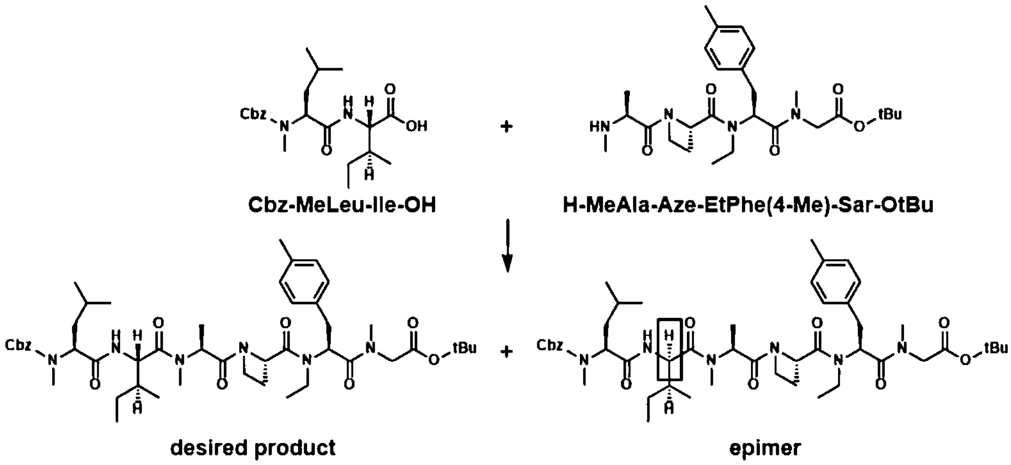


Conditions A

To a test tube with a stirrer, a solution of Cbz-MeLeu-Ile-OH in IPAC (concentration 185.0 mg/mL, 0.240 mL, 44.4 mg, 0.113 mmol) and DIPEA (22.4 μL, 0.128 mmol) were added, and the mixture was cooled to -20°C while stirring. To the reaction mixture, PivCl (13.2 μL, 0.107 mmol) was added, and the mixture was stirred for 1 hour and 25 minutes. To the reaction mixture, a solution of H-MeAla-Aze-EtPhe(4-Me)-Sar-OtBu in IPAC (concentration 156.0 mg/mL, 0.280 mL, 43.7 mg, 0.087 mmol) was added, and the mixture was stirred for 15 minutes while raising the temperature to 0°C. An aqueous $Na_2CO_3$ solution (27.2 mg $Na_2CO_3$, 0.270 mL $H_2O$) was then added, and the mixture was stirred continuously for 8 hours.

Conditions B

To a test tube with a stirrer, a solution of Cbz-MeLeu-Ile-OH in IPAC (concentration 185.0 mg/mL, 0.240 mL, 44.4 mg, 0.113 mmol) and DIPEA (22.4 μL, 0.128 mmol) were added, and the mixture was cooled to -20°C while stirring. To the reaction mixture, PivCl (13.2 μL, 0.107 mmol) was added, and the mixture was stirred for 1 hour and 25 minutes. To the reaction mixture, a solution of H-MeAla-Aze-EtPhe(4-Me)-Sar-OtBu in IPAC (concentration 156.0 mg/mL, 0.280 mL, 43.7 mg, 0.087 mmol) was added, and the mixture was stirred for 15 minutes while raising the temperature to 0°C. DIPEA (44.7 μL, 0.257 mmol) was then added, and the mixture was stirred continuously for 8 hours.

Conditions C

To a vial with a stirrer, HATU (76.0 mg, 0.199 mmol), a solution of Cbz-MeLeu-Ile-OH in MeCN (concentration 200.0 mg/mL, 0.210 mL, 42.0 mg, 0.107 mmol), a solution of H-MeAla-Aze-EtPhe(4-Me)-Sar-OtBu in MeCN (concentration 142.9 mg/mL, 0.270 mL, 38.6 mg, 0.077 mmol), and DIPEA (34.7 μL, 0.199 mmol) were sequentially added, and the mixture was stirred.

Conditions D

To a test tube with a stirrer, HOPO (9.5 mg, 0.086 mmol), a solution of Cbz-MeLeu-Ile-OH in IPAC (concentration 185.0 mg/mL, 0.180 mL, 33.6 mg, 0.086 mmol), a solution of H-MeAla-Aze-EtPhe(4-Me)-Sar-OtBu in IPAC (concentration 156.0 mg/mL, 0.280 mL, 43.0 mg, 0.086 mmol), and water (0.46 mL) were sequentially added, and the mixture was cooled

to 0°C. EDC·HCl (18.0 mg, 0.094 mmol) was added to the reaction mixture, and the mixture was stirred at 0°C for 14 hours.

Synthesis of epimer

The epimer was synthesized according to conditions A except for using Cbz-MeLeu-D-allo-Ile-OH instead of Cbz-MeLeu-Ile-OH.

Measurement method: HPLC Method A
Retention time: H-MeAla-Aze-EtPhe(4-Me)-Sar-OtBu: 7.8 min, desired product: 16.2 min, epimer: 16.6 min
Mass spectrometry: H-MeAla-Aze-EtPhe(4-Me)-Sar-OtBu: m/z 503.79 ($[M+H]^+$), desired product: m/z 732.87 ($[M-Sar-OtBu]^+$), epimer: m/z 732.87 ($[M-Sar-OtBu]^+$)

[Table 2]

| Reaction conditions | Analysis results | |
|---|---|---|
| | Conversion (%) | Diastereomeric ratio |
| Conditions A: Two-layer conditions of organic solvent and water | 90.0 | 99.5 / 0.5 |
| Conditions B: One-layer conditions of organic solvent only | 76.3 | 91.0 / 9.0 |
| Conditions C: Condensation conditions using HATU | 92.4 | 35.4 / 64.6 |
| Conditions D: Known two-layer conditions | 8.4 | 81.7 / 18.3 |

As shown in Table 2, it was found that both of the conversion and diastereomeric ratio were high in the two-layer conditions of organic solvent and water (conditions A), while either or both of the conversion and diastereomeric ratio were significantly low in the one-layer conditions of organic solvent only (conditions B), the conditions using HATU as a condensation agent (conditions C), and the known two-layer conditions (conditions D).

(Example 3) Condensation reaction of Cbz-MeLeu-Ile-OH and H-MeLeu-Ile-MeAla-Aze-EtPhe(4-Me)-Sar-OtBu

The condensation reaction was carried out under the following conditions A to C.

[Formula 3]

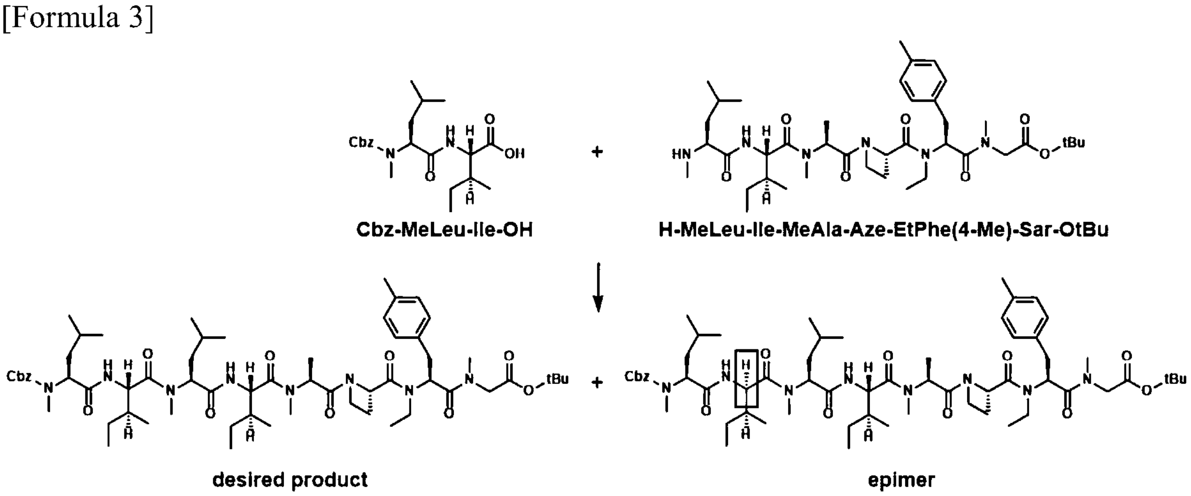

Conditions A

To a test tube with a stirrer, a solution of Cbz-MeLeu-Ile-OH in IPAC (concentration 185.0 mg/mL, 0.220 mL, 40.7 mg, 0.100 mmol) and DIPEA (18.8 μL, 0.108 mmol) were added, and the mixture was cooled to -20°C while stirring. To the reaction mixture, PivCl (11.6 μL, 0.094 mmol) was added, and the mixture was stirred for 1 hour and 30 minutes. To the reaction mixture, a solution of H-MeLeu-Ile-MeAla-Aze-EtPhe(4-Me)-Sar-OtBu in toluene (concentration 200.0 mg/mL, 0.250 mL, 50.0 mg, 0.067 mmol) was added, and the mixture was stirred for 15 minutes while raising the temperature to

0°C. An aqueous $Na_2CO_3$ solution (21.0 mg $Na_2CO_3$, 0.210 mL $H_2O$) was then added, and the mixture was stirred continuously for 22 hours.

Conditions B

To a test tube with a stirrer, a solution of Cbz-MeLeu-Ile-OH in IPAC (concentration 185.0 mg/mL, 0.220 mL, 40.7 mg, 0.100 mmol) and DIPEA (18.8 μL, 0.108 mmol) were added, and the mixture was cooled to -20°C while stirring. To the reaction mixture, PivCl (11.6 μL, 0.094 mmol) was added, and the mixture was stirred for 1 hour and 30 minutes. To the reaction mixture, a solution of H-MeLeu-Ile-MeAla-Aze-EtPhe(4-Me)-Sar-OtBu in toluene (concentration 200.0 mg/mL, 0.250 mL, 50.0 mg, 0.067 mmol) was added, and the mixture was stirred for 15 minutes while raising the temperature to 0°C. DIPEA (35.2 μL, 0.202 mmol) was then added, and the mixture was stirred continuously for 22 hours.

Conditions C

To a vial with a stirrer, HATU (51.2 mg, 0.135 mmol), a solution of Cbz-MeLeu-Ile-OH in MeCN (concentration 200.0 mg/mL, 0.150 mL, 30.0 mg, 0.076 mmol), a solution of H-MeLeu-Ile-MeAla-Aze-EtPhe(4-Me)-Sar-OtBu in MeCN (concentration 170.0 mg/mL, 0.300 mL, 51.0 mg, 0.069 mmol), and DIPEA (23.4 μL, 0.134 mmol) were sequentially added, and the mixture was stirred for 3 hours.

Synthesis of epimer

The epimer was synthesized according to conditions A except for using Cbz-MeLeu-D-allo-Ile-OH instead of Cbz-MeLeu-Ile-OH.

Measurement method: HPLC Method A
Retention time: H-MeLeu-Ile-MeAla-Aze-EtPhe(4-Me)-Sar-OtBu: 9.8 min, desired product: 17.9 min, epimer: 18.5 min
Mass spectrometry: H-MeLeu-Ile-MeAla-Aze-EtPhe(4-Me)-Sar-OtBu: m/z 743.92 ($[M+H]^+$), desired product: m/z 1118.26 ($[M+H]^+$), epimer: m/z 1118.32 ($[M+H]^+$)

[Table 3]

| Reaction conditions | Analysis results | |
|---|---|---|
| | Conversion (%) | Diastereomeric ratio |
| Conditions A: Two-layer conditions of organic solvent and water | 94.4 | 100.0 / 0.0 |
| Conditions B: One-layer conditions of organic solvent only | 53.5 | 95.7 / 4.3 |
| Conditions C: Condensation conditions using HATU | 55.8 | 34.7 / 65.3 |

As shown in Table 3, it was found that both of the conversion and diastereomeric ratio were high in the two-layer conditions of organic solvent and water (conditions A), while either or both of the conversion and diastereomeric ratio were significantly low in the one-layer conditions of organic solvent only (conditions B) and the conditions using HATU as a condensation agent (conditions C).

(Example 4) Condensation reaction of Cbz-MeLeu-Ile-OH and H-MeGly(cPent)-MeAsp(OtBu)-$NMe_2$

The condensation reaction was carried out under conditions A to C shown below.

[Formula 4]

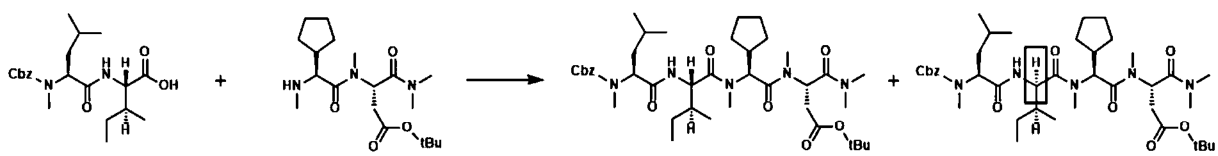

Cbz-MeLeu-Ile-OH H-MeGly(cPent)-MeAsp(OtBu)-$NMe_2$ desired product epimer

Conditions A

To a test tube with a stirrer, a solution of Cbz-MeLeu-Ile-OH in IPAC (concentration 185.0 mg/mL, 0.230 mL, 42.6 mg, 0.108 mmol) and DIPEA (21.2 μL, 0.122 mmol) were added, and the mixture was cooled to -20°C while stirring. To the reaction mixture, PivCl (12.5 μL, 0.101 mmol) was added, and the mixture was stirred for 1 hour and 15 minutes. To the reaction mixture, a solution of H-MeGly(cPent)-MeAsp(OtBu)-$NMe_2$ in toluene (concentration 100.0 mg/mL, 0.300 mL, 30.0 mg, 0.081 mmol) was added, and the mixture was stirred for 15 minutes while raising the temperature to 0°C. An aqueous $Na_2CO_3$ solution (26.0 mg $Na_2CO_3$, 0.260 mL $H_2O$) was then added, and the mixture was stirred continuously for 9 hours.

Conditions B

To a test tube with a stirrer, a solution of Cbz-MeLeu-Ile-OH in IPAC (concentration 185.0 mg/mL, 0.230 mL, 42.6 mg, 0.108 mmol) and DIPEA (21.2 μL, 0.122 mmol) were added, and the mixture was cooled to -20°C while stirring. To the reaction mixture, PivCl (12.5 μL, 0.101 mmol) was added, and the mixture was stirred for 1 hour and 15 minutes. To the reaction mixture, a solution of H-MeGly(cPent)-MeAsp(OtBu)-$NMe_2$ in toluene (concentration 100.0 mg/mL, 0.300 mL, 30.0 mg, 0.081 mmol) was added, and the mixture was stirred for 15 minutes while raising the temperature to 0°C. DIPEA (42.4 μL, 0.244 mmol) was then added, and the mixture was stirred continuously for 9 hours.

Conditions C

To a vial with a stirrer, HATU (51.5 mg, 0.135 mmol), a solution of Cbz-MeLeu-Ile-OH in MeCN (concentration 200.0 mg/mL, 0.150 mL, 30.0 mg, 0.076 mmol), a solution of H-MeGly(cPent)-MeAsp(OtBu)-$NMe_2$ in MeCN (concentration 75.8 mg/mL, 0.330 mL, 25.0 mg, 0.068 mmol), and DIPEA (23.4 μL, 0.134 mmol) were sequentially added, and the mixture was stirred for 2 hours.

Synthesis of epimer

The epimer was synthesized according to conditions A except for using Cbz-MeLeu-D-allo-Ile-OH instead of Cbz-MeLeu-Ile-OH.

Measurement method: HPLC Method A
Retention time: H-MeGly(cPent)-MeAsp(OtBu)-$NMe_2$: 5.6 min, desired product: 16.7 min, epimer: 17.5 min
Mass spectrometry: H-MeGly(cPent)-MeAsp(OtBu)-$NMe_2$: m/z 370.64 ($[M+H]^+$), desired product: m/z 766.78 ($[M+Na]^+$), epimer: m/z 767.01 ($[M+Na]^+$)

[Table 4]

| Reaction conditions | Analysis results | |
|---|---|---|
| | Conversion (%) | Diastereomeric ratio |
| Conditions A: Two-layer conditions of organic solvent and water | 95.5 | 100.0 / 0.0 |
| Conditions B: One-layer conditions of organic solvent only | 67.9 | 99.3 / 0.7 |
| Conditions C: Condensation conditions using HATU | 29.0 | 63.8 / 36.2 |

As shown in Table 4, it was found that both of the conversion and diastereomeric ratio were high in the two-layer conditions of organic solvent and water (conditions A), while either or both of the conversion and diastereomeric ratio were significantly low in the one-layer conditions of organic solvent only (conditions B) and the conditions using HATU as a condensation agent (conditions C).

(Example 5) Condensation reaction of Cbz-MeLeu-Ala-OH and H-MeLeu-Ile-MeAla-Aze-EtPhe(4-Me)-Sar-OtBu

The condensation reaction was carried out under conditions A to C shown in the table below.

[Formula 5]

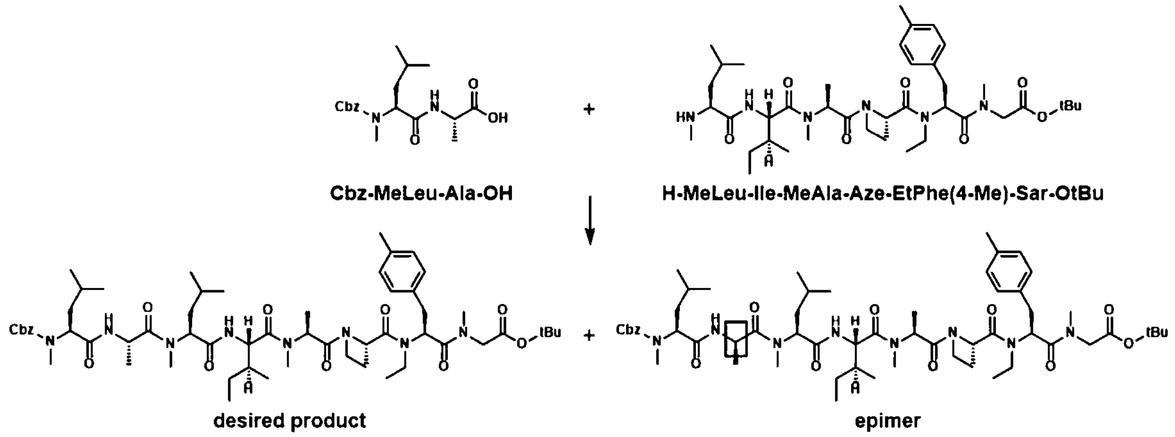


Conditions A

To a test tube with a stirrer, a solution of Cbz-MeLeu-Ile-OH in IPAC (concentration 156.0 mg/mL, 0.240 mL, 37.4 mg, 0.107 mmol) and 2,6-lutidine (13.5 μL, 0.115 mmol) were added, and the mixture was cooled to -10°C while stirring. To the reaction mixture, PivCl (12.0 μL, 0.098 mmol) was added, and the mixture was stirred for 3 hours. To the reaction mixture, a solution of H-MeLeu-Ile-MeAla-Aze-EtPhe(4-Me)-Sar-OtBu in toluene (concentration 200.0 mg/mL, 0.330 mL, 66.0 mg, 0.089 mmol) was added, and the mixture was stirred for 10 minutes while raising the temperature to 0°C. An aqueous $Na_2CO_3$ solution (28.0 mg $Na_2CO_3$, 0.280 mL $H_2O$) was then added, and the mixture was stirred continuously for 6 hours.

Conditions B

To a test tube with a stirrer, a solution of Cbz-MeLeu-Ile-OH in IPAC (concentration 156.0 mg/mL, 0.240 mL, 37.4 mg, 0.107 mmol) and 2,6-lutidine (13.5 μL, 0.115 mmol) were added, and the mixture was cooled to -10°C while stirring. To the reaction mixture, PivCl (12.0 μL, 0.098 mmol) was added, and the mixture was stirred for 3 hours. To the reaction mixture, a solution of H-MeLeu-Ile-MeAla-Aze-EtPhe(4-Me)-Sar-OtBu in toluene (concentration 200.0 mg/mL, 0.330 mL, 66.0 mg, 0.089 mmol) was added, and the mixture was stirred for 10 minutes while raising the temperature to 0°C. DIPEA (46.0 μL, 0.266 mmol) was then added, and the mixture was stirred continuously for 6 hours.

Conditions C

To a vial with a stirrer, HATU (67.6 mg, 0.178 mmol), a solution of Cbz-MeLeu-Ala-OH in IPAC (concentration 156.0 mg/mL, 0.240 mL, 37.4 mg, 0.107 mmol), a solution of H-MeLeu-Ile-MeAla-Aze-EtPhe(4-Me)-Sar-OtBu in MeCN (concentration 170 mg/mL, 0.390 mL, 66.0 mg, 0.089 mmol), and DIPEA (31.0 μL, 0.178 mmol) were sequentially added, and the mixture was stirred.

Measurement method: HPLC Method A
Retention time: H-MeLeu-Ile-MeAla-Aze-EtPhe(4-Me)-Sar-OtBu: 10.4 min, desired product: 17.4 min, epimer: 17.7 min
Mass spectrometry: H-MeLeu-Ile-MeAla-Aze-EtPhe(4-Me)-Sar-OtBu: m/z 744.15 ($[M+H]^+$), desired product: m/z 931.09 ($[M-Sar-OtBu]^+$), epimer: m/z 931.27 ($[M-Sar-OtBu]^+$)

[Table 5]

| Reaction conditions | Analysis results | |
|---|---|---|
| | Conversion (%) | Diastereomeric ratio |
| Conditions A: Two-layer conditions of organic solvent and water | 99.1 | 100.0 / 0.0 |
| Conditions B: One-layer conditions of organic solvent only | 83.6 | 87.8 / 12.2 |

(continued)

| Reaction conditions | Analysis results | |
|---|---|---|
| | Conversion (%) | Diastereomeric ratio |
| Conditions C: Condensation conditions using HATU | 100.0 | 43.7 / 56.3 |

As shown in Table 5, it was found that both of the conversion and diastereomeric ratio were high in the two-layer conditions of organic solvent and water (conditions A), while either or both of the conversion and diastereomeric ratio were lower in the one-layer conditions of organic solvent only (conditions B) and the conditions using HATU as a condensation agent (conditions C) compared to those in conditions A.

(Example 6) Condensation reaction of Cbz-MeLeu-Phe-OH and H-MeLeu-Ile-MeAla-Aze-EtPhe(4-Me)-Sar-OtBu

The condensation reaction was carried out under the following conditions A to C.

[Formula 6]

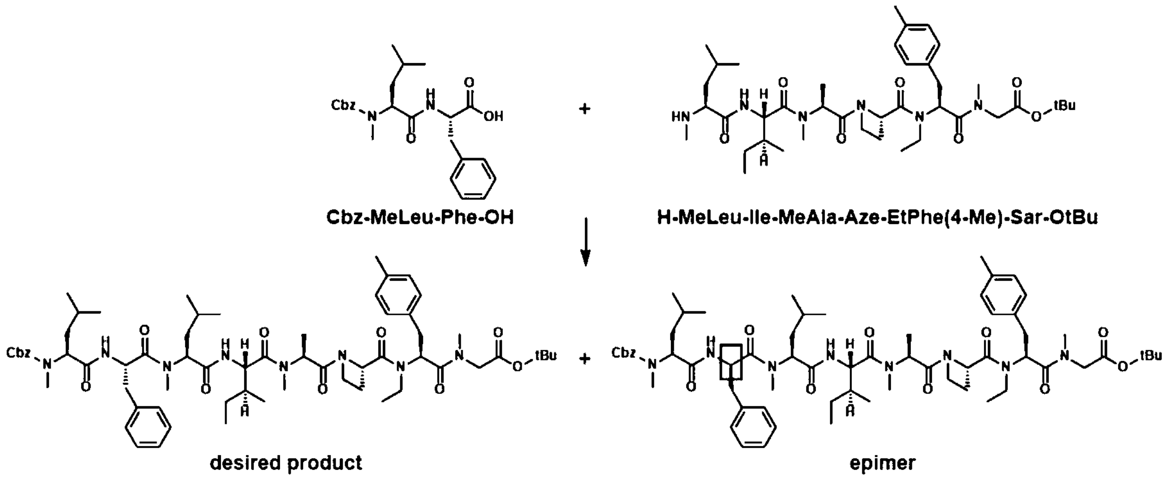

Cbz-MeLeu-Phe-OH

H-MeLeu-Ile-MeAla-Aze-EtPhe(4-Me)-Sar-OtBu

desired product

epimer

Conditions A

To a test tube with a stirrer, a solution of Cbz-MeLeu-Phe-OH in IPAC (concentration 159.0 mg/mL, 0.270 mL, 42.9 mg, 0.101 mmol) and 2,6-lutidine (12.6 μL, 0.108 mmol) were added, and the mixture was cooled to -10°C while stirring. To the reaction mixture, PivCl (11.3 μL, 0.092 mmol) was added, and the mixture was stirred for 3 hours and 30 minutes. To the reaction mixture, a solution of H-MeLeu-Ile-MeAla-Aze-EtPhe(4-Me)-Sar-OtBu in toluene (concentration 200.0 mg/mL, 0.310 mL, 62.0 mg, 0.083 mmol) was added, and the mixture was stirred for 10 minutes while raising the temperature to 0°C. An aqueous $Na_2CO_3$ solution (26.0 mg $Na_2CO_3$, 0.260 mL $H_2O$) was then added, and the mixture was stirred continuously for 3 hours.

Conditions B

To a test tube with a stirrer, a solution of Cbz-MeLeu-Phe-OH in IPAC (concentration 159.0 mg/mL, 0.270 mL, 42.9 mg, 0.101 mmol) and 2,6-lutidine (12.6 μL, 0.108 mmol) were added, and the mixture was cooled to -10°C while stirring. To the reaction mixture, PivCl (11.3 μL, 0.092 mmol) was added, and the mixture was stirred for 3 hours and 30 minutes. To the reaction mixture, a solution of H-MeLeu-Ile-MeAla-Aze-EtPhe(4-Me)-Sar-OtBu in toluene (concentration 200.0 mg/mL, 0.310 mL, 62.0 mg, 0.083 mmol) was added, and the mixture was stirred for 10 minutes while raising the temperature to 0°C. DIPEA (43.6 μL, 0.250 mmol) was then added, and the mixture was stirred continuously for 3 hours.

Conditions C

To a vial with a stirrer, HATU (63.5 mg, 0.167 mmol), a solution of Cbz-MeLeu-Phe-OH in MeCN (concentration 195.0 mg/mL, 0.200 mL, 39.0 mg, 0.091 mmol), a solution of H-MeLeu-Ile-MeAla-Aze-EtPhe(4-Me)-Sar-OtBu in MeCN (concentration 170.0 mg/mL, 0.360 mL, 61.2 mg, 0.082 mmol), and DIPEA (29.0 μL, 0.167 mmol) were sequentially

added, and the mixture was stirred.

Measurement method: HPLC Method A
Retention time: H-MeLeu-Ile-MeAla-Aze-EtPhe(4-Me)-Sar-OtBu: 10.4 min, desired product: 18.2 min, epimer: 18.7 min
Mass spectrometry: H-MeLeu-Ile-MeAla-Aze-EtPhe(4-Me)-Sar-OtBu: m/z 744.04 ([M+H]$^+$), desired product: m/z 1007.35 ([M-Sar-OtBu]$^+$), epimer: m/z 1007.30 ([M-Sar-OtBu]$^+$)

[Table 6]

| Reaction conditions | Analysis results | |
|---|---|---|
| | Conversion (%) | Diastereomeric ratio |
| Conditions A: Two-layer conditions of organic solvent and water | 98.2 | 99.9 / 0.1 |
| Conditions B: One-layer conditions of organic solvent only | 70.9 | 99.1 / 0.9 |
| Conditions C: Condensation conditions using HATU | 94.2 | 37.1 / 62.9 |

As shown in Table 6, it was found that both of the conversion and diastereomeric ratio were high in the two-layer conditions of organic solvent and water (conditions A), while either or both of the conversion and diastereomeric ratio were significantly low in the one-layer conditions of organic solvent only (conditions B) and the conditions using HATU as a condensation agent (conditions C).

(Example 7) Condensation reaction of Cbz-MeLeu-Ser(OtBu)-OH and H-MeAla-Aze-EtPhe(4-Me)-Sar-OtBu

The condensation reaction was carried out under the following conditions A to C.

[Formula 7]

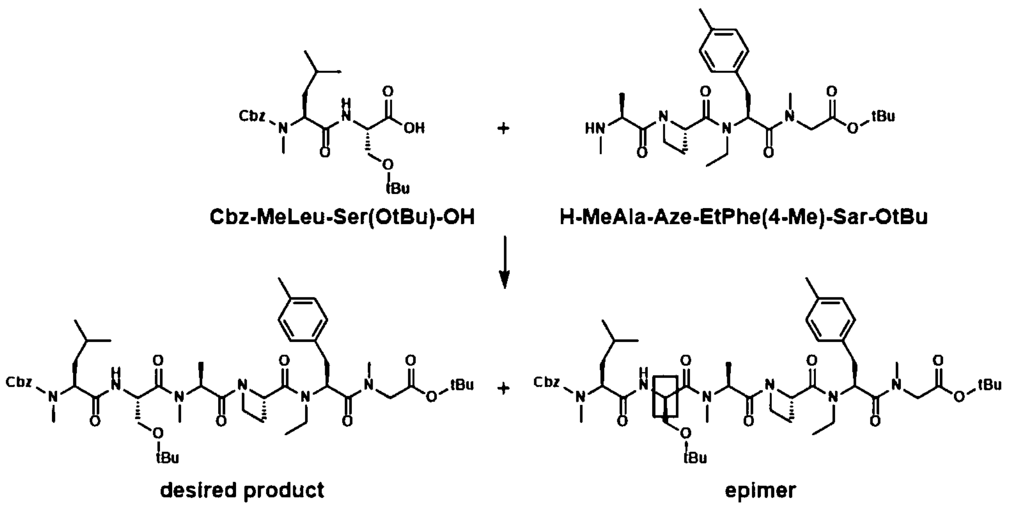

Cbz-MeLeu-Ser(OtBu)-OH + H-MeAla-Aze-EtPhe(4-Me)-Sar-OtBu

desired product + epimer

<u>Conditions A</u>

To a test tube with a stirrer, a solution of Cbz-MeLeu-Ser(OtBu)-OH in IPAC (concentration 155.0 mg/mL, 0.260 mL, 40.3 mg, 0.096 mmol) and 2,6-lutidine (12.0 μL, 0.103 mmol) were added, and the mixture was cooled to -20°C while stirring. To the reaction mixture, PivCl (10.8 μL, 0.088 mmol) was added, and the mixture was stirred for 3 hours. To the reaction mixture, a solution of H-MeAla-Aze-EtPhe(4-Me)-Sar-OtBu in toluene (concentration 200.0 mg/mL, 0.200 mL, 40.0 mg, 0.080 mmol) was added, and the mixture was stirred for 15 minutes while raising the temperature to 0°C. An aqueous $Na_2CO_3$ solution (25.0 mg $Na_2CO_3$, 0.250 mL $H_2O$) was then added, and the mixture was stirred continuously for 3 hours.

Conditions B

To a test tube with a stirrer, a solution of Cbz-MeLeu-Ser(OtBu)-OH in IPAC (concentration 155.0 mg/mL, 0.260 mL, 40.3 mg, 0.096 mmol) and 2,6-lutidine (12.0 μL, 0.103 mmol) were added, and the mixture was cooled to -20°C while stirring. To the reaction mixture, PivCl (10.8 μL, 0.088 mmol) was added, and the mixture was stirred for 3 hours. To the reaction mixture, a solution of H-MeAla-Aze-EtPhe(4-Me)-Sar-OtBu in toluene (concentration 200.0 mg/mL, 0.200 mL, 40.0 mg, 0.080 mmol) was added, and the mixture was stirred for 15 minutes while raising the temperature to 0°C. DIPEA (41.6 μL, 0.239 mmol) was then added, and the mixture was stirred continuously for 3 hours.

Conditions C

To a vial with a stirrer, HATU (71.1 mg, 0.187 mmol), a solution of Cbz-MeLeu-Ser(OtBu)-OH in IPAC (concentration 155.0 mg/mL, 0.280 mL, 43.4 mg, 0.103 mmol), a solution of H-MeAla-Aze-EtPhe(4-Me)-Sar-OtBu in MeCN (concentration 142.9 mg/mL, 0.330 mL, 47.1 mg, 0.094 mmol), and DIPEA (32.6 μL, 0.187 mmol) were sequentially added, and the mixture was stirred.

Synthesis of epimer

The epimer was synthesized according to conditions A except for using Cbz-MeLeu-D-Ser(OtBu)-OH instead of Cbz-MeLeu-Ser(OtBu)-OH.

Measurement method: HPLC Method A
Retention time: H-MeAla-Aze-EtPhe(4-Me)-Sar-OtBu: 8.5 min, desired product: 17.1 min, epimer: 17.3 min
Mass spectrometry: H-MeAla-Aze-EtPhe(4-Me)-Sar-OtBu: m/z 503.74 ($[M+H]^+$), desired product: m/z 762.96 ($[M\text{-Sar-OtBu}]^+$), epimer: m/z 763.02 ($[M\text{-Sar-OtBu}]^+$)

[Table 7]

| Reaction conditions | Analysis results | |
|---|---|---|
| | Conversion (%) | Diastereomeric ratio |
| Conditions A: Two-layer conditions of organic solvent and water | 100.0 | 99.8 / 0.2 |
| Conditions B: One-layer conditions of organic solvent only | 99.1 | 99.7 / 0.3 |
| Conditions C: Condensation conditions using HATU | 99.1 | 52.5 / 47.5 |

As shown in Table 7, it was found that both of the conversion and diastereomeric ratio were high in the two-layer conditions of organic solvent and water (conditions A), while either or both of the conversion and diastereomeric ratio were lower in the one-layer conditions of organic solvent (conditions B) and the conditions using HATU as a condensation agent (conditions C) compared to those in conditions A.

(Example 8) Condensation reaction of Cbz-MeLeu-Ile-OH and H-MeLeu-Ile-OtBu

The condensation reaction was carried out under the following conditions A or B.

[Formula 8]

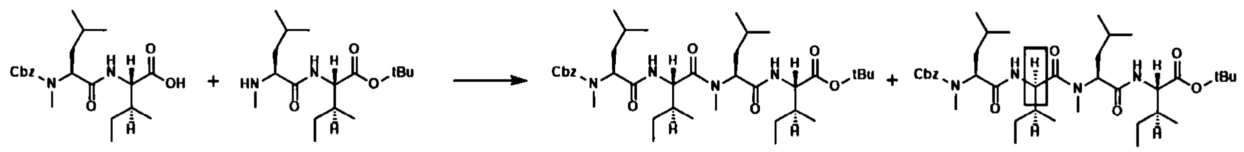

Cbz-MeLeu-Ile-OH H-MeLeu-Ile-OtBu desired product epimer

Conditions A

A solution of Cbz-MeLeu-Ile-OH in IPAC (concentration 185.0 mg/mL, 12.14 mL, 2.247 g, 5.72 mmol) and 2,6-

lutidine (756 μL, 6.49 mmol) were added to a 100 mL flask after nitrogen replacement, and the mixture was cooled to -10°C while stirring. To the reaction mixture, PivCl (657 μL, 5.34 mmol) was added, and the mixture was stirred for 3 hours. To the reaction mixture, a solution of H-MeLeu-Ile-OtBu in IPAC (concentration 137.6 mg/mL, 8.72 mL, 1.20 g, 3.82 mmol) and an aqueous $Na_2CO_3$ solution (1.215 g $Na_2CO_3$, 12.13 mL $H_2O$) were added. After the temperature was raised to 0°C, the mixture was stirred continuously for 5 hours.

Conditions B

To a test tube with a stirrer, COMU (109.0 mg, 0.254 mmol), a solution of Cbz-MeLeu-Ile-OH in IPAC (concentration 185.0 mg/mL, 0.364 mL, 67.4 mg, 0.172 mmol), a solution of H-MeLeu-Ile-OtBu in IPAC (concentration 137.6 mg/mL, 0.291 mL, 40.0 mg, 0.127 mmol), and DIPEA (44.3 μL, 0.254 mmol) were sequentially added, and the mixture was stirred.

Synthesis of epimer

The epimer was synthesized according to conditions A except for using Cbz-MeLeu-D-allo-Ile-OH instead of Cbz-MeLeu-Ile-OH.

Measurement method: HPLC Method A
Retention time: H-MeLeu-Ile-OtBu: 8.0 min, desired product: 18.3 min, epimer: 18.8 min
Mass spectrometry: H-MeLeu-Ile-OtBu: m/z 315.37 ($[M+H]^+$), desired product: m/z 689.65 ($[M+H]^+$), epimer: m/z 689.56 ($[M+H]^+$)

[Table 8]

| Reaction conditions | Analysis results | |
|---|---|---|
| | Conversion (%) | Diastereomeric ratio |
| Conditions A: Two-layer conditions of organic solvent and water | 95.7 | 99.9 / 0.1 |
| Conditions B: Condensation conditions using COMU | 90.7 | 42.6 / 57.4 |

As shown in Table 8, it was found that both of the conversion and diastereomeric ratio were high in the two-layer conditions of organic solvent and water (conditions A), while the diastereomeric ratio was significantly low in the conditions using COMU as a condensation agent (conditions B).

(Example 9) Condensation reaction of Cbz-MeLeu-Ile-MeLeu-Ile-OH and H-MeAla-Aze-EtPhe(4-Me)-Sar-OtBu

The condensation reaction was carried out under conditions A to C shown below.

[Formula 9]

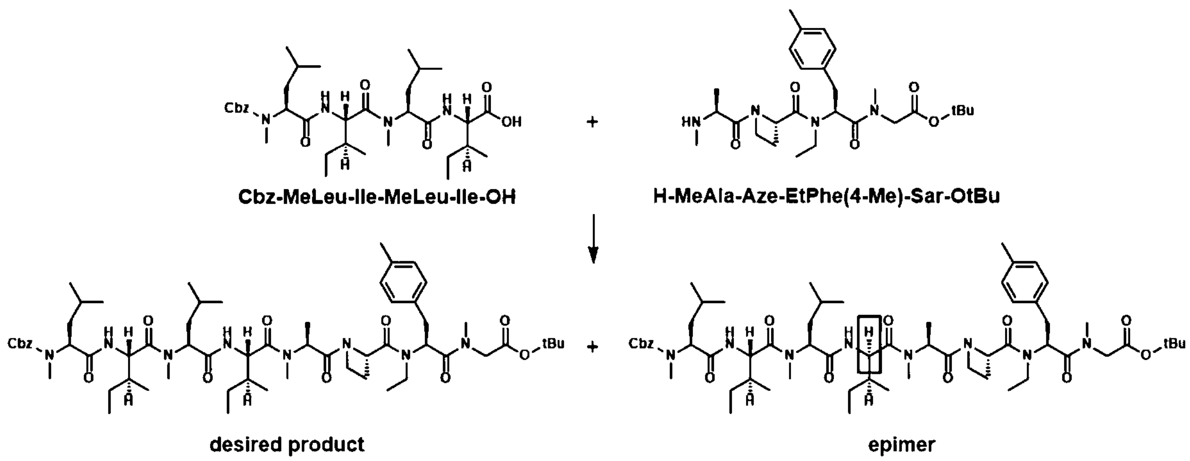

Cbz-MeLeu-Ile-MeLeu-Ile-OH + H-MeAla-Aze-EtPhe(4-Me)-Sar-OtBu

desired product + epimer

Conditions A

To a test tube with a stirrer, a solution of Cbz-MeLeu-Ile-MeLeu-Ile-OH in IPAC (concentration 116.2 mg/mL, 0.731 mL, 85.0 mg, 0.134 mmol) and 2,6-lutidine (17.4 μL, 0.149 mmol) were added, and the mixture was cooled to -10°C while stirring. To the reaction mixture, PivCl (15.3 μL, 0.124 mmol) was added, and the mixture was stirred for 5 hours and 30 minutes. To the reaction mixture, a solution of H-MeAla-Aze-EtPhe(4-Me)-Sar-OtBu in IPAC (concentration 148.7 mg/mL, 0.336 mL, 50.0 mg, 0.099 mmol) and an aqueous $Na_2CO_3$ solution (31.9 mg $Na_2CO_3$, 0.316 mL $H_2O$) were added. After the temperature was raised to 0°C, the mixture was stirred continuously for 16 hours.

Conditions B

To a test tube with a stirrer, a solution of Cbz-MeLeu-Ile-MeLeu-Ile-OH in IPAC (concentration 116.2 mg/mL, 0.585 mL, 68.0 mg, 0.107 mmol) and 2,6-lutidine (13.8 μL, 0.119 mmol) were added, and the mixture was cooled to -10°C while stirring. To the reaction mixture, PivCl (12.3 μL, 0.100 mmol) was added, and the mixture was stirred for 5 hours and 30 minutes. To the reaction mixture, a solution of H-MeAla-Aze-EtPhe(4-Me)-Sar-OtBu in IPAC (concentration 148.7 mg/mL, 0.269 mL, 40.0 mg, 0.080 mmol) and DIPEA (41.6 μL, 0.239 mmol) were added. After the temperature was raised to 0°C, the mixture was stirred continuously for 16 hours.

Conditions C

To a test tube with a stirrer, COMU (42.6 mg, 0.099 mmol), a solution of Cbz-MeLeu-Ile-MeLeu-Ile-OH in IPAC (concentration 116.2 mg/mL, 0.366 mL, 42.5 mg, 0.067 mmol), a solution of H-MeAla-Aze-EtPhe(4-Me)-Sar-OtBu in MeCN (concentration 148.7 mg/mL, 0.168 mL, 25.0 mg, 0.050 mmol), and DIPEA (17.3 μL, 0.099 mmol) were sequentially added, and the mixture was stirred.

Measurement method: HPLC Method A
Retention time: H-MeAla-Aze-EtPhe(4-Me)-Sar-OtBu: 8.0 min, desired product: 17.8 min, epimer: 18.5 min
Mass spectrometry: H-MeAla-Aze-EtPhe(4-Me)-Sar-OtBu: m/z 503.36 ($[M+H]^+$), desired product: m/z 1117.83 ($[M+H]^+$), epimer: m/z 1117.88 ($[M+H]^+$)

[Table 9]

| Reaction conditions | Analysis results | |
|---|---|---|
| | Conversion (%) | Diastereomeric ratio |
| Conditions A: Two-layer conditions of organic solvent and water | 94.4 | 99.8 / 0.2 |
| Conditions B: One-layer conditions of organic solvent only | 79.6 | 99.2 / 0.8 |
| Conditions C: Condensation conditions using COMU | 79.8 | 11.6 / 88.4 |

As shown in Table 9, it was found that both of the conversion and diastereomeric ratio were high in the two-layer conditions of organic solvent and water (conditions A), while either or both of the conversion and diastereomeric ratio were significantly low in the one-layer conditions of organic solvent only (conditions B) and the conditions using COMU as a condensation agent (conditions C).

(Example 10) Condensation reaction of Cbz-MeLeu-Ile-MeLeu-Ile-OH and H-MeGly(cPent)-MeAsp(OtBu)-$NMe_2$

The condensation reaction was carried out under the following conditions A to C.

[Formula 10]

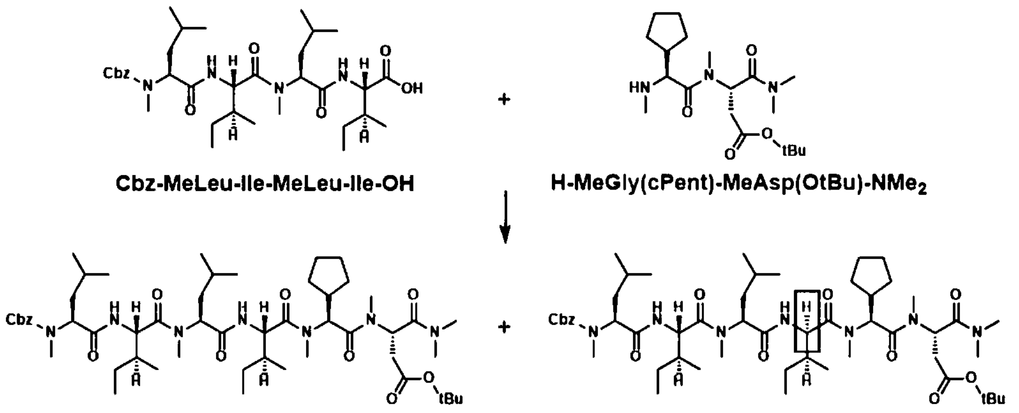

Cbz-MeLeu-Ile-MeLeu-Ile-OH

H-MeGly(cPent)-MeAsp(OtBu)-NMe$_2$

desired product

epimer

Conditions A

To a test tube with a stirrer, a solution of Cbz-MeLeu-Ile-MeLeu-Ile-OH in IPAC (concentration 116.2 mg/mL, 0.597 mL, 69.4 mg, 0.110 mmol) and 2,6-lutidine (14.2 μL, 0.122 mmol) were added, and the mixture was cooled to -10°C while stirring. To the reaction mixture, PivCl (12.5 μL, 0.101 mmol) was added, and the mixture was stirred for 7 hours. To the reaction mixture, a solution of H-MeGly(cPent)-MeAsp(OtBu)-NMe$_2$ in toluene (concentration 140.2 mg/mL, 0.214 mL, 30.0 mg, 0.081 mmol) and an aqueous $Na_2CO_3$ solution (25.8 mg $Na_2CO_3$, 0.258 mL $H_2O$) were added. After the temperature was raised to 0°C, the mixture was stirred continuously for 24 hours.

Conditions B

To a test tube with a stirrer, a solution of Cbz-MeLeu-Ile-MeLeu-Ile-OH in IPAC (concentration 116.2 mg/mL, 0.597 mL, 69.4 mg, 0.110 mmol) and 2,6-lutidine (14.1 μL, 0.122 mmol) were added, and the mixture was cooled to -10°C while stirring. To the reaction mixture, PivCl (12.5 μL, 0.101 mmol) was added, and the mixture was stirred for 7 hours. To the reaction mixture, a solution of H-MeGly(cPent)-MeAsp(OtBu)-NMe$_2$ in toluene (concentration 140.2 mg/mL, 0.214 mL, 30.0 mg, 0.081 mmol) and DIPEA (42.4 μL, 0.244 mmol) were added. After the temperature was raised to 0°C, the mixture was stirred continuously for 24 hours.

Conditions C

To a test tube with a stirrer, COMU (34.8 mg, 0.081 mmol), a solution of Cbz-MeLeu-Ile-MeLeu-Ile-OH in IPAC (concentration 116.2 mg/mL, 0.298 mL, 34.7 mg, 0.055 mmol), a solution of H-MeGly(cPent)-MeAsp(OtBu)-NMe$_2$ in toluene (concentration 140.2 mg/mL, 0.107 mL, 15.0 mg, 0.041 mmol), and DIPEA (14.1 μL, 0.081 mmol) were sequentially added, and the mixture was stirred.

Measurement method: HPLC Method A
Retention time: H-MeGly(cPent)-MeAsp(OtBu)-NMe$_2$: 5.6 min, desired product: 18.3 min, epimer: 19.4 min
Mass spectrometry: H-MeGly(cPent)-MeAsp(OtBu)-NMe$_2$: m/z 370.39 ([M+H]$^+$), desired product: m/z 1007.04 ([M+Na]$^+$), epimer: m/z 1006.86 ([M+Na]$^+$)

[Table 10]

| Reaction conditions | Analysis results | |
|---|---|---|
| | Conversion (%) | Diastereomeric ratio |
| Conditions A: Two-layer conditions of organic solvent and water | 90.0 | 100.0 / 0.0 |
| Conditions B: One-layer conditions of organic solvent only | 71.4 | 99.8 / 0.2 |
| Conditions C: Condensation conditions using COMU | 38.2 | 48.5 / 51.5 |

As shown in Table 10, it was found that both of the conversion and diastereomeric ratio were high in the two-layer conditions of organic solvent and water (conditions A), while either or both of the conversion and diastereomeric ratio were significantly low in the one-layer conditions of organic solvent only (conditions B) and the conditions using COMU as a condensation agent (conditions C).

(Example 11) Condensation reaction of Cbz-MeLeu-Ile-OH and H-cLeu-MeGly(cPent)-MeAsp(OtBu)-$NMe_2$

The condensation reaction was carried out under the following conditions A to C.

[Formula 11]

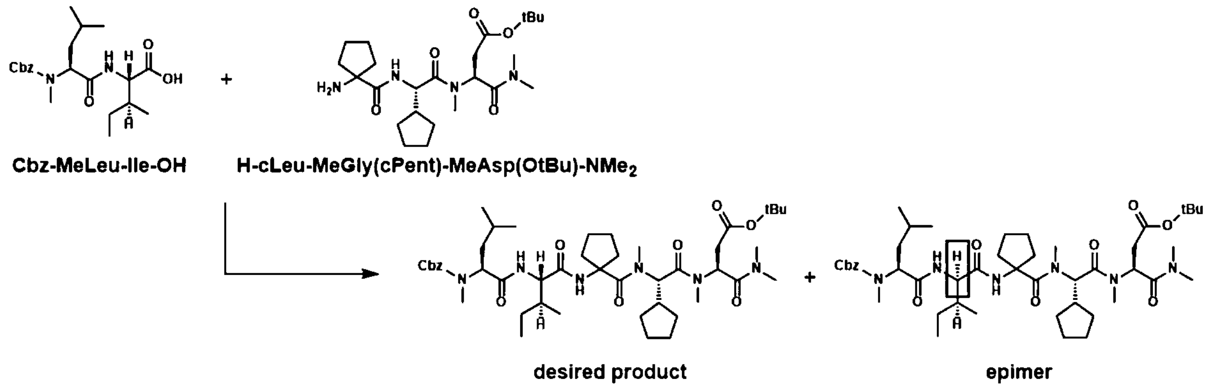

Cbz-MeLeu-Ile-OH

H-cLeu-MeGly(cPent)-MeAsp(OtBu)-$NMe_2$

desired product

epimer

Conditions A

To a test tube with a stirrer, a solution of Cbz-MeLeu-Ile-OH in IPAC (concentration 185.0 mg/mL, 0.298 mL, 55.1 mg, 0.140 mmol) and 2,6-lutidine (18.2 μL, 0.156 mmol) were added, and the mixture was cooled to -10°C while stirring. To the reaction mixture, PivCl (16.0 μL, 0.130 mmol) was added, and the mixture was stirred for 3 hours and 30 minutes. To the reaction mixture, a solution of H-cLeu-MeGly(cPent)-MeAsp(OtBu)-$NMe_2$ in toluene (concentration 128.7 mg/mL, 0.389 mL, 50.0 mg, 0.104 mmol) and an aqueous $Na_2CO_3$ solution (33.1 mg $Na_2CO_3$, 0.331 mL $H_2O$) were added. After the temperature was raised to 0°C, the mixture was stirred continuously for 19 hours.

Conditions B

To a test tube with a stirrer, a solution of Cbz-MeLeu-Ile-OH in IPAC (concentration 185.0 mg/mL, 0.298 mL, 55.1 mg, 0.140 mmol) and 2,6-lutidine (18.1 μL, 0.156 mmol) were added, and the mixture was cooled to -10°C while stirring. To the reaction mixture, PivCl (16.0 μL, 0.130 mmol) was added, and the mixture was stirred for 3 hours and 30 minutes. To the reaction mixture, a solution of H-cLeu-MeGly(cPent)-MeAsp(OtBu)-$NMe_2$ in toluene (concentration 128.7 mg/mL, 0.389 mL, 50.0 mg, 0.104 mmol) and DIPEA (54.4 μL, 0.312 mmol) were added. After the temperature was raised to 0°C, the mixture was stirred continuously for 19 hours.

Conditions C

To a test tube with a stirrer, COMU (44.7 mg, 0.104 mmol), a solution of Cbz-MeLeu-Ile-OH in IPAC (concentration 185.0 mg/mL, 0.149 mL, 27.6 mg, 0.070 mmol), a solution of H-cLeu-MeGly(cPent)-MeAsp(OtBu)-$NMe_2$ in toluene (concentration 128.7 mg/mL, 0.194 mL, 25.0 mg, 0.052 mmol), and DIPEA (18.1 μL, 0.104 mmol) were sequentially added, and the mixture was stirred.

Synthesis of epimer

The epimer was synthesized according to conditions A except for using Cbz-MeLeu-D-allo-Ile-OH instead of Cbz-MeLeu-Ile-OH.

Measurement method: HPLC Method A
Retention time: H-cLeu-MeGly(cPent)-MeAsp(OtBu)-$NMe_2$: 7.6 min, desired product: 16.4 min, epimer: 16.9 min
Mass spectrometry: H-cLeu-MeGly(cPent)-MeAsp(OtBu)-$NMe_2$: m/z 481.43 ($[M+H]^+$), desired product: m/z 877.81

([M+Na]+), epimer: m/z 877.79 ([M+Na]+)

[Table 11]

| Reaction conditions | Analysis results | |
|---|---|---|
| | Conversion (%) | Diastereomeric ratio |
| Conditions A: Two-layer conditions of organic solvent and water | 100.0 | 100.0 / 0.0 |
| Conditions B: One-layer conditions of organic solvent only | 88.0 | 99.7 / 0.3 |
| Conditions C: Condensation conditions using COMU | 94.3 | 37.5 / 62.5 |

As shown in Table 11, it was found that both of the conversion and diastereomeric ratio were high in the two-layer conditions of organic solvent and water (conditions A), while either or both of the conversion and diastereomeric ratio were lower in the one-layer conditions of organic solvent only (conditions B) and the conditions using COMU as a condensation agent (conditions C) compared to those in conditions A.

(Example 12) Evaluation of effect of base and solvent on mixed acid anhydride formation

The condensation reaction was carried out under the conditions shown in Table 12 below.

[Formula 12]

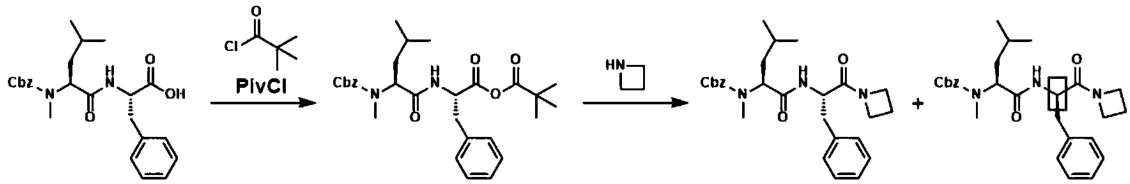


Cbz-MeLeu-Phe-OH Cbz-MeLeu-Phe-OPiv Cbz-MeLeu-Phe-Aze epimer

To a test tube with a stirrer, a solution of Cbz-MeLeu-Phe-OH in each solvent (concentration 198.0 mg/mL, 0.300 mL, 59.4 mg, 0.139 mmol) and each base (0.153 mmol) were added, and the mixture was cooled to -20°C while stirring. To the reaction mixture, PivCl (16.5 μL, 0.134 mmol) was added, and the mixture was stirred. 5.0 μL of the reaction mixture was collected over time, and dissolved in MeCN (1.0 mL) and azetidine (0.1 mL), then analyzed by HPLC.

Measurement method: HPLC Method A
Retention time: Cbz-MeLeu-Phe-OH: 13.1 min, Cbz-MeLeu-Phe-Aze: 13.35 min, epimer: 13.44 min
Mass spectrometry: Cbz-MeLeu-Phe-OH: m/z 427.88 ([M+H]+), Cbz-MeLeu-Phe-Aze: m/z 466.87 ([M+H]+), epimer: m/z 466.87 ([M+H]+)

[Table 12]

| Reaction conditions | | | Analysis results (HPLC area ratio) | | |
|---|---|---|---|---|---|
| Base | Solvent | Time (min) | Cbz-MeLeu-Phe-OH | Cbz-MeLeu-Phe-Aze | epimer |
| 2,6-lutidine | MeCN | 70 | 1.4 | 98.6 | 0.0 |
| | | 430 | 1.3 | 97.8 | 0.9 |
| | 2-MeTHF | 70 | 13.9 | 86.1 | 0.0 |
| | | 420 | 3.2 | 96.8 | 0.0 |
| | IPAC | 70 | 3.6 | 96.4 | 0.0 |
| | | 430 | 1.4 | 98.6 | 0.0 |

(continued)

| Reaction conditions | | | Analysis results (HPLC area ratio) | | |
|---|---|---|---|---|---|
| Base | Solvent | Time (min) | Cbz-MeLeu-Phe-OH | Cbz-MeLeu-Phe-Aze | epimer |
| DIPEA | MeCN | 70 | 8.0 | 76.6 | 15.5 |
| | | 430 | 19.8 | 51.9 | 28.3 |
| | 2-MeTHF | 70 | 2.8 | 96.6 | 0.6 |
| | | 420 | 1.4 | 97.3 | 1.3 |
| | IPAC | 70 | 1.4 | 96.4 | 0.3 |
| | | 430 | 1.2 | 98.0 | 0.8 |

As shown in Table 12, under conditions using 2,6-lutidine or diisopropylethylamine as the base, the conversion and diastereomeric ratio were found to be high.

(Example 12-1) Effect of solvent in activation reaction of Cbz-MeLeu-Ile-OH

The activation reaction was carried out under the following conditions.

[Formula 13]

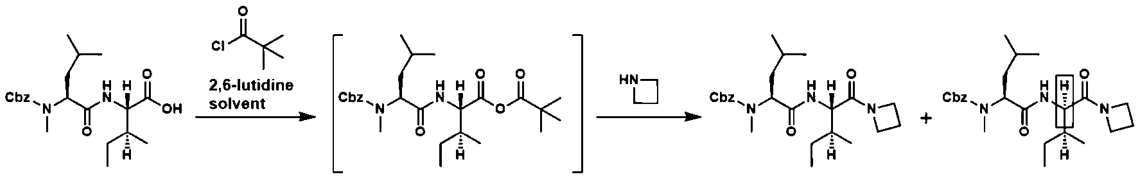


Cbz-MeLeu-Ile-OH Cbz-MeLeu-Ile-aze epimer

To a test tube with a stirrer, Cbz-MeLeu-Ile-OH (53.4 mg, 0.136 mmol), a solvent (0.400 mL) and 2,6-lutidine (17.6 μL, 0.151 mmol) were sequentially added, and the mixture was cooled to 0°C while stirring. To the reaction mixture, PivCl (15.5 μL, 0.126 mmol) was added, and the mixture was stirred. 3 μL of the reaction mixture was collected over time and dissolved in MeCN (1.0 mL) and azetidine (0.1 mL), then the conversion of the activation reaction and the diastereomeric ratio were analyzed by HPLC.

Measurement method: HPLC Method A
Retention time: Cbz-MeLeu-Ile-OH: 13.2 min, Cbz-MeLeu-Ile-Aze: 13.4 min, epimer: 13.8 min
Mass spectrometry: Cbz-MeLeu-Ile-OH: m/z 393.79 ($[M+H]^+$), Cbz-MeLeu-Ile-Aze: m/z 432.90 ($[M+H]^+$), epimer: m/z 432.84 ($[M+H]^+$)

Conversion = [(Cbz-MeLeu-Ile-aze + epimer)/(Cbz-MeLeu-Ile-OH + Cbz-MeLeu-Ile-aze + epimer)] x 100

Diastereomeric ratio = Cbz-MeLeu-Ile-aze/epimer

[Table 12-1]

| Reaction conditions | | Analysis results | |
|---|---|---|---|
| Solvent | Time (h) | Conversion (%) | Diastereomeric ratio |
| MeCN | 1 | 88.3 | 99.8 / 0.2 |
| | 5 | 86.2 | 98.7 / 1.3 |
| | 10 | 83.3 | 96.7 / 3.3 |
| THF | 1 | 69.0 | 100.0 / 0.0 |
| | 5 | 82.4 | 99.7 / 0.3 |
| | 10 | 83.9 | 99.4 / 0.6 |

(continued)

| Reaction conditions | | Analysis results | |
|---|---|---|---|
| Solvent | Time (h) | Conversion (%) | Diastereomeric ratio |
| 2-MeTHF | 1 | 66.7 | 100.0 / 0.0 |
| | 5 | 81.6 | 99.6 / 0.4 |
| | 10 | 83.6 | 99.3 / 0.7 |
| MTHP | 1 | 70.9 | 100.0 / 0.0 |
| | 5 | 83.8 | 99.7 / 0.3 |
| | 10 | 85.3 | 99.4 / 0.6 |
| CPME | 1 | 71.1 | 99.9 / 0.1 |
| | 5 | 85.1 | 99.6 / 0.4 |
| | 10 | 86.2 | 99.3 / 0.7 |
| IPAC | 1 | 83.8 | 100.0 / 0.0 |
| | 5 | 87.6 | 99.6 / 0.4 |
| | 10 | 85.9 | 99.2 / 0.8 |
| sec-BuOAc | 1 | 86.7 | 100.0 / 0.0 |
| | 5 | 90.7 | 99.7 / 0.3 |
| | 10 | 90.9 | 99.4 / 0.6 |
| cHexOAc | 1 | 80.9 | 100.0 / 0.0 |
| | 5 | 88.1 | 99.7 / 0.3 |
| | 10 | 87.7 | 99.3 / 0.7 |
| toluene | 1 | 82.4 | 100.0 / 0.0 |
| | 5 | 90.0 | 99.8 / 0.2 |
| | 10 | 90.2 | 99.5 / 0.5 |
| $CF_3$-toluene | 1 | 89.3 | 100.0 / 0.0 |
| | 5 | 90.6 | 99.7 / 0.3 |
| | 10 | 90.4 | 99.2 / 0.8 |
| chlorobenzene | 1 | 88.7 | 100.0 / 0.0 |
| | 5 | 91.4 | 99.6 / 0.4 |
| | 10 | 90.5 | 99.3 / 0.7 |
| anisole | 1 | 89.8 | 100.0 / 0.0 |
| | 5 | 90.2 | 99.6 / 0.4 |
| | 10 | 90.2 | 99.3 / 0.7 |

As shown in Table 12-1, both of the reactivity and the diastereomeric ratio resulted in high under the conditions of using an ester-based solvent such as IPAC and an aromatic ring-based solvent such as toluene.

(Example 12-2) Effect of base in activation reaction of Cbz-MeLeu-Ile-OH

The activation reaction was carried out under the following conditions.

[Formula 14]

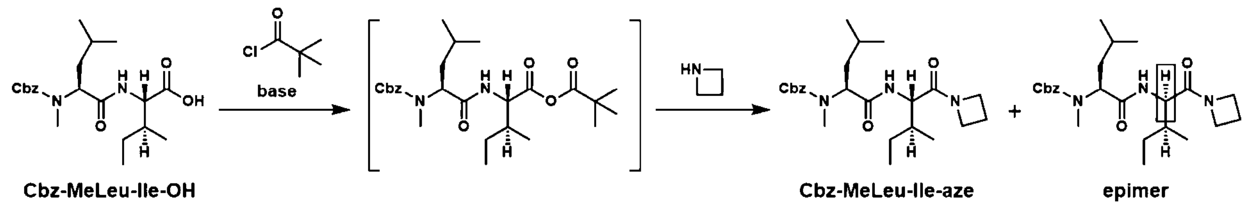


To a test tube with a stirrer, Cbz-MeLeu-Ile-OH (53.4 mg, 0.136 mmol), toluene (0.400 mL), and each base (0.151 mmol) were sequentially added, and the mixture was cooled to 0°C while stirring. To the reaction mixture, PivCl (15.5 μL, 0.126 mmol) was added, and the mixture was stirred. 3 μL of the reaction mixture was collected over time and dissolved in MeCN (1.0 mL) and azetidine (0.1 mL), then the conversion of the activation reaction and the diastereomeric ratio were analyzed by HPLC.

Measurement method: HPLC Method A
Retention time: Cbz-MeLeu-Ile-OH: 13.2 min, Cbz-MeLeu-Ile-Aze: 13.4 min, epimer: 13.8 min
Mass spectrometry: Cbz-MeLeu-Ile-OH: m/z 393.79 ($[M+H]^+$), Cbz-MeLeu-Ile-Aze: m/z 432.90 ($[M+H]^+$), epimer: m/z 432.84 ($[M+H]^+$)

Conversion = [(Cbz-MeLeu-Ile-aze + epimer)/(Cbz-MeLeu-Ile-OH + Cbz-MeLeu-Ile-aze + epimer)] x 100

Diastereomeric ratio = Cbz-MeLeu-Ile-aze/epimer

[Table 12-2]

| Reaction conditions | | Analysis results | |
|---|---|---|---|
| Base | Time (h) | Conversion (%) | Diastereomeric ratio |
| DIPEA | 1 | 88.2 | 96.4 / 3.6 |
| | 5 | 81.0 | 74.4 / 25.6 |
| | 10 | 79.0 | 61.8 / 38.2 |
| 2,4,6-collidine | 1 | 90.7 | 100.0 / 0.0 |
| | 5 | 92.2 | 99.6 / 0.4 |
| | 10 | 92.1 | 98.9 / 1.1 |
| 2,6-lutidine | 1 | 82.4 | 100.0 / 0.0 |
| | 5 | 90.0 | 99.8 / 0.2 |
| | 10 | 90.2 | 99.5 / 0.5 |
| 2,6-di-tert-butylpyridine | 1 | 0.5 | NA |
| | 5 | 1.5 | NA |
| | 10 | 2.2 | NA |
| pyridine | 1 | 86.3 | 100.0 / 0.0 |
| | 5 | 90.6 | 99.7 / 0.3 |
| | 10 | 91.2 | 99.5 / 0.5 |

As shown in Table 12-2, both of the reactivity and the diastereomeric ratio resulted in high under the conditions of using weak bases such as pyridine and 2,6-lutidine. (The diastereomeric ratio under the conditions using 2,6-di-tert-butylpyridine is not shown because the reaction conversion was significantly low (NA: Not Analyzed).)

(Example 13) Evaluation of effect of temperature on mixed acid anhydride formation

The condensation reaction was carried out under the conditions shown in Table 13 below.

[Formula 15]

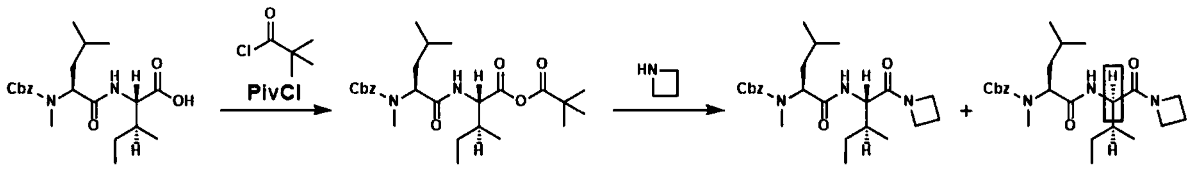


Cbz-MeLeu-Ile-OH Cbz-MeLeu-Ile-OPiv Cbz-MeLeu-Ile-Aze epimer

To a test tube with a stirrer, a solution of Cbz-MeLeu-Ile-OH in IPAC (concentration 200.0 mg/mL, 0.200 mL, 40.0 mg, 0.102 mmol) and 2,6-lutidine (11.8 μL, 0.101 mmol) were added, and the mixture was cooled to each temperature described in Table 13 while stirring. To the reaction mixture, PivCl (10.4 μL, 0.084 mmol) was added, and the mixture was stirred. 5.0 μL of the reaction mixture was collected over time, and dissolved in MeCN (1.0 mL) and azetidine (0.1 mL), then analyzed by HPLC.

Measurement method: HPLC Method A
Retention time: Cbz-MeLeu-Ile-OH: 12.9 min, Cbz-MeLeu-Ile-Aze: 13.1 min, epimer: 13.5 min
Mass spectrometry: Cbz-MeLeu-Ile-OH: m/z 393.74 ($[M+H]^+$), Cbz-MeLeu-Ile-Aze: m/z 432.95 ($[M+H]^+$), epimer: m/z 432.90 ($[M+H]^+$)

[Table 13]

| Reaction conditions | | Analysis results (HPLC area ratio) | | |
|---|---|---|---|---|
| Temperature (°C) | Time (min) | Cbz-MeLeu-Ile-OH | Cbz-MeLeu-Ile-Aze | epimer |
| -18 | 300 | 5.1 | 92.6 | 0.06 |
| | 480 | 4.6 | 93.4 | 0.07 |
| | 660 | 4.4 | 93.7 | 0.09 |
| -5 | 120 | 7.3 | 89.8 | 0.10 |
| | 300 | 6.7 | 90.8 | 0.21 |
| | 480 | 7.3 | 89.6 | 0.34 |

As shown in Table 13, the conversion and diastereomeric ratio were found to be high at both temperatures of -18°C and -5°C.

(Example 13-1) Reaction acceleration effect of additive in activation reaction of Cbz-MeLeu-Ile-OH

The activation reaction was carried out under the following conditions.

[Formula 16]

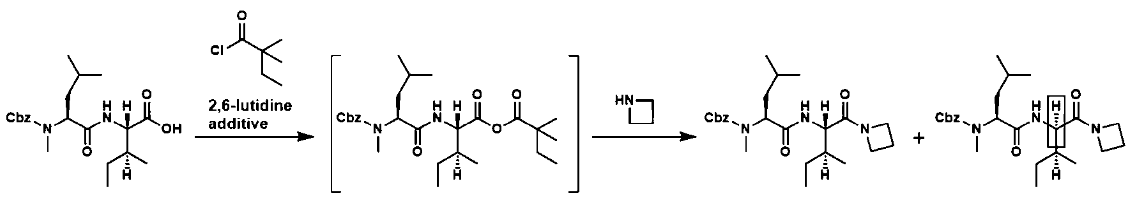


Cbz-MeLeu-Ile-OH Cbz-MeLeu-Ile-aze epimer

To a test tube with a stirrer, an additive, a solution of Cbz-MeLeu-Ile-OH in IPAC (concentration 157.0 mg/mL, 0.200 mL, 31.4 mg, 0.081 mmol) and 2,6-lutidine (10.4 μL, 0.090 mmol) were added, and the mixture was cooled to -10°C while stirring. To the reaction mixture, 2,2-dimethylbutyryl chloride (10.2 μL, 0.075 mmol) was added, and the mixture was stirred. 2-3 μL of the reaction mixture was collected over time and dissolved in MeCN (1.0 mL) and azetidine (0.1 mL), then the conversion of the activation reaction and the diastereomeric ratio were analyzed by HPLC.

Measurement method: HPLC Method A
Retention time: Cbz-MeLeu-Ile-OH: 12.8 min, Cbz-MeLeu-Ile-Aze: 13.1 min, epimer: 13.4 min
Mass spectrometry: Cbz-MeLeu-Ile-OH: m/z 393.74 ($[M+H]^+$), Cbz-MeLeu-Ile-Aze: m/z 432.95 ($[M+H]^+$), epimer: m/z 432.90 ($[M+H]^+$)

Conversion = [(Cbz-MeLeu-Ile-aze + epimer)/(Cbz-MeLeu-Ile-OH + Cbz-MeLeu-Ile-aze + epimer)] x 100

Diastereomeric ratio = Cbz-MeLeu-Ile-aze/epimer

[Table 13-1]

| Reaction conditions | | Analysis results | |
|---|---|---|---|
| Additive (equivalent) | Time (h) | Conversion (%) | Diastereomeric ratio |
| none | 1 | 67.8 | 100.0 / 0.0 |
| | 3 | 86.6 | 99.9 / 0.1 |
| NaBr (1.0 eq.) | 1 | 68.6 | 100.0 / 0.0 |
| | 3 | 89.4 | 99.9 / 0.1 |
| NaI (1.0 eq.) | 1 | 69.2 | 100.0 / 0.0 |
| | 3 | 91.9 | 99.9 / 0.1 |
| NaOTf (1.0 eq.) | 1 | 88.1 | 100.0 / 0.0 |
| | 3 | 92.4 | 99.8 / 0.2 |
| NaOTf (0.5 eq.) | 1 | 81.1 | 100.0 / 0.0 |
| | 3 | 91.7 | 99.8 / 0.2 |
| KOTf (0.5 eq.) | 1 | 78.5 | 100.0 / 0.0 |
| | 3 | 91.5 | 99.9 / 0.1 |

As shown in Table 13-1, a significant reaction acceleration effect was found under the conditions with the addition of NaOTf, KOTf, or NaI compared to the conditions without additives.

(Example 14) Evaluation of effect of solvent on fragment condensation reaction

The condensation reaction was carried out under the conditions shown in Table 14 below.

[Formula 17]

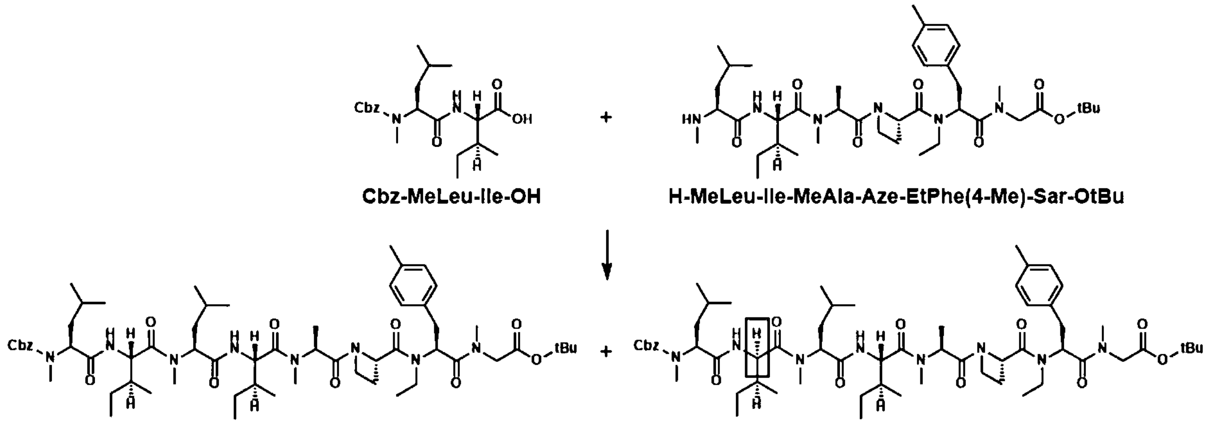

Cbz-MeLeu-Ile-OH + H-MeLeu-Ile-MeAla-Aze-EtPhe(4-Me)-Sar-OtBu

desired product + epimer

To a test tube with a stirrer, a solution of Cbz-MeLeu-Ile-OH in IPAC (concentration 185.0 mg/mL, 0.231 mL, 42.8 mg, 0.109 mmol) and 2,6-lutidine (14.0 μL, 0.121 mmol) were added, and the mixture was cooled to -10°C while stirring. To the reaction mixture, PivCl (12.4 μL, 0.101 mmol) was added, and the mixture was stirred for 3 hours. To the reaction mixture, a solution of H-MeLeu-Ile-MeAla-Aze-EtPhe(4-Me)-Sar-OtBu in each solvent (concentration 170.0 mg/mL, 0.353 mL, 60.0 mg, 0.081 mmol) and an aqueous $Na_2CO_3$ solution (25.7 mg $Na_2CO_3$, 0.257 mL $H_2O$) were added. After the temperature was raised to 0°C, the mixture was stirred continuously for 5 hours.

Measurement method: HPLC Method A
Retention time: H-MeLeu-Ile-MeAla-Aze-EtPhe(4-Me)-Sar-OtBu: 9.8 min, desired product: 17.9 min, epimer: 18.5 min
Mass spectrometry: H-MeLeu-Ile-MeAla-Aze-EtPhe(4-Me)-Sar-OtBu: m/z 743.60 ($[M+H]^+$), desired product: m/z 1117.78 ($[M+H]^+$), epimer: m/z 1117.85 ($[M+H]^+$)

[Table 14]

| Solvents of condensation reaction | Analysis results | |
|---|---|---|
| | Conversion (%) | Diastereomeric ratio |
| IPAC/Toluene/$H_2O$ | 76.0 | 100.0 / 0.0 |
| IPAC/$H_2O$ | 65.5 | 100.0 / 0.0 |
| IPAC/MTBE/$H_2O$ | 71.5 | 100.0 / 0.0 |
| IPAC/DCM/$H_2O$ | 67.9 | 100.0 / 0.0 |
| IPAC/2-MeTHF/$H_2O$ | 58.9 | 99.9 / 0.1 |
| IPAC/MeCN/$H_2O$ | 34.2 | 99.5 / 0.5 |

It was found that the conversion and diastereomeric ratio are high under the conditions using the solvents shown in Table 14.

(Example 15) Evaluation of scope of acyl halide reagent in fragment condensation reaction

The condensation reaction was carried out under the conditions shown in Table 15 below.

[Formula 18]

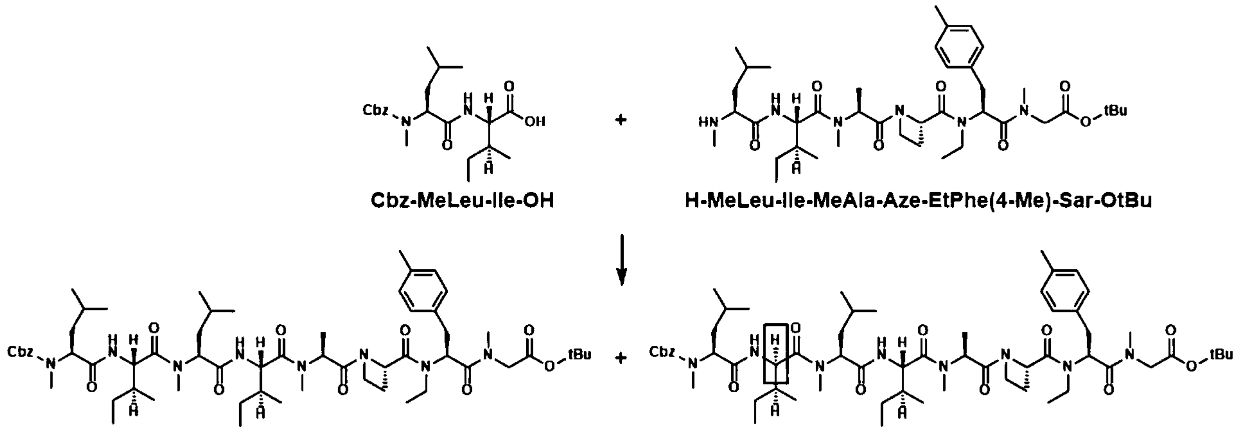

Cbz-MeLeu-Ile-OH + H-MeLeu-Ile-MeAla-Aze-EtPhe(4-Me)-Sar-OtBu

desired product + epimer

To a test tube with a stirrer, a solution of Cbz-MeLeu-Ile-OH in IPAC (concentration 185.0 mg/mL, 0.231 mL, 42.8 mg, 0.109 mmol) and 2,6-lutidine (14.0 μL, 0.121 mmol) were added, and the mixture was cooled to -10°C while stirring. To the reaction mixture, each acyl halide reagent (pivaloyl chloride (12.4 μL, 0.101 mmol), 1-methylcyclohexanecarbonyl chloride (16.1 μL, 0.101 mmol), 1-adamantanecarbonyl chloride (21.1 mg, 0.101 mmol), 2-ethylbutyryl chloride (14.0 μL, 0.101 mmol) was added, and the mixture was stirred for 3 hours. To the reaction mixture, a solution of H-MeLeu-Ile-MeAla-Aze-EtPhe(4-Me)-Sar-OtBu in toluene (concentration 170.0 mg/mL, 0.353 mL, 60.0 mg, 0.081 mmol) and an aqueous $Na_2CO_3$ solution (25.7 mg $Na_2CO_3$, 0.257 mL $H_2O$) were added. After the temperature was raised to 0°C, the mixture was stirred continuously for 5 hours.

The Example using 2,2-dimethylbutyryl chloride was carried out in the following procedure. To a test tube with a stirrer, a solution of Cbz-MeLeu-Ile-OH in IPAC (concentration 180.0 mg/mL, 0.225 mL, 40.5 mg, 0.103 mmol) and DIPEA (19.9 μL, 0.114 mmol) were added, and the mixture was cooled to -20°C while stirring (hereinafter referred to as test tube A). To the reaction mixture, 2,2-dimethylbutyryl chloride (13.4 μL, 0.098 mmol) was added, and the mixture was stirred for 1 hour and 15 minutes. To another test tube, a solution of H-MeLeu-Ile-MeAla-Aze-EtPhe(4-Me)-Sar-OtBu in toluene (concentration 200.0 mg/mL, 0.250 mL, 50.0 mg, 0.067 mmol) and an aqueous $Na_2CO_3$ solution (21.4 mg $Na_2CO_3$, 0.210 mL $H_2O$) were added, and the mixture was cooled to 0°C (hereinafter referred to as test tube B). The content in test tube A was added to test tube B, and the mixture was stirred at 0°C for 6 hours.

Measurement method: HPLC Method A
Retention time: H-MeLeu-Ile-MeAla-Aze-EtPhe(4-Me)-Sar-OtBu: 9.8 min, desired product: 17.9 min, epimer: 18.5 min
Mass spectrometry: H-MeLeu-Ile-MeAla-Aze-EtPhe(4-Me)-Sar-OtBu: m/z 743.60 ($[M+H]^+$), desired product: m/z 1117.78 ($[M+H]^+$), epimer: m/z 1117.85 ($[M+H]^+$)

[Table 15]

| Acyl halide reagent | Analysis results | |
|---|---|---|
| | Conversion (%) | Diastereomeric ratio |
| pivaloyl chloride | 82.4 | 100.0 / 0.0 |
| 2,2-dimethylbutyryl chloride | 84.0 | 100.0 / 0.0 |
| 1-methylcyclohexanecarbonyl chloride | 79.5 | 100.0 / 0.0 |
| 1-adamantanecarbonyl chloride | 78.6 | 99.9 / 0.1 |
| 2-ethylbutyryl chloride | 72.3 | 100.0 / 0.0 |

It was found that the conversion and diastereomeric ratio are high under the conditions using the acyl halide reagents shown in Table 15.

(Example 16) Evaluation of scope of base in fragment condensation reaction

The condensation reaction was carried out under the conditions shown in Table 16 below.

[Formula 19]

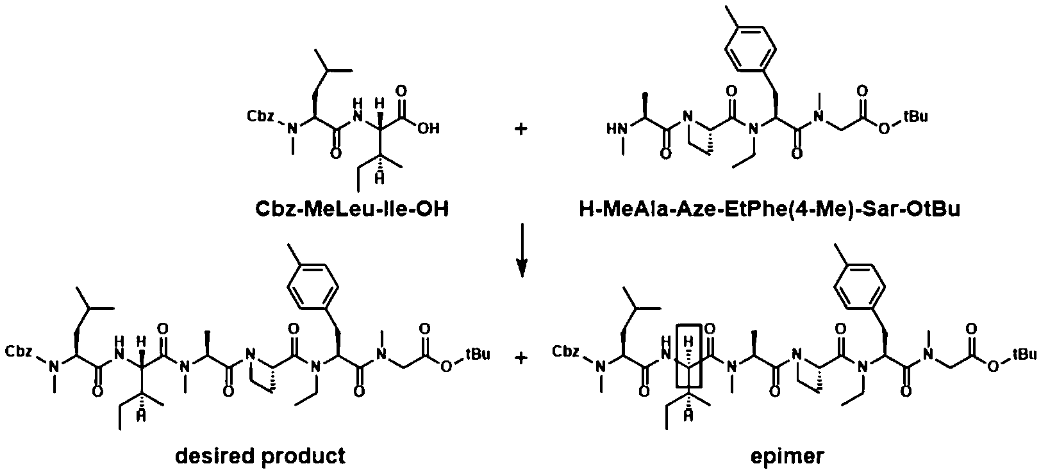

To a test tube with a stirrer, a solution of Cbz-MeLeu-Ile-OH in IPAC (concentration 185.0 mg/mL, 0.285 mL, 52.7 mg, 0.134 mmol) and 2,6-lutidine (17.3 μL, 0.149 mmol) were added, and the mixture was cooled to -10°C while stirring. To the reaction mixture, PivCl (15.3 μL, 0.124 mmol) was added, and the mixture was stirred for 5 hours. To the reaction mixture, a solution of H-MeAla-Aze-EtPhe(4-Me)-Sar-OtBu in toluene (concentration 144.2 mg/mL, 0.347 mL, 50.0 mg, 0.100 mmol), each base ($Na_2CO_3$ (31.7 mg), $K_2CO_3$ (41.9 mg), $Cs_2CO_3$ (97.6 mg), $K_3PO_4$ (64.1 mg), $NaHCO_3$ (25.7 mg), $Na_2HPO_4$ (42.5 mg), TEA (41.6 μL), pyridine (24.1 μL), 0.298 mmol), and water (0.316 mL) were added. After the temperature was raised to 0°C, the mixture was stirred continuously for 4 hours.

Measurement method: HPLC Method A
Retention time: H-MeAla-Aze-EtPhe(4-Me)-Sar-OtBu: 7.9 min, desired product: 16.3 min, epimer: 16.8 min
Mass spectrometry: H-MeAla-Aze-EtPhe(4-Me)-Sar-OtBu: m/z 503.36 ($[M+H]^+$), desired product: m/z 877.71 ($[M+H]^+$), epimer: m/z 877.73 ($[M+H]^+$)

[Table 16]

| Base used for condensation reaction | Analysis results | |
|---|---|---|
| | Conversion (%) | Diastereomeric ratio |
| None | 86.7 | 99.9 / 0.1 |
| $Na_2CO_3$ | 98.1 | 100.0 / 0.0 |
| $K_2CO_3$ | 98.8 | 100.0 / 0.0 |
| $Cs_2CO_3$ | 99.0 | 100.0 / 0.0 |
| $K_3PO_4$ | 94.5 | 100.0 / 0.0 |
| $NaHCO_3$ | 84.6 | 99.6 / 0.4 |
| $Na_2HPO_4$ | 88.8 | 99.9 / 0.1 |
| TEA | 97.9 | 100.0 / 0.0 |
| pyridine | 85.9 | 99.8 / 0.2 |

It was found that the conversion and diastereomeric ratio are high under the conditions using the bases shown in Table 16 and the use of a stronger base further increases the conversion and diastereomeric ratio.

(Example 17) Condensation reaction of Cbz-Leu-Ile-OH and H-MeAla-Aze-EtPhe(4-Me)-Sar-OtBu

The condensation reaction was carried out under the following conditions A and B.

[Formula 20]

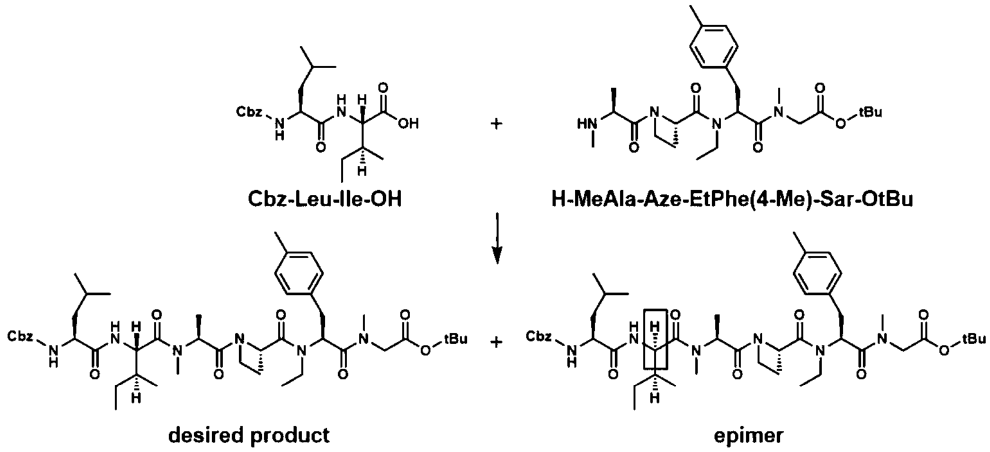

Cbz-Leu-Ile-OH

H-MeAla-Aze-EtPhe(4-Me)-Sar-OtBu

desired product

epimer

Conditions A

To a test tube with a stirrer, Cbz-Leu-Ile-OH (50.8 mg, 0.134 mmol), IPAC (0.3 mL), 2-MeTHF (0.05 mL), and 2,6-lutidine (17.4 μL, 0.149 mmol) were added, and the mixture was cooled to -10°C while stirring. To the reaction mixture, PivCl (15.3 μL, 0.124 mmol) was added, and the mixture was stirred for 2 hours. To the reaction mixture, 10% aqueous $K_2CO_3$ solution (0.382 mL, 41.2 mg $K_2CO_3$) and a solution of H-MeAla-Aze-EtPhe(4-Me)-Sar-OtBu in toluene (concentration 200.0 mg/mL, 0.250 mL, 50.0 mg, 0.100 mmol) were sequentially added. After the temperature was raised to 0°C, the mixture was stirred continuously for 6 hours and 30 minutes.

Conditions B

To a test tube with a stirrer, Cbz-Leu-Ile-OH (41.5 mg, 0.110 mmol), MeCN (0.25 mL), a solution of H-MeAla-Aze-EtPhe(4-Me)-Sar-OtBu in MeCN (concentration 200.0 mg/mL, 0.250 mL, 50.0 mg, 0.100 mmol), and DIPEA (34.7 μL, 0.199 mmol) were sequentially added, and the mixture was cooled to 10°C while stirring. HATU (75.6 mg, 0.199 mmol) was added to the reaction mixture, and the mixture was stirred continuously for 4 hours.

Measurement method: HPLC Method A
Retention time: H-MeAla-Aze-EtPhe(4-Me)-Sar-OtBu: 8.7 min, desired product: 16.3 min, epimer: 16.7 min
Mass spectrometry: H-MeAla-Aze-EtPhe(4-Me)-Sar-OtBu: m/z 503.79 ($[M+H]^+$), desired product: m/z 864.05 ($[M+H]^+$), epimer: m/z 864.22 ($[M+H]^+$)

[Table 17]

| Reaction conditions | Analysis results | |
|---|---|---|
| | Conversion (%) | Diastereomeric ratio |
| Conditions A: Two-layer conditions of organic solvent and water | 99.1 | 99.8 / 0.2 |
| Conditions B: Condensation conditions using HATU | 97.1 | 27.9 / 72.1 |

As shown in Table 17, it was found that both of the conversion and diastereomeric ratio were high under the two-layer conditions of organic solvent and water (conditions A), while the diastereomeric ratio was significantly low under the conditions using HATU as a condensation agent (conditions B).

(Example 18) Condensation reaction of Cbz-Leu-Ile-OH and H-MeGly(cPent)-MeAsp(OtBu)-$NMe_2$

The condensation reaction was carried out under the following conditions A and B.

[Formula 21]

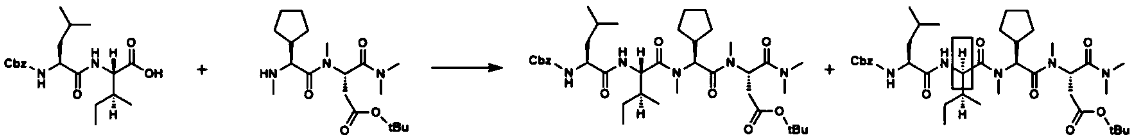

Cbz-Leu-Ile-OH H-MeGly(cPent)-MeAsp(OtBu)-$NMe_2$ desired product epimer

Conditions A

To a test tube with a stirrer, Cbz-Leu-Ile-OH (37.0 mg, 0.098 mmol), IPAC (0.3 mL) and 2,6-lutidine (13.2 µL, 0.114 mmol) were sequentially added, and the mixture was cooled to -10°C while stirring. To the reaction mixture, PivCl (11.5 µL, 0.093 mmol) was added, and the mixture was stirred for 2 hours and 55 minutes. To the reaction mixture, 10% aqueous $K_2CO_3$ solution (0.315 mL, 33.9 mg $K_2CO_3$) and a solution of H-MeGly(cPent)-MeAsp(OtBu)-$NMe_2$ in toluene (concentration 100.0 mg/mL, 0.300 mL, 30.0 mg, 0.081 mmol) were sequentially added. After the temperature was raised to 0°C, the mixture was stirred continuously for 20 hours.

Conditions B

To a test tube with a stirrer, Cbz-Leu-Ile-OH (36.9 mg, 0.097 mmol), MeCN (0.3 mL), a solution of H-MeGly(cPent)-MeAsp(OtBu)-$NMe_2$ in MeCN (concentration 75.8 mg/mL, 0.400 mL, 30.3 mg, 0.081 mmol), and DIPEA (28.3 µL, 0.162 mmol) were sequentially added, and the mixture was cooled to 10°C while stirring. HATU (61.7 mg, 0.162 mmol) was added to the reaction mixture, and the mixture was stirred continuously for 3 hours.

Measurement method: HPLC Method A
Retention time: H-MeGly(cPent)-MeAsp(OtBu)-$NMe_2$: 5.6 min, desired product: 15.5 min, epimer: 16.5 min
Mass spectrometry: H-MeGly(cPent)-MeAsp(OtBu)-$NMe_2$: m/z 370.71 ($[M+H]^+$), desired product: m/z 752.96 ($[M+Na]^+$), epimer: m/z 753.24 ($[M+Na]^+$)

[Table 18]

| Reaction conditions | Analysis results | |
|---|---|---|
| | Conversion (%) | Diastereomeric ratio |
| Conditions A: Two-layer conditions of organic solvent and water | 96.6 | 100.0 / 0.0 |
| Conditions B: Condensation conditions using HATU | 76.8 | 58.3 / 41.7 |

As shown in Table 18, it was found that both of the conversion and diastereomeric ratio were high under the two-layer conditions of organic solvent and water (conditions A), while the diastereomeric ratio was significantly low under the conditions using HATU as a condensation agent (conditions B).

(Example 19) Condensation reaction of Cbz-Leu-D-allo-Ile-OH and H-MeGly(cPent)-MeAsp(OtBu)-$NMe_2$

The condensation reaction was carried out under the following conditions A and B.

[Formula 22]

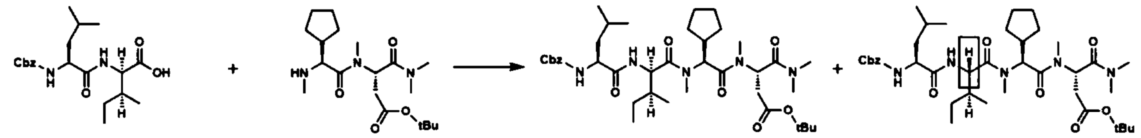

Cbz-Leu-D-allo-Ile-OH H-MeGly(cPent)-MeAsp(OtBu)-$NMe_2$ desired product epimer

To a test tube with a stirrer, Cbz-Leu-D-allo-Ile-OH (36.9 mg, 0.097 mmol), IPAC (0.3 mL) and 2,6-lutidine (13.2 μL, 0.114 mmol) were sequentially added, and the mixture was cooled to -10°C while stirring. To the reaction mixture, PivCl (11.5 μL, 0.093 mmol) was added, and the mixture was stirred for 3 hours and 30 minutes. To the reaction mixture, 10% aqueous $K_2CO_3$ solution (0.315 mL, 33.9 mg $K_2CO_3$) and a solution of H-MeGly(cPent)-MeAsp(OtBu)-$NMe_2$ in toluene (concentration 100.0 mg/mL, 0.300 mL, 30.0 mg, 0.081 mmol) were sequentially added. After the temperature was raised to 0°C, the mixture was stirred continuously for 8 hours.

Conditions B

To a test tube with a stirrer, Cbz-Leu-D-allo-Ile-OH (36.9 mg, 0.097 mmol), MeCN (0.3 mL), a solution of H-MeGly(cPent)-MeAsp(OtBu)-$NMe_2$ in MeCN (concentration 75.8 mg/mL, 0.400 mL, 30.3 mg, 0.081 mmol), and DIPEA (28.3 μL, 0.162 mmol) were sequentially added, and the mixture was cooled to 10°C while stirring. HATU (61.7 mg, 0.162 mmol) was added to the reaction mixture, and the mixture was stirred continuously for 3 hours.

Measurement method: HPLC Method A
Retention time: H-MeGly(cPent)-MeAsp(OtBu)-$NMe_2$: 5.6 min, desired product: 16.5 min, epimer: 15.5 min
Mass spectrometry: H-MeGly(cPent)-MeAsp(OtBu)-$NMe_2$: m/z 370.71 ($[M+H]^+$), desired product: m/z 685.98 ($[M-NMe_2]^+$), epimer: m/z 685.98 ($[M-NMe_2]^+$)

[Table 19]

| Reaction conditions | Analysis results | |
|---|---|---|
| | Conversion (%) | Diastereomeric ratio |
| Conditions A: Two-layer conditions of organic solvent and water | 99.0 | 100.0 / 0.0 |
| Conditions B: Condensation conditions using HATU | 55.3 | 54.9 / 45.1 |

As shown in Table 19, it was found that both of the conversion and diastereomeric ratio were high under the two-layer conditions of organic solvent and water (conditions A), while the diastereomeric ratio was significantly low under the conditions using HATU as a condensation agent (conditions B).

(Example 20) Condensation reaction of Cbz-D-Leu-Ile-OH and H-MeGly(cPent)-MeAsp(OtBu)-$NMe_2$

The condensation reaction was carried out under the following conditions A and B.

[Formula 23]

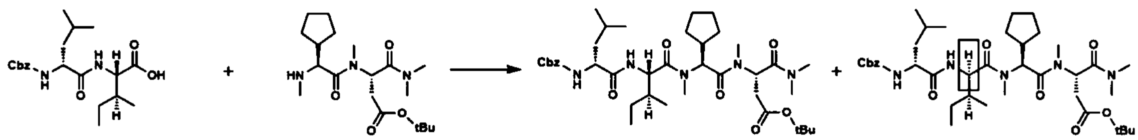

Cbz-D-Leu-Ile-OH H-MeGly(cPent)-MeAsp(OtBu)-$NMe_2$ desired product epimer

Conditions A

To a test tube with a stirrer, Cbz-D-Leu-Ile-OH (36.9 mg, 0.097 mmol), IPAC (0.3 mL) and 2,6-lutidine (13.2 μL,

0.114 mmol) were sequentially added, and the mixture was cooled to -10°C while stirring. To the reaction mixture, PivCl (11.5 μL, 0.093 mmol) was added, and the mixture was stirred for 3 hours and 30 minutes. To the reaction mixture, 10% aqueous $K_2CO_3$ solution (0.315 mL, 33.9 mg $K_2CO_3$) and a solution of H-MeGly(cPent)-MeAsp(OtBu)-$NMe_2$ in toluene (concentration 100.0 mg/mL, 0.300 mL, 30.0 mg, 0.081 mmol) were sequentially added. After the temperature was raised to 0°C, the mixture was stirred continuously for 8 hours.

Conditions B

To a test tube with a stirrer, Cbz-D-Leu-Ile-OH (36.9 mg, 0.097 mmol), MeCN (0.3 mL), a solution of H-MeGly(cPent)-MeAsp(OtBu)-$NMe_2$ in MeCN (concentration 75.8 mg/mL, 0.400 mL, 30.3 mg, 0.081 mmol), and DIPEA (28.3 μL, 0.162 mmol) were sequentially added, and the mixture was cooled to 10°C while stirring. HATU (61.7 mg, 0.162 mmol) was added to the reaction mixture, and the mixture was stirred continuously for 4 hours.

Measurement method: HPLC Method A
Retention time: H-MeGly(cPent)-MeAsp(OtBu)-$NMe_2$: 5.6 min, desired product: 15.7 min, epimer: 16.2 min
Mass spectrometry: H-MeGly(cPent)-MeAsp(OtBu)-$NMe_2$: m/z 370.71 ($[M+H]^+$), desired product: m/z 686.04 ($[M-NMe_2]^+$), epimer: m/z 686.10 ($[M-NMe_2]^+$)

[Table 20]

| Reaction conditions | Analysis results | |
|---|---|---|
| | Conversion (%) | Diastereomeric ratio |
| Conditions A: Two-layer conditions of organic solvent and water | 92.6 | 100.0 / 0.0 |
| Conditions B: Condensation conditions using HATU | 50.3 | 62.5 / 37.5 |

As shown in Table 20, it was found that both of the conversion and diastereomeric ratio were high under the two-layer conditions of organic solvent and water (conditions A), while the diastereomeric ratio was significantly low under the conditions using HATU as a condensation agent (conditions B).

(Example 21) Condensation reaction of Cbz-MeLeu-Thr(OtBu)-OH and H-MeGly(cPent)-MeAsp(OtBu)-$NMe_2$

The condensation reaction was carried out under the following conditions A and B.

[Formula 24]

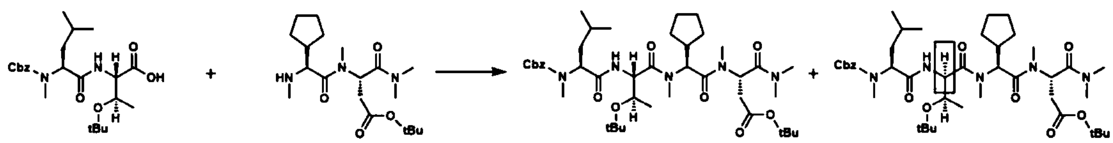

Cbz-MeLeu-Thr(OtBu)-OH H-MeGly(cPent)-MeAsp(OtBu)-$NMe_2$ desired product epimer

Conditions A

To a test tube with a stirrer, a solution of Cbz-MeLeu-Thr(OtBu)-OH in IPAC (concentration 147.0 mg/mL, 0.300 mL, 44.1 mg, 0.101 mmol) and 2,6-lutidine (13.2 μL, 0.114 mmol) were added, and the mixture was cooled to -10°C while stirring. To the reaction mixture, PivCl (11.7 μL, 0.095 mmol) was added, and the mixture was stirred for 3 hours and 30 minutes. To the reaction mixture, 10% aqueous $K_2CO_3$ solution (0.300 mL, 32.3 mg $K_2CO_3$) and a solution of H-MeGly(cPent)-MeAsp(OtBu)-$NMe_2$ in toluene (concentration 100.0 mg/mL, 0.280 mL, 28.0 mg, 0.076 mmol) were sequentially added. The temperature was then raised to 0°C, and the mixture was stirred continuously for 20 hours.

Conditions B

To a test tube with a stirrer, a solution of Cbz-MeLeu-Thr(OtBu)-OH in IPAC (concentration 147.0 mg/mL, 0.200 mL, 29.4 mg, 0.067 mmol), a solution of H-MeGly(cPent)-MeAsp(OtBu)-$NMe_2$ in MeCN (concentration 75.8 mg/mL, 0.250 mL, 19.0 mg, 0.050 mmol), and DIPEA (17.5 μL, 0.100 mmol) were sequentially added, and the mixture was cooled to 10°C while stirring. HATU (38.3 mg, 0.100 mmol) was added to the reaction mixture, and the mixture was stirred for 3 hours.

Measurement method: HPLC Method A
Retention time: H-MeGly(cPent)-MeAsp(OtBu)-$NMe_2$: 6.7 min, desired product: 18.7 min, epimer: 19.0 min
Mass spectrometry: H-MeGly(cPent)-MeAsp(OtBu)-$NMe_2$: m/z 370.77 ($[M+H]^+$), desired product: m/z 811.10 ($[M+Na]^+$), epimer: m/z 811.61 ($[M+Na]^+$)

[Table 21]

| Reaction conditions | Analysis results | |
|---|---|---|
| | Conversion (%) | Diastereomeric ratio |
| Conditions A: Two-layer conditions of organic solvent and water | 98.0 | 100.0 / 0.0 |
| Conditions B: Condensation conditions using HATU | 16.1 | 49.5 / 50.5 |

As shown in Table 21, it was found that both of the conversion and diastereomeric ratio were high under the two-layer conditions of organic solvent and water (conditions A), while the diastereomeric ratio was significantly low under the conditions using HATU as a condensation agent (conditions B).

(Example 22) Condensation reaction of Cbz-MeLeu-Thr(OtBu)-OH and H-MeGly(nPr)-Ile-Pro-OtBu

The condensation reaction was carried out under the following conditions A and B.

[Formula 25]

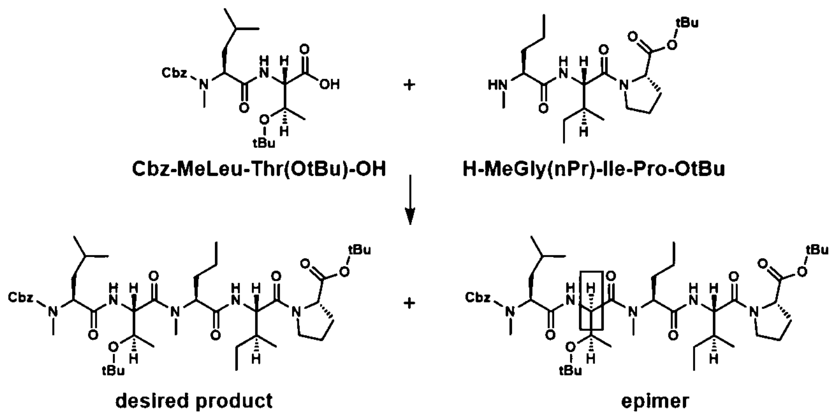


Conditions A

To a test tube with a stirrer, a solution of Cbz-MeLeu-Thr(OtBu)-OH in IPAC (concentration 147.0 mg/mL, 0.300 mL, 44.1 mg, 0.101 mmol) and 2,6-lutidine (13.2 μL, 0.114 mmol) were added, and the mixture was cooled to -10°C while stirring. To the reaction mixture, 2,2-dimethylbutyryl chloride (13.0 μL, 0.095 mmol) was added, and the mixture was stirred for 6 hours. To the reaction mixture, 10% aqueous $K_2CO_3$ solution (0.300 mL, 32.3 mg $K_2CO_3$), a solution of H-MeGly(nPr)-Ile-Pro-OtBu in toluene (concentration 100.0 mg/mL, 0.300 mL, 30.0 mg, 0.076 mmol) were sequentially added. The temperature was then raised to 0°C, and the mixture was stirred continuously for 6 hours.

Conditions B

To a test tube with a stirrer, H-MeGly(nPr)-Ile-Pro-OtBu (31.0 mg, 0.076 mmol), MeCN (0.3 mL), a solution of Cbz-MeLeu-Thr(OtBu)-OH in IPAC (concentration 147.0 mg/mL, 0.300 mL, 44.1 mg, 0.101 mmol), and DIPEA (26.3 μL, 0.151 mmol) were sequentially added, and the mixture was cooled to 10°C while stirring. HATU (57.4 mg, 0.151 mmol) was added to the reaction mixture, and the mixture was stirred for 5 hours.

Measurement method: HPLC Method A
Retention time: H-MeGly(nPr)-Ile-Pro-OtBu: 8.6 min, desired product: 19.3 min, epimer: 19.8 min
Mass spectrometry: H-MeGly(nPr)-Ile-Pro-OtBu: m/z 398.81 ($[M+H]^+$), desired product: m/z 817.37 ($[M+H]^+$), epimer: m/z 817.26 ($[M+H]^+$)

[Table 22]

| Reaction conditions | Analysis results | |
|---|---|---|
| | Conversion (%) | Diastereomeric ratio |
| Conditions A: Two-layer conditions of organic solvent and water | 94.0 | 100.0 / 0.0 |
| Conditions B: Condensation conditions using HATU | 45.1 | 47.7 / 52.3 |

As shown in Table 22, it was found that both of the conversion and diastereomeric ratio were high under the two-layer conditions of organic solvent and water (conditions A), while the diastereomeric ratio was significantly low under the conditions using HATU as a condensation agent (conditions B).

(Example 23) Condensation reaction of Cbz-MeLeu-Hph(3,5-$F_2$-4-$CF_3$)-OH and H-MeLeu-Ile-MeAla-Aze-EtPhe(4-Me)-Sar-OtBu

The condensation reaction was carried out under the following conditions A and B.

[Formula 26]

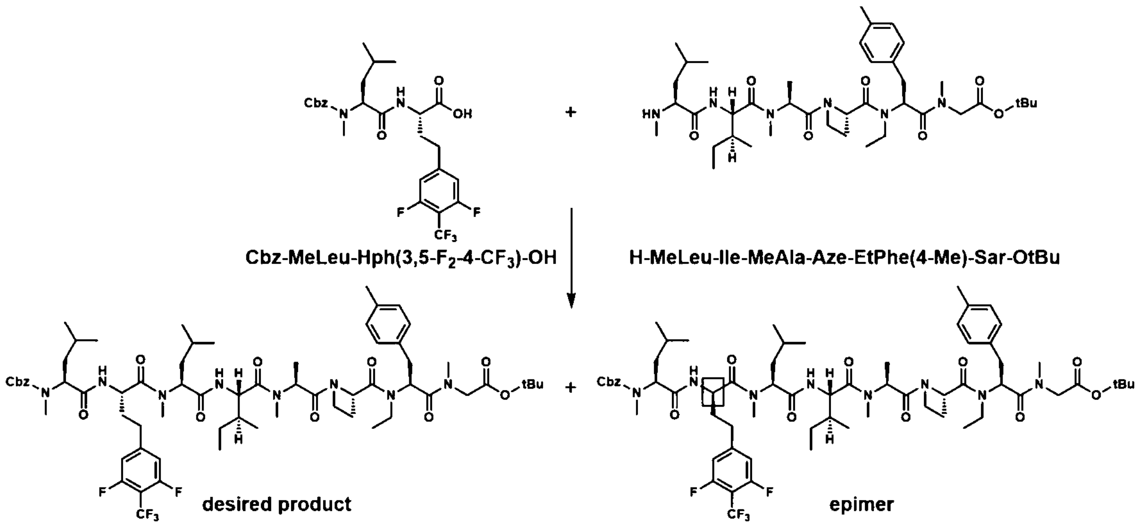

Cbz-MeLeu-Hph(3,5-$F_2$-4-$CF_3$)-OH

H-MeLeu-Ile-MeAla-Aze-EtPhe(4-Me)-Sar-OtBu

desired product

epimer

Conditions A

To a test tube with a stirrer, a solution of Cbz-MeLeu-Hph(3,5-$F_2$-4-$CF_3$)-OH in IPAC (concentration 145.0 mg/mL, 0.430 mL, 62.3 mg, 0.115 mmol) and 2,6-lutidine (15.4 μL, 0.132 mmol) were added, and the mixture was cooled to -10°C while stirring. To the reaction mixture, PivCl (13.4 μL, 0.109 mmol) was added, and the mixture was stirred for 3 hours. To the reaction mixture, 10% aqueous $K_2CO_3$ solution (0.370 mL, 39.9 mg $K_2CO_3$), a solution of H-MeLeu-Ile-MeAla-Aze-EtPhe(4-Me)-Sar-OtBu in toluene (concentration 200.0 mg/mL, 0.350 mL, 70.0 mg, 0.095 mmol) were

sequentially added. The temperature was then raised to 0°C, and the mixture was stirred continuously for 6 hours.

Conditions B

To a test tube with a stirrer, a solution of Cbz-MeLeu-Hph(3,5-$F_2$-4-$CF_3$)-OH in IPAC (concentration 145.0 mg/mL, 0.430 mL, 62.3 mg, 0.115 mmol), a solution of H-MeLeu-Ile-MeAla-Aze-EtPhe(4-Me)-Sar-OtBu in MeCN (concentration 170.0 mg/mL, 0.410 mL, 69.7 mg, 0.095 mmol), and DIPEA (33.0 μL, 0.189 mmol) were sequentially added, and the mixture was cooled to 10°C while stirring. HATU (72.0 mg, 0.189 mmol) was added to the reaction mixture, and the mixture was stirred for 3 hours.

Measurement method: HPLC Method A
Retention time: H-MeLeu-Ile-MeAla-Aze-EtPhe(4-Me)-Sar-OtBu 9.9 min, desired product: 18.9 min, epimer: 19.2 min
Mass spectrometry: H-MeLeu-Ile-MeAla-Aze-EtPhe(4-Me)-Sar-OtBu: m/z 744.18 ($[M+H]^+$), desired product: m/z 1125.35 ($[M\text{-}Sar\text{-}OtBu]^+$), epimer: m/z 1125.29 ($[M\text{-}Sar\text{-}OtBu]^+$)

[Table 23]

| Reaction conditions | Analysis results | |
|---|---|---|
| | Conversion (%) | Diastereomeric ratio |
| Conditions A: Two-layer conditions of organic solvent and water | 96.7 | 99.9 / 0.1 |
| Conditions B: Condensation conditions using HATU | 100.0 | 37.3 / 62.7 |

As shown in Table 23, it was found that both of the conversion and diastereomeric ratio were high under the two-layer conditions of organic solvent and water (conditions A), while the diastereomeric ratio was significantly low under the conditions using HATU as a condensation agent (conditions B).

(Example 24) Condensation reaction of Cbz-MeLeu-Hph(3,5-$F_2$-4-$CF_3$)-OH and H-MeGly(cPent)-MeAsp(OtBu)-$NMe_2$

The condensation reaction was carried out under the following conditions A and B.

[Formula 27]

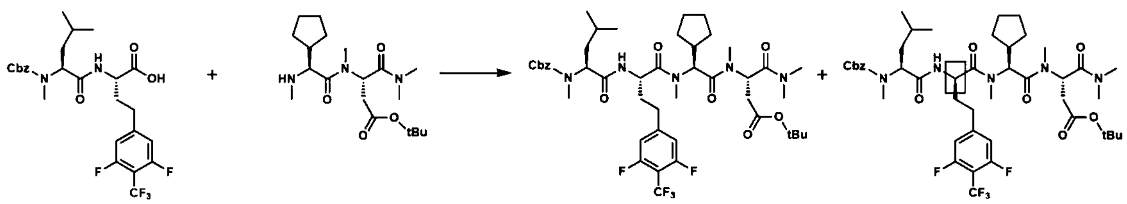

Cbz-MeLeu-Hph (3,5-$F_2$-4-$CF_3$)-OH H-MeGly(cPent)-MeAsp(OtBu)-$NMe_2$ desired product epimer

Conditions A

To a test tube with a stirrer, a solution of Cbz-MeLeu-Hph(3,5-$F_2$-4-$CF_3$)-OH in IPAC (concentration 145.0 mg/mL, 0.430 mL, 62.3 mg, 0.115 mmol) and 2,6-lutidine (15.4 μL, 0.133 mmol) were added, and the mixture was cooled to -10°C while stirring. To the reaction mixture, PivCl (13.4 μL, 0.109 mmol) was added, and the mixture was stirred for 3 hours. To the reaction mixture, 10% aqueous $K_2CO_3$ solution (0.370 mL, 39.9 mg $K_2CO_3$) and a solution of H-MeGly(cPent)-MeAsp(OtBu)-$NMe_2$ in toluene (concentration 100.0 mg/mL, 0.350 mL, 35.0 mg, 0.095 mmol) were sequentially added. The temperature was then raised to 0°C, and the mixture was stirred continuously for 6 hours.

Conditions B

To a test tube with a stirrer, a solution of Cbz-MeLeu-Hph(3,5-$F_2$-4-$CF_3$)-OH in IPAC (concentration 145.0

mg/mL, 0.430 mL, 62.3 mg, 0.115 mmol), a solution of H-MeGly(cPent)-MeAsp(OtBu)-$NMe_2$ in MeCN (concentration 75.8 mg/mL, 0.460 mL, 34.9 mg, 0.095 mmol), and DIPEA (33.0 μL, 0.189 mmol) were sequentially added, and the mixture was cooled to 10°C while stirring. HATU (72.0 mg, 0.189 mmol) was added to the reaction mixture, and the mixture was stirred for 3 hours.

Measurement method: HPLC Method A
Retention time: H-MeGly(cPent)-MeAsp(OtBu)-$NMe_2$: 5.6 min, desired product: 18.2 min, epimer: 18.5 min
Mass spectrometry: H-MeGly(cPent)-MeAsp(OtBu)-$NMe_2$: m/z 370.71 ($[M+H]^+$), desired product: m/z 852.14 ($[M-NMe_2]^+$), epimer: m/z 852.03 ($[M-NMe_2]^+$)

[Table 24]

| Reaction conditions | Analysis results | |
|---|---|---|
| | Conversion (%) | Diastereomeric ratio |
| Conditions A: Two-layer conditions of organic solvent and water | 97.4 | 99.8 / 0.2 |
| Conditions B: Condensation conditions using HATU | 96.3 | 57.2 / 42.8 |

As shown in Table 24, it was found that both of the conversion and diastereomeric ratio were high under the two-layer conditions of organic solvent and water (conditions A), while the diastereomeric ratio was significantly low under the conditions using HATU as a condensation agent (conditions B).

(Example 25) Condensation reaction of Cbz-MeLeu-Cys(SBn)-OH and H-MeAla-Aze-EtPhe(4-Me)-Sar-OtBu

The condensation reaction was carried out under the following conditions A and B.

[Formula 28]

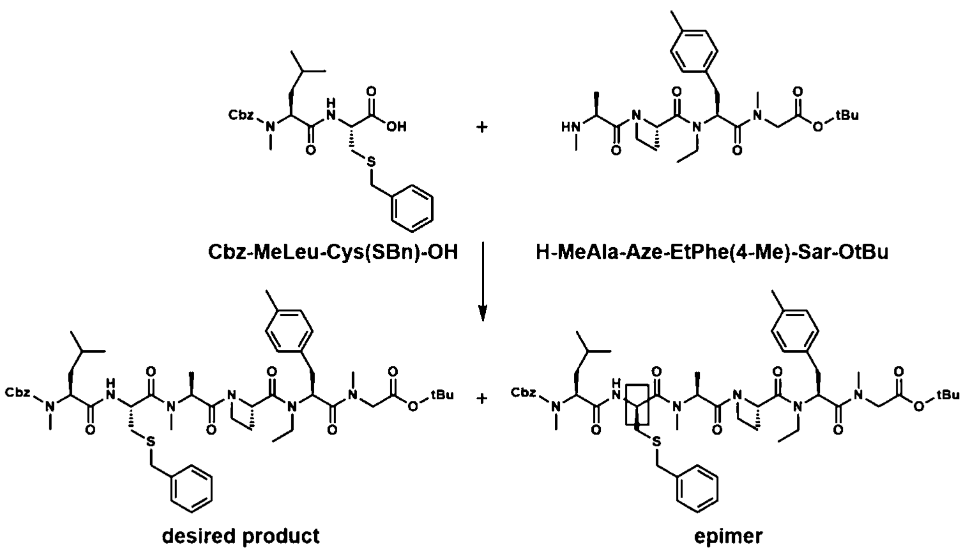
Cbz-MeLeu-Cys(SBn)-OH
H-MeAla-Aze-EtPhe(4-Me)-Sar-OtBu
desired product
epimer

Conditions A

To a test tube with a stirrer, a solution of Cbz-MeLeu-Cys(SBn)-OH in IPAC (concentration 116.0 mg/mL, 0.475 mL, 55.1 mg, 0.107 mmol) and 2,6-lutidine (13.9 μL, 0.119 mmol) were added, and the mixture was cooled to -10°C while stirring. To the reaction mixture, PivCl (12.2 μL, 0.100 mmol) was added, and the mixture was stirred for 3 hours. To the reaction mixture, 10% aqueous $K_2CO_3$ solution (0.300 mL, 32.3 mg $K_2CO_3$), a solution of H-MeAla-Aze-EtPhe(4-Me)-Sar-OtBu in toluene (concentration 169.0 mg/mL, 0.240 mL, 40.6 mg, 0.080 mmol) were sequentially added. After the temperature was raised to 0°C, the mixture was stirred continuously for 6 hours.

Conditions B

To a test tube with a stirrer, a solution of Cbz-MeLeu-Cys(SBn)-OH in IPAC (concentration 116.0 mg/mL, 0.475 mL, 55.1 mg, 0.107 mmol), a solution of H-MeAla-Aze-EtPhe(4-Me)-Sar-OtBu in MeCN (concentration 200.0 mg/mL, 0.200 mL, 40.0 mg, 0.080 mmol), and DIPEA (27.7 μL, 0.159 mmol) were sequentially added, and the mixture was cooled to 10°C while stirring. HATU (60.5 mg, 0.159 mmol) was added to the reaction mixture, and the mixture was stirred for 6 hours.

Measurement method: HPLC Method A
Retention time: H-MeAla-Aze-EtPhe(4-Me)-Sar-OtBu: 9.5 min, desired product: 18.7 min, epimer: 18.9 min
Mass spectrometry: H-MeAla-Aze-EtPhe(4-Me)-Sar-OtBu: m/z 503.92 ([M+H]$^+$), desired product: m/z 958.28 ([M+H]$^+$), epimer: m/z 958.39 ([M+H]$^+$)

[Table 25]

| Reaction conditions | Analysis results | |
|---|---|---|
| | Conversion (%) | Diastereomeric ratio |
| Conditions A: Two-layer conditions of organic solvent and water | 100.0 | 99.3 / 0.7 |
| Conditions B: Condensation conditions using HATU | 100.0 | 52.9 / 47.1 |

As shown in Table 25, it was found that both of the conversion and diastereomeric ratio were high under the two-layer conditions of organic solvent and water (conditions A), while the diastereomeric ratio was significantly low under the conditions using HATU as a condensation agent (conditions B).

(Example 26) Condensation reaction of Cbz-MeAla-Ile-OH and H-MeLeu-Ile-MeAla-Aze-EtPhe(4-Me)-Sar-OtBu

The condensation reaction was carried out under the following conditions A and B.

[Formula 29]

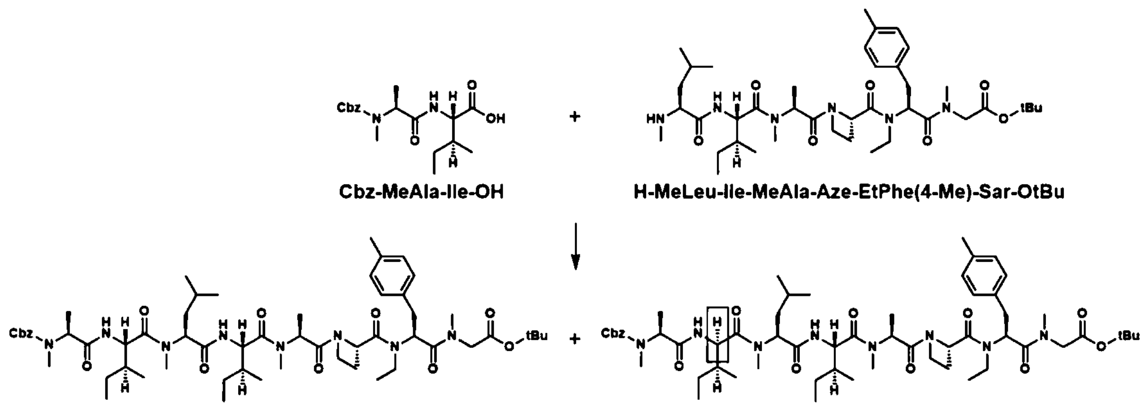

Cbz-MeAla-Ile-OH

H-MeLeu-Ile-MeAla-Aze-EtPhe(4-Me)-Sar-OtBu

desired product

epimer

Conditions A

To a test tube with a stirrer, Cbz-MeAla-Ile-OH (37.5 mg, 0.102 mmol), IPAC (0.300 mL) and 2,6-lutidine (13.2 μL, 0.113 mmol) were sequentially added, and the mixture was cooled to -10°C while stirring. To the reaction mixture, PivCl (11.6 μL, 0.094 mmol) was added, and the mixture was stirred for 3 hours. To the reaction mixture, 10% aqueous $K_2CO_3$ solution (0.300 mL, 32.3 mg $K_2CO_3$) and a solution of H-MeLeu-Ile-MeAla-Aze-EtPhe(4-Me)-Sar-OtBu in toluene (concentration 200.0 mg/mL, 0.280 mL, 56.0 mg, 0.075 mmol) were sequentially added. The temperature was then raised to 0°C, the mixture was stirred continuously for 20 hours.

Conditions B

To a test tube with a stirrer, Cbz-MeAla-Ile-OH (37.5 mg, 0.102 mmol), H-MeLeu-Ile-MeAla-Aze-EtPhe(4-Me)-Sar-OtBu (56.0 mg, 0.075 mmol), MeCN (0.500 mL), and DIPEA (26.3 μL, 0.151 mmol) were sequentially added, and the mixture was cooled to 10°C while stirring. HATU (57.3 mg, 0.151 mmol) was added to the reaction mixture, and the mixture was stirred for 6 hours.

Measurement method: HPLC Method A
Retention time: H-MeLeu-Ile-MeAla-Aze-EtPhe(4-Me)-Sar-OtBu 10.9 min, desired product: 17.8 min, epimer: 18.4 min
Mass spectrometry: H-MeLeu-Ile-MeAla-Aze-EtPhe(4-Me)-Sar-OtBu: m/z 744.12 ($[M+H]^+$), desired product: m/z 931.43 ($[M\text{-}Sar\text{-}OtBu]^+$), epimer: m/z 931.49 ($[M\text{-}Sar\text{-}OtBu]^+$)

[Table 26]

| Reaction conditions | Analysis results | |
|---|---|---|
| | Conversion (%) | Diastereomeric ratio |
| Conditions A: Two-layer conditions of organic solvent and water | 99.0 | 100.0 / 0.0 |
| Conditions B: Condensation conditions using HATU | 94.2 | 25.0 / 75.0 |

As shown in Table 26, it was found that both of the conversion and diastereomeric ratio were high under the two-layer conditions of organic solvent and water (conditions A), while the diastereomeric ratio was significantly low under the conditions using HATU as a condensation agent (conditions B).

(Example 27) Condensation reaction of Cbz-MeAla-Ile-OH and H-MeGly(nPr)-Ile-Pro-OtBu

The condensation reaction was carried out under the following conditions A and B.

[Formula 30]

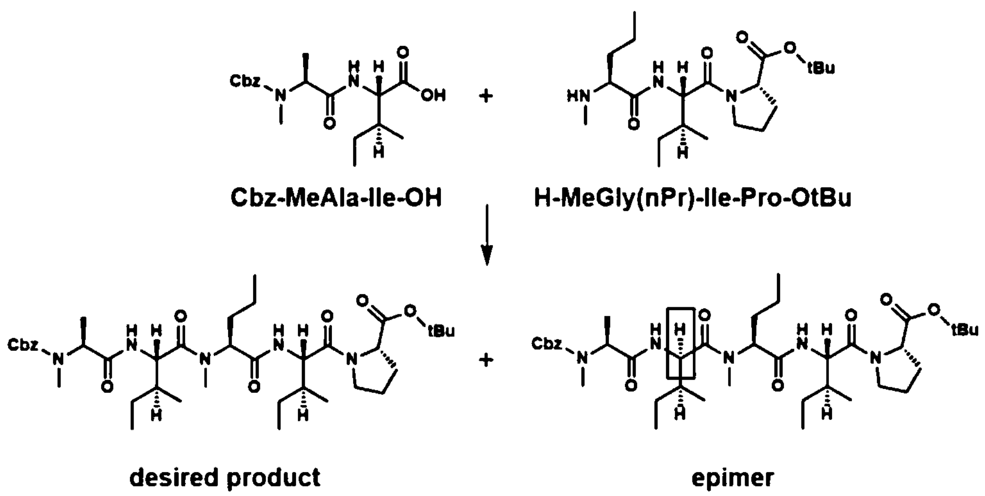


Conditions A

To a test tube with a stirrer, Cbz-MeAla-Ile-OH (37.5 mg, 0.102 mmol), IPAC (0.300 mL), and 2,6-lutidine (13.2 μL, 0.113 mmol) were sequentially added, and the mixture was cooled to -10°C while stirring. To the reaction mixture, PivCl (11.6 μL, 0.094 mmol) was added, and the mixture was stirred for 3 hours. To the reaction mixture, 10% aqueous $K_2CO_3$ solution (0.300 mL, 32.3 mg $K_2CO_3$) and a solution of H-MeGly(nPr)-Ile-Pro-OtBu in toluene (concentration 100.0 mg/mL, 0.300 mL, 30.0 mg, 0.076 mmol) were sequentially added. The temperature was then raised to 0°C, and the mixture was stirred continuously for 20 hours.

Conditions B

To a test tube with a stirrer, Cbz-MeAla-Ile-OH (37.6 mg, 0.102 mmol), H-MeGly(nPr)-Ile-Pro-OtBu (30.0 mg, 0.075 mmol), MeCN (0.500 mL), and DIPEA (26.3 μL, 0.151 mmol) were sequentially added, and the mixture was cooled to 10°C while stirring. HATU (57.4 mg, 0.151 mmol) was added to the reaction mixture, and the mixture was stirred for 6 hours.

Measurement method: HPLC Method A
Retention time: H-MeGly(nPr)-Ile-Pro-OtBu: 8.6 min, desired product: 17.1 min, epimer: 17.6 min
Mass spectrometry: H-MeGly(nPr)-Ile-Pro-OtBu: m/z 398.75 ([M+H]$^+$), desired product: m/z 731.24 ([M+H]$^+$), epimer: m/z 731.18 ([M+H]$^+$)

[Table 27]

| Reaction conditions | Analysis results | |
|---|---|---|
| | Conversion (%) | Diastereomeric ratio |
| Conditions A: Two-layer conditions of organic solvent and water | 98.3 | 100.0 / 0.0 |
| Conditions B: Condensation conditions using HATU | 86.5 | 28.4 / 71.6 |

As shown in Table 27, it was found that both of the conversion and diastereomeric ratio were high under the two-layer conditions of organic solvent and water (conditions A), while the diastereomeric ratio was significantly low under the conditions using HATU as a condensation agent (conditions B).

(Example 28) Condensation reaction of Cbz-MeLeu-MeAla-Ile-OH and H-MeLeu-Ile-MeAla-Aze-EtPhe(4-Me)-Sar-OtBu

The condensation reaction was carried out under the following conditions A and B.

[Formula 31]

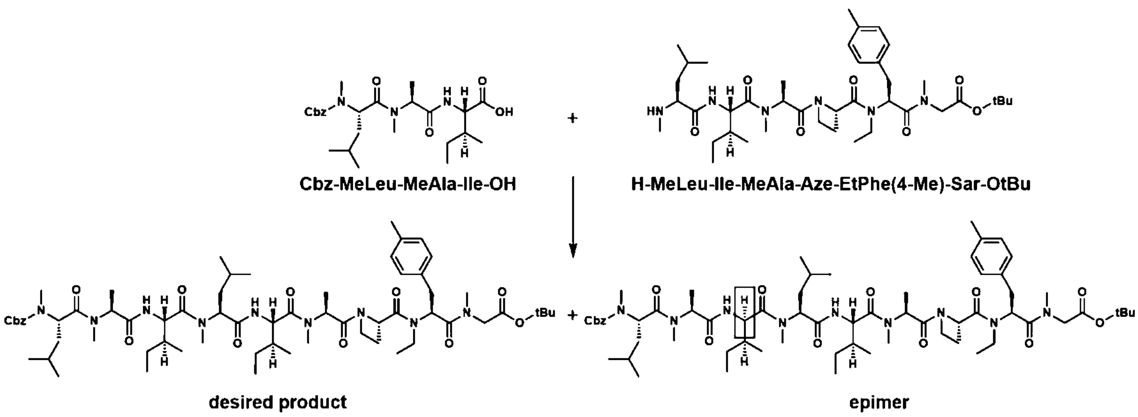


Conditions A

To a test tube with a stirrer, a solution of Cbz-MeLeu-MeAla-Ile-OH in IPAC (concentration 146.0 mg/mL, 0.330 mL, 48.2 mg, 0.102 mmol) and 2,6-lutidine (13.2 μL, 0.114 mmol) were added, and the mixture was cooled to -10°C while stirring. To the reaction mixture, PivCl (11.6 μL, 0.095 mmol) was added, and the mixture was stirred for 8 hours. To the reaction mixture, 10% aqueous $K_2CO_3$ solution (0.300 mL, 32.3 mg $K_2CO_3$) and a solution of H-MeLeu-Ile-MeAla-Aze-EtPhe(4-Me)-Sar-OtBu in toluene (concentration 200.0 mg/mL, 0.280 mL, 56.0 mg, 0.076 mmol) were sequentially added. The temperature was then raised to 0°C, the mixture was stirred continuously for 18 hours.

Conditions B

To a test tube with a stirrer, a solution of H-MeLeu-Ile-MeAla-Aze-EtPhe(4-Me)-Sar-OtBu in MeCN (concentration 170.0 mg/mL, 0.330 mL, 56.1 mg, 0.076 mmol), a solution of Cbz-MeLeu-MeAla-Ile-OH in IPAC (concentration 146.0 mg/mL, 0.330 mL, 48.2 mg, 0.102 mmol), and DIPEA (26.3 μL, 0.152 mmol) were sequentially added, and the mixture was cooled to 10°C while stirring. HATU (57.4 mg, 0.152 mmol) was added to the reaction mixture, and the mixture was stirred for 14 hours.

Measurement method: HPLC Method A
Retention time: H-MeLeu-Ile-MeAla-Aze-EtPhe(4-Me)-Sar-OtBu 10.9 min, desired product: 19.1 min, epimer: 19.8 min
Mass spectrometry: H-MeLeu-Ile-MeAla-Aze-EtPhe(4-Me)-Sar-OtBu: m/z 744.07 ($[M+H]^+$), desired product: m/z 1058.43 ($[M\text{-}Sar\text{-}OtBu]^+$), epimer: m/z 1059.45 ($[M\text{-}Sar\text{-}OtBu]^+$)

[Table 28]

| Reaction conditions | Analysis results | |
|---|---|---|
| | Conversion (%) | Diastereomeric ratio |
| Conditions A: Two-layer conditions of organic solvent and water | 97.0 | 100.0 / 0.0 |
| Conditions B: Condensation conditions using HATU | 95.9 | 41.5 / 58.5 |

As shown in Table 28, it was found that both of the conversion and diastereomeric ratio were high under the two-layer conditions of organic solvent and water (conditions A), while the diastereomeric ratio was significantly low under the conditions using HATU as a condensation agent (conditions B).

(Example 29) Condensation reaction of Cbz-MeLeu-MeAla-Ile-OH and H-MeGly(nPr)-Ile-Pro-OtBu

The condensation reaction was carried out under the following conditions A and B.

[Formula 32]

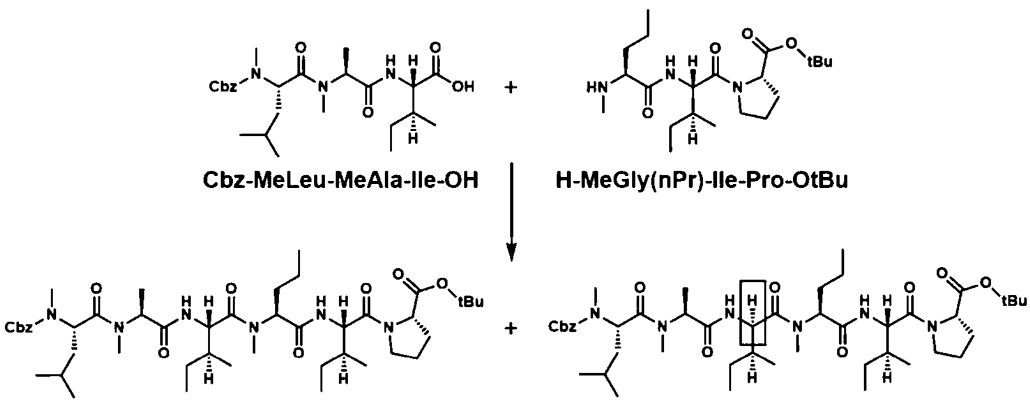
Cbz-MeLeu-MeAla-Ile-OH
H-MeGly(nPr)-Ile-Pro-OtBu
desired product
epimer

Conditions A

To a test tube with a stirrer, a solution of Cbz-MeLeu-MeAla-Ile-OH in IPAC (concentration 146.0 mg/mL, 0.330 mL, 48.2 mg, 0.102 mmol) and 2,6-lutidine (13.2 μL, 0.114 mmol) were added, and the mixture was cooled to -10°C while stirring. To the reaction mixture, PivCl (11.6 μL, 0.095 mmol) was added, and the mixture was stirred for 6 hours. To the reaction mixture, 10% aqueous $K_2CO_3$ solution (0.300 mL, 32.3 mg $K_2CO_3$) and a solution of H-MeGly(nPr)-Ile-Pro-OtBu in toluene (concentration 100.0 mg/mL, 0.300 mL, 30.0 mg, 0.076 mmol) were sequentially added. The temperature was then raised to 0°C, and the mixture was stirred continuously for 18 hours.

Conditions B

To a test tube with a stirrer, H-MeGly(nPr)-Ile-Pro-OtBu (30.0 mg, 0.076 mmol), a solution of Cbz-MeLeu-MeAla-Ile-OH in IPAC (concentration 146.0 mg/mL, 0.330 mL, 48.2 mg, 0.102 mmol), and DIPEA (26.3 μL, 0.152 mmol) were sequentially added, and the mixture was cooled to 10°C while stirring. HATU (57.4 mg, 0.152 mmol) was added to the reaction mixture, and the mixture was stirred for 5 hours.

Measurement method: HPLC Method A
Retention time: H-MeGly(nPr)-Ile-Pro-OtBu: 8.5 min, desired product: 18.7 min, epimer: 19.2 min
Mass spectrometry: H-MeGly(nPr)-Ile-Pro-OtBu: m/z 398.87 ($[M+H]^+$), desired product: m/z 858.24 ($[M+H]^+$), epimer: m/z 858.18 ($[M+H]^+$)

[Table 29]

| Reaction conditions | Analysis results | |
|---|---|---|
| | Conversion (%) | Diastereomeric ratio |
| Conditions A: Two-layer conditions of organic solvent and water | 98.2 | 100.0 / 0.0 |
| Conditions B: Condensation conditions using HATU | 86.0 | 43.6 / 56.4 |

As shown in Table 29, it was found that both of the conversion and diastereomeric ratio were high under the two-layer conditions of organic solvent and water (conditions A), while the diastereomeric ratio was significantly low under the conditions using HATU as a condensation agent (conditions B).

(Example 30) Condensation reaction of Cbz-MeLeu-MeAla-Ile-OH and H-MeGly(cPent)-MeAsp(OtBu)-$NMe_2$

The condensation reaction was carried out under the following conditions A and B.

[Formula 33]

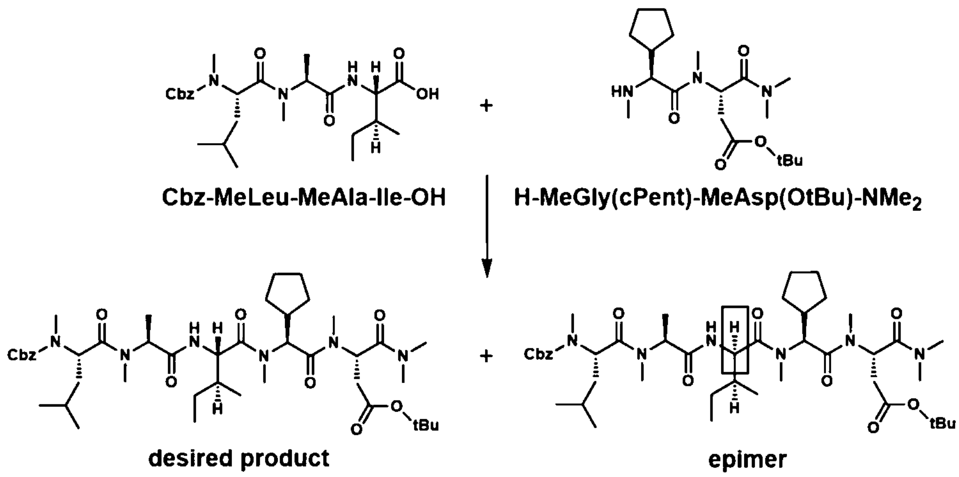

Cbz-MeLeu-MeAla-Ile-OH

H-MeGly(cPent)-MeAsp(OtBu)-$NMe_2$

desired product

epimer

Conditions A

To a test tube with a stirrer, a solution of Cbz-MeLeu-MeAla-Ile-OH in IPAC (concentration 146.0 mg/mL, 0.330 mL, 48.2 mg, 0.102 mmol) and 2,6-lutidine (13.2 μL, 0.114 mmol) were added, and the mixture was cooled to -10°C while stirring. To the reaction mixture, PivCl (11.6 μL, 0.095 mmol) was added, and the mixture was stirred for 6 hours. To the reaction mixture, 10% aqueous $K_2CO_3$ solution (0.300 mL, 32.3 mg $K_2CO_3$) and a solution of H-MeGly(cPent)-MeAsp(OtBu)-$NMe_2$ in toluene (concentration 100.0 mg/mL, 0.280 mL, 28.0 mg, 0.076 mmol) were sequentially added. The temperature was then raised to 0°C, and the mixture was stirred continuously for 18 hours.

Conditions B

To a test tube with a stirrer, a solution of Cbz-MeLeu-MeAla-Ile-OH in IPAC (concentration 146.0 mg/mL, 0.330 mL, 48.2 mg, 0.102 mmol), a solution of H-MeGly(cPent)-MeAsp(OtBu)-$NMe_2$ in 2-MeTHF (concentration 129.6 mg/mL, 0.220 mL, 28.5 mg, 0.077 mmol), and DIPEA (26.3 μL, 0.152 mmol) were sequentially added, and the mixture was cooled to 10°C while stirring. HATU (57.4 mg, 0.152 mmol) was added to the reaction mixture, and the mixture was stirred for 5 hours.

Measurement method: HPLC Method A
Retention time: H-MeGly(cPent)-MeAsp(OtBu)-$NMe_2$: 6.6 min, desired product: 17.7 min, epimer: 18.9 min
Mass spectrometry: H-MeGly(cPent)-MeAsp(OtBu)-$NMe_2$: m/z 370.71 ($[M+H]^+$), desired product: m/z 785.22 ($[M-NMe_2]^+$), epimer: m/z 785.11 ($[M-NMe_2]^+$)

[Table 30]

| Reaction conditions | Analysis results | |
|---|---|---|
| | Conversion (%) | Diastereomeric ratio |
| Conditions A: Two-layer conditions of organic solvent and water | 98.2 | 99.7 / 0.3 |
| Conditions B: Condensation conditions using HATU | 23.5 | 75.4 / 24.6 |

As shown in Table 30, it was found that both of the conversion and diastereomeric ratio were high under the two-layer conditions of organic solvent and water (conditions A), while the diastereomeric ratio was significantly low under the conditions using HATU as a condensation agent (conditions B).

(Example 31) Condensation reaction of Boc-MeLeu-Phe-OH and H-MeAla-Aze-EtPhe(4-Me)-Sar-OtBu

The condensation reaction was carried out under the following conditions A and B.

[Formula 34]

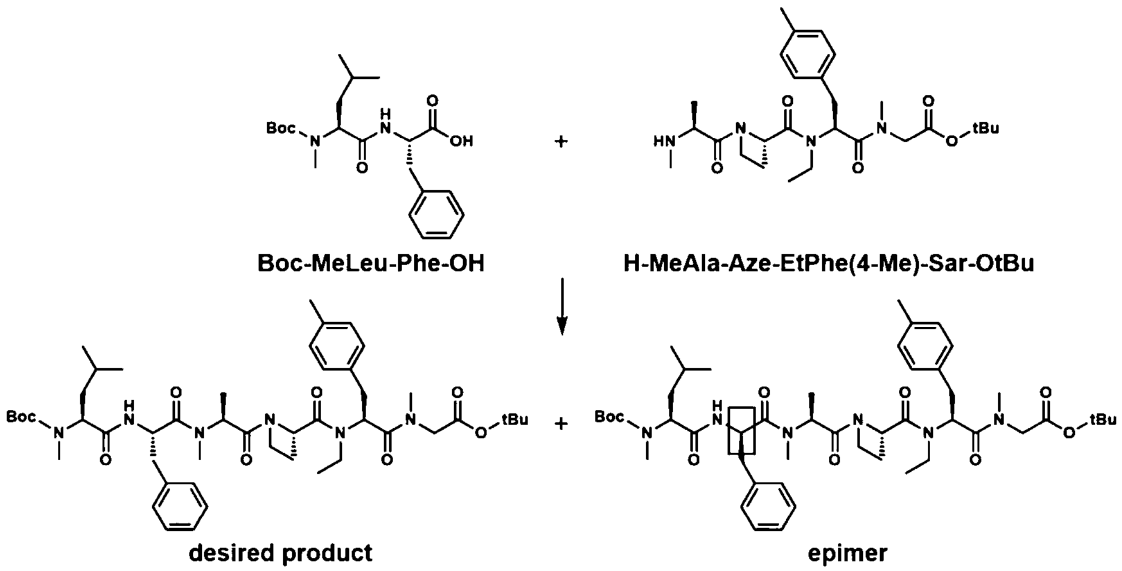


Conditions A

To a test tube with a stirrer, a solution of Boc-MeLeu-Phe-OH in IPAC (concentration 134.0 mg/mL, 0.280 mL, 37.5 mg, 0.096 mmol) and 2,6-lutidine (13.0 μL, 0.111 mmol) were added, and the mixture was cooled to -8°C while stirring. To the reaction mixture, PivCl (10.8 μL, 0.088 mmol) was added, and the mixture was stirred for 3 hours. To the reaction mixture, 10% aqueous $K_2CO_3$ solution (0.300 mL, 32.3 mg $K_2CO_3$), a solution of H-MeAla-Aze-EtPhe(4-Me)-Sar-OtBu in

toluene (concentration 169.0 mg/mL, 0.240 mL, 40.6 mg, 0.080 mmol) were sequentially added. After the temperature was raised to 0°C, the mixture was stirred continuously for 3 hours.

Conditions B

To a test tube with a stirrer, a solution of Boc-MeLeu-Phe-OH in IPAC (concentration 134.0 mg/mL, 0.280 mL, 37.5 mg, 0.096 mmol), a solution of H-MeAla-Aze-EtPhe(4-Me)-Sar-OtBu in MeCN (concentration 200 mg/mL, 0.200 mL, 40.0 mg, 0.080 mmol), and DIPEA (27.7 μL, 0.159 mmol) were sequentially added, and the mixture was cooled to 10°C while stirring. HATU (60.5 mg, 0.159 mmol) was added to the reaction mixture, and the mixture was stirred for 5 hours.

Measurement method: HPLC Method A
Retention time: H-MeAla-Aze-EtPhe(4-Me)-Sar-OtBu: 9.5 min, desired product: 18.4 min, epimer: 18.5 min
Mass spectrometry: H-MeAla-Aze-EtPhe(4-Me)-Sar-OtBu: m/z 503.92 ($[M+H]^+$), desired product: m/z 878.13 ($[M+H]^+$), epimer: m/z 878.08 ($[M+H]^+$)

[Table 31]

| Reaction conditions | Analysis results | |
|---|---|---|
| | Conversion (%) | Diastereomeric ratio |
| Conditions A: Two-layer conditions of organic solvent and water | 99.7 | 99.7 / 0.3 |
| Conditions B: Condensation conditions using HATU | 100.0 | 59.3 / 40.7 |

As shown in Table 31, it was found that both of the conversion and diastereomeric ratio were high under the two-layer conditions of organic solvent and water (conditions A), while the diastereomeric ratio was significantly low under the conditions using HATU as a condensation agent (conditions B).

(Example 32) Condensation reaction of Fmoc-MeAlGly-Val-OH and H-MeAla-Aze-EtPhe(4-Me)-Sar-OtBu

The condensation reaction was carried out under the following conditions A and B.

[Formula 35]

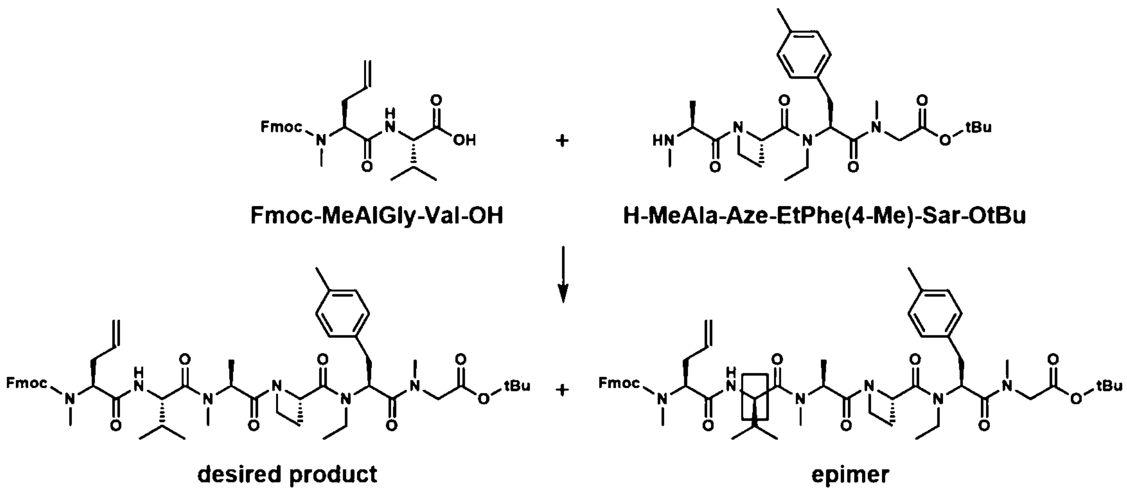

Conditions A

To a test tube with a stirrer, Fmoc-MeAlGly-Val-OH (52.6 mg, content 91.7%, net 48.4 mg, 0.107 mmol), IPAC (0.300 mL) and 2,6-lutidine (13.9 μL, 0.119 mmol) were sequentially added, and the mixture was cooled to -8°C while stirring. To the reaction mixture, PivCl (12.2 μL, 0.100 mmol) was added, and the mixture was stirred for 3 hours. To the reaction mixture, 10% aqueous $K_2CO_3$ solution (0.300 mL, 32.3 mg $K_2CO_3$) and a solution of H-MeAla-Aze-EtPhe(4-Me)-Sar-OtBu in toluene (concentration 169.0 mg/mL, 0.240 mL, 40.6 mg, 0.080 mmol) were sequentially added. After the temperature was raised to 0°C, the mixture was stirred continuously for 5 hours.

Conditions B

To a test tube with a stirrer, Fmoc-MeAlGly-Val-OH (52.6 mg, content 91.7%, net 48.4 mg, 0.107 mmol), MeCN (0.300 mL), a solution of H-MeAla-Aze-EtPhe(4-Me)-Sar-OtBu in MeCN (concentration 200.0 mg/mL, 0.200 mL, 40.0 mg, 0.080 mmol), and DIPEA (27.7 μL, 0.159 mmol) were sequentially added, and the mixture was cooled to 10°C while stirring. HATU (60.5 mg, 0.159 mmol) was added to the reaction mixture, and the mixture was stirred for 8 hours.

Measurement method: HPLC Method A
Retention time: H-MeAla-Aze-EtPhe(4-Me)-Sar-OtBu: 9.6 min, desired product: 18.6 min, epimer: 18.7 min
Mass spectrometry: H-MeAla-Aze-EtPhe(4-Me)-Sar-OtBu: m/z 503.81 ([M+H]$^+$), desired product: m/z 936.10 ([M+H]$^+$), epimer: m/z 936.39 ([M+H]$^+$)

[Table 32]

| Reaction conditions | Analysis results | |
|---|---|---|
| | Conversion (%) | Diastereomeric ratio |
| Conditions A: Two-layer conditions of organic solvent and water | 97.5 | 99.9 / 0.1 |
| Conditions B: Condensation conditions using HATU | 96.6 | 48.3 / 51.7 |

As shown in Table 32, it was found that both of the conversion and diastereomeric ratio were high under the two-layer conditions of organic solvent and water (conditions A), while the diastereomeric ratio was significantly low under the conditions using HATU as a condensation agent (conditions B).

(Example 33) Condensation reaction of Fmoc-MeAlGly-Val-OH and H-MeGly(nPr)-Ile-Pro-OtBu

The condensation reaction was carried out under the following conditions A and B.

[Formula 36]

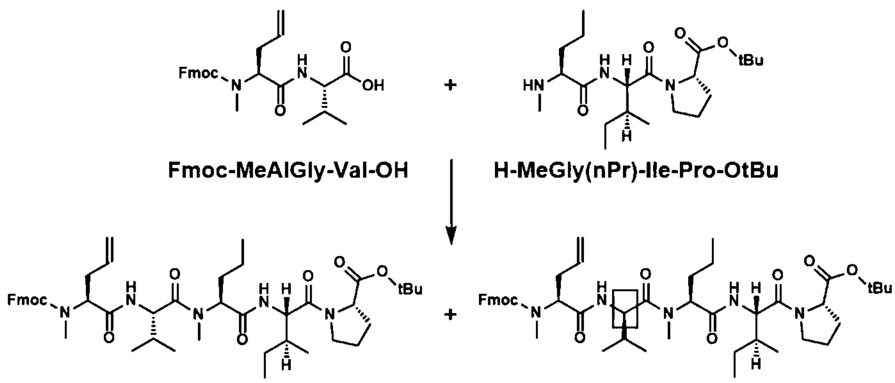

Fmoc-MeAlGly-Val-OH H-MeGly(nPr)-Ile-Pro-OtBu

desired product epimer

Conditions A

To a test tube with a stirrer, Fmoc-MeAlGly-Val-OH (52.6 mg, content 91.7%, net 48.4 mg, 0.107 mmol), IPAC (0.300 mL), and 2,6-lutidine (13.9 μL, 0.119 mmol) were sequentially added, and the mixture was cooled to -8°C while stirring. To the reaction mixture, PivCl (12.2 μL, 0.100 mmol) was added, and the mixture was stirred for 3 hours. To the reaction mixture, 10% aqueous $K_2CO_3$ solution (0.300 mL, 32.3 mg $K_2CO_3$) and a solution of H-MeGly(nPr)-Ile-Pro-OtBu in toluene (concentration 100.0 mg/mL, 0.316 mL, 31.6 mg, 0.080 mmol) were sequentially added. The temperature was then raised to 0°C, and the mixture was stirred continuously for 5 hours.

Conditions B

To a test tube with a stirrer, Fmoc-MeAlGly-Val-OH (52.6 mg, content 91.7%, net 48.4 mg, 0.107 mmol), H-MeGly(nPr)-Ile-Pro-OtBu (31.6 mg, 0.080 mmol), MeCN (0.500 mL), and DIPEA (27.7 μL, 0.159 mmol) were sequentially added, and the mixture was cooled to 10°C while stirring. HATU (60.5 mg, 0.159 mmol) was added to the reaction mixture, and the mixture was stirred for 8 hours.

Measurement method: HPLC Method A
Retention time: H-MeGly(nPr)-Ile-Pro-OtBu: 9.2 min, desired product: 19.5 min, epimer: 19.9 min
Mass spectrometry: H-MeGly(nPr)-Ile-Pro-OtBu: m/z 398.81 ($[M+H]^+$), desired product: m/z 831.51 ($[M+H]^+$), epimer: m/z 831.16 ($[M+H]^+$)

[Table 33]

| Reaction conditions | Analysis results | |
|---|---|---|
| | Conversion (%) | Diastereomeric ratio |
| Conditions A: Two-layer conditions of organic solvent and water | 99.2 | 100.0 / 0.0 |
| Conditions B: Condensation conditions using HATU | 94.7 | 37.7 / 62.3 |

As shown in Table 33, it was found that both of the conversion and diastereomeric ratio were high under the two-layer conditions of organic solvent and water (conditions A), while the diastereomeric ratio was significantly low under the conditions using HATU as a condensation agent (conditions B).

(Example 34) Condensation reaction of Cbz-MeLeu-Ile-OH and H-MeAla-Aze-EtPhe(4-Me)-Sar-OtBu

The condensation reaction was carried out in the following operation procedure.

[Formula 37]

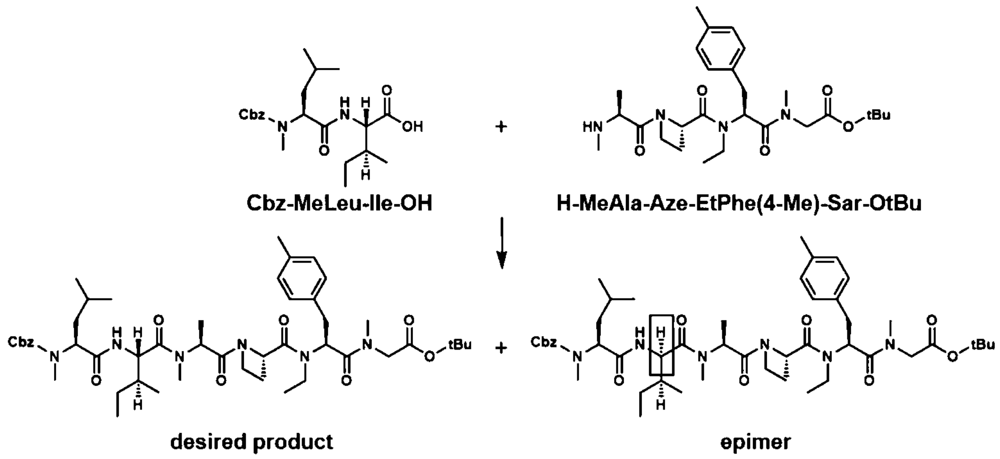

A solution of Cbz-MeLeu-Ile-OH in IPAC (concentration 157 mg/mL, 0.400 mL, 62.8 mg, 0.160 mmol) and 2,6-lutidine (20.8 μL, 0.179 mmol) were added to a test tube (hereinafter referred to as "test tube A") with a stirrer, and the reaction mixture was cooled to -10°C while stirring. To the reaction mixture, PivCl (18.4 μL, 0.149 mmol) was added, and the mixture was stirred for 3 hours. The progress of activation was confirmed by HPLC analysis. 10% aqueous $Na_2CO_3$ solution (0.380 mL) was added, and the reaction mixture was stirred at -5°C for 2 hours, and the temperature was raised to 0°C. A solution of H-MeAla-Aze-EtPhe(4-Me)-Sar-OtBu in toluene (concentration 200 mg/mL, 0.300 mL, 60.0 mg, 0.119 mmol) was added to test tube A over 1 hour and 30 minutes using a syringe pump. The reaction mixture was stirred at 0°C for 17 hours.

Reaction conversion: 98.8%

Diastereomeric ratio: 99.4/0.6
Piv-MeAla-Aze-EtPhe(4-Me)-Sar-OtBu: 0.9%
Measurement method: HPLC Method A
Retention time: H-MeAla-Aze-EtPhe(4-Me)-Sar-OtBu: 8.6 min, Piv-MeAla-Aze-EtPhe(4-Me)-Sar-OtBu: 13.7 min, desired product: 17.0 min, epimer: 17.3 min
Mass spectrometry: H-MeAla-Aze-EtPhe(4-Me)-Sar-OtBu: m/z 503.35 ([M+H]$^+$), Piv-MeAla-Aze-EtPhe(4-Me)-Sar-OtBu: m/z 587.45 ([M+H]$^+$), desired product: m/z 899.54 ([M+Na]$^+$), epimer: m/z 899.59 ([M+Na]$^+$)

(Synthesis of raw material 1) Synthesis of Cbz-MeLeu-Ile-OH

[Formula 38]

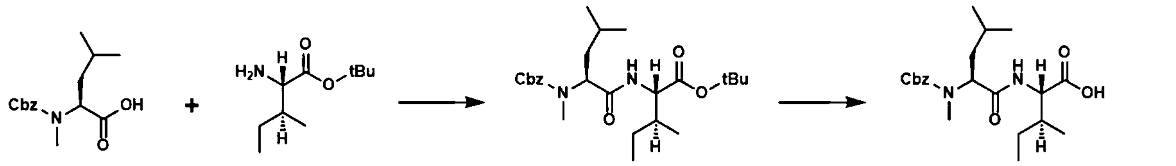

Cbz-MeLeu-OH H-Ile-OtBu Cbz-MeLeu-Ile-OtBu Cbz-MeLeu-Ile-OH

To a separatory funnel, H-Ile-OtBu·HCl (6.04 g, 27.0 mmol) and 2-MeTHF (120 mL) were added. The mixture was washed twice with 5% aqueous $Na_2CO_3$ solution (60 mL x 2), and the organic layer was concentrated under reduced pressure to obtain H-Ile-OtBu (4.8 g, 25.6 mmol). To the resulting concentrate, Cbz-MeLeu-OH (7.39 g, 26.4 mmol), MeCN (50 mL), and NMM (5.6 mL, 51.3 mmol) were added, and the reaction mixture was cooled while stirring. HATU (12.07 g, 31.74 mmol) was added to the reaction mixture while keeping the internal temperature of the reaction mixture at 17°C or lower, and the mixture was stirred continuously at room temperature. The stirring of the reaction solution was continued for 1 hour and 30 minutes, and the completion of the reaction was confirmed by HPLC analysis. To the reaction mixture, CPME (100 mL), 5% aqueous $K_2CO_3$ solution (50 mL), and NMI (2.0 mL, 25.6 mmol) were added, and the mixture was stirred continuously for 1 h. All the contents of the flask were transferred to a separatory funnel and the aqueous layer was removed. The organic layer was washed 3 times with 2.5% aqueous ammonia solution (50 mL x 3), 2 times with 5% aqueous $NaHSO_4$ solution (50 mL x 2), and 1 time with 5% aqueous $Na_2CO_3$ solution (50 mL x 1). The resulting organic layer was dried over anhydrous $Na_2SO_4$. The desiccant was filtered off and then the filtrate was concentrated under reduced pressure to obtain Cbz-MeLeu-Ile-OtBu (11.28 g, 25.14 mmol). To the obtained concentrate, 2-MeTHF (110 mL) and HMDS (21.0 mL, 100.30 mmol) were added, and TMSOTf (14.0 mL, 75.23 mmol) was added dropwise. The stirring of the reaction mixture was continued for 3 hours, and the completion of the reaction was confirmed by HPLC analysis. The flask was cooled, and then 5% aqueous $NaHCO_3$ solution (100 mL) was added dropwise to the reaction mixture while keeping the reaction mixture at 28°C or lower. All the contents of the flask were transferred to a separatory funnel and the organic layer was removed. To the resulting aqueous layer, 2-MeTHF (250 mL) was added, and 85% $H_3PO_4$ (10 mL) was added. The aqueous layer was removed, and the organic layer was washed with 5% aqueous NaCl solution (50 mL) and concentrated under reduced pressure. The resulting concentrate was purified by silica gel column chromatography to obtain Cbz-MeLeu-Ile-OH (8.20 g).

Yield: 77% (over 2 steps)
Purity: 100%
Measurement method: HPLC Method A, Retention time: 12.7 min
Mass spectrometry: m/z 393.55 ([M+H]$^+$)

(Synthesis of analytical preparation 1) Synthesis of Cbz-MeLeu-D-allo-Ile-OH

[Formula 39]

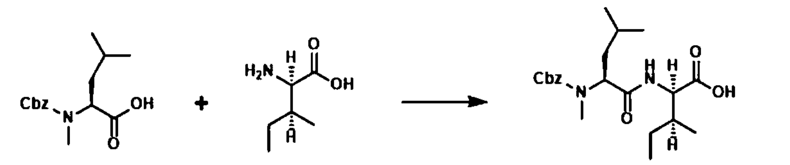

Cbz-MeLeu-OH H-D-allo-Ile-OH Cbz-MeLeu-D-allo-Ile-OH

To a 300 mL three-neck flask with a stirrer, Cbz-MeLeu-OH (1.00 g, 3.58 mmol) and THF (70 mL) were added, and the mixture was stirred and cooled to the internal temperature of 0°C. To the reaction mixture, TEA (1.50 mL, 10.74 mmol) and isobutyl chloroformate (0.44 mL, 3.40 mmol) were sequentially added, and the mixture was stirred for 30 minutes. An aqueous solution prepared by dissolving H-D-allo-Ile-OH (0.71 g, 5.37 mmol) in water (70 mL) was added dropwise, and the reaction mixture was stirred for 4 hours and 30 minutes. The reaction mixture was concentrated under reduced pressure, and the resulting concentrate was transferred to a separatory funnel with the addition of 2-MeTHF (150 mL) and 5% aqueous $NaHSO_4$ solution (50 mL). The aqueous layer was removed and the resulting organic layer was concentrated under reduced pressure conditions. The resulting concentrate was purified by silica gel column chromatography to obtain Cbz-MeLeu-D-allo-Ile-OH (0.71 g).

Yield: 51%
HPLC purity: 95.3%
Measurement method: HPLC Method A, Retention time: 13.1 min
Mass spectrometry: m/z 393.55 ($[M+H]^+$)

(Synthesis of raw material 2) Synthesis of Cbz-MeLeu-Ala-OH

[Formula 40]

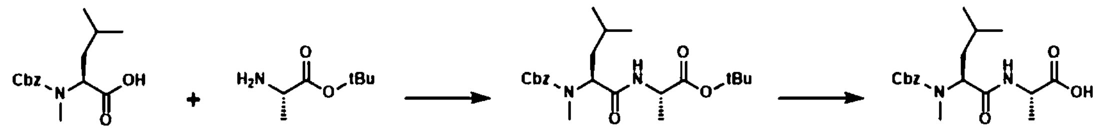

Cbz-MeLeu-OH H-Ala-OtBu Cbz-MeLeu-Ala-OtBu Cbz-MeLeu-Ala-OH

To a separatory funnel, H-Ala-OtBu·HCl (3.01 g, 16.57 mmol) and 2-MeTHF (60 mL) were added. The mixture was washed twice with 5% aqueous $Na_2CO_3$ solution (30 mL x 2), and the organic layer was concentrated under reduced pressure to obtain H-Ala-OtBu (1.22 g, 8.40 mmol). To the resulting concentrate, Cbz-MeLeu-OH (2.54 g, 9.09 mmol), MeCN (25 mL), and NMM (1.8 mL, 16.80 mmol) were added, and the mixture was cooled while stirring. HATU (3.89 g, 10.23 mmol) was added to the reaction mixture, and the mixture was stirred continuously at room temperature. The stirring was continued for 1 hour, and the completion of the reaction was confirmed by HPLC analysis. To the reaction mixture, CPME (60 mL), 5% aqueous $K_2CO_3$ solution (20 mL), and NMI (0.65 mL, 8.23 mmol) were added, and the mixture was stirred continuously for 1 hour. All the contents of the flask were transferred to a separatory funnel and the aqueous layer was removed. The organic layer was washed 3 times with 2.5% aqueous ammonia solution (25 mL x 3), 2 times with 5% aqueous $NaHSO_4$ solution (25 mL x 2), and 1 time with 5% aqueous $Na_2CO_3$ solution (5 mL x 1). The obtained organic layer was dried over anhydrous $Na_2SO_4$. The desiccant was filtered off and then the filtrate was concentrated under reduced pressure to obtain Cbz-MeLeu-Ala-OtBu (3.27 g, 8.04 mmol). To the obtained concentrate, 2-MeTHF (33 mL) and HMDS (5.1 mL, 24.1 mmol) were added, and TMSOTf (2.2 mL, 12.1 mmol) was added dropwise. The stirring was continued for 2 hours and 30 minutes, and the completion of the reaction was confirmed by HPLC analysis. The flask was cooled, and then 5% aqueous $NaHCO_3$ solution (35 mL) was added dropwise to the reaction mixture while keeping the reaction mixture at 19°C or lower. All the contents of the flask were transferred to a separatory funnel and the organic layer was removed. To the aqueous layer, 2-MeTHF (70 mL) was added, and 85% $H_3PO_4$ (5 mL) was added gradually. The aqueous layer was removed, and the organic layer was washed with 5% aqueous NaCl solution (25 mL) and concentrated under reduced pressure. The resulting concentrate was purified by silica gel column chromatography to obtain Cbz-MeLeu-Ala-OH (2.32 g).

Yield: 79% (over 2 steps)
Purity: 100%
Measurement method: HPLC Method A, Retention time: 11.2 min
Mass spectrometry: m/z 351.48 ([M+H]$^+$)

(Synthesis of raw material 3) Synthesis of Cbz-MeLeu-Phe-OH

[Formula 41]

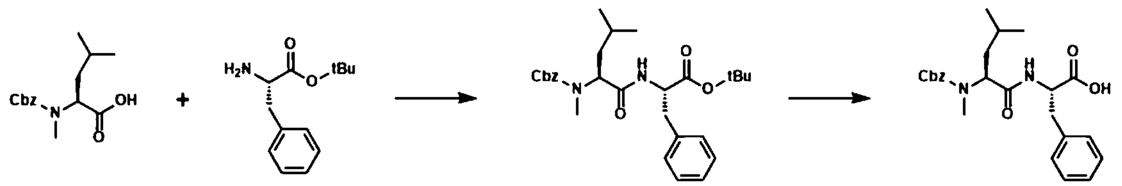

Cbz-MeLeu-OH H-Phe-OtBu Cbz-MeLeu-Phe-OtBu Cbz-MeLeu-Phe-OH

To a separatory funnel, H-Phe-OtBu·HCl (5.00 g, 19.40 mmol) and 2-MeTHF (100 mL) were added. The mixture was washed twice with 5% aqueous $Na_2CO_3$ solution (50 mL x 2), and the organic layer was concentrated under reduced pressure to obtain H-Phe-OtBu (4.09 g, 18.48 mmol). To the resulting concentrate, Cbz-MeLeu-OH (5.71 g, 20.33 mmol), MeCN (30 mL), and NMM (6.0 mL, 55.40 mmol) were added. HATU (9.14 g, 24.03 mmol) was added to the reaction mixture, and the mixture was stirred continuously at room temperature. The stirring was continued for 2 hours, and the completion of the reaction was confirmed by HPLC analysis. To the reaction mixture, CPME (80 mL), 5% aqueous $K_2CO_3$ solution (40 mL), and NMI (1.47 mL, 18.48 mmol) were added, and the mixture was stirred continuously for 30 minutes. All the contents of the flask were transferred to a separatory funnel and the aqueous layer was removed. The organic layer was washed 3 times with 3% aqueous ammonia solution (40 mL x 3), 2 times with 5% aqueous $NaHSO_4$ solution (40 mL x 2), and 1 time with 5% aqueous $Na_2CO_3$ solution (40 mL x 2). The obtained organic layer was dried over anhydrous $Na_2SO_4$. The desiccant was filtered off and then the filtrate was concentrated under reduced pressure to obtain Cbz-MeLeu-Phe-OtBu (9.10 g, 18.86 mmol). To the obtained concentrate, 2-MeTHF (91 mL) and HMDS (20.0 mL, 95.42 mmol) were added, and TMSOTf (14.0 mL, 77.35 mmol) was added dropwise. The stirring was continued for 1 hour and 30 minutes, and the completion of the reaction was confirmed by HPLC analysis. The flask was cooled, and then 5% aqueous $NaHCO_3$ solution (50 mL) was added dropwise to the reaction mixture while keeping the reaction mixture at 25°C or lower. All the contents of the flask were transferred to a separatory funnel, then 2-MeTHF (100 mL) was added, and 85% $H_3PO_4$ (6 mL) was added gradually. The aqueous layer was removed, and the organic layer was washed with 5% aqueous NaCl solution (50 mL) and concentrated under reduced pressure. The resulting concentrate was purified by silica gel column chromatography to obtain Cbz-MeLeu-Phe-OH (6.93 g).

Yield: 84% (over 2 steps)
Purity: 100%
Measurement method: HPLC Method A, Retention time: 13.1 min
Mass spectrometry: m/z 427.82 ([M+H]$^+$)

(Synthesis of raw material 4) Synthesis of Cbz-MeLeu-Ser(OtBu)-OH

[Formula 42]

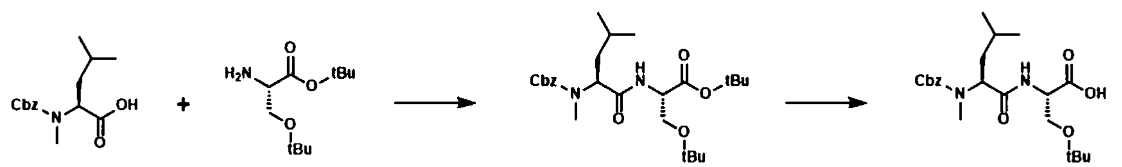

Cbz-MeLeu-OH H-Ser(OtBu)-OtBu Cbz-MeLeu-Ser(OtBu)-OtBu Cbz-MeLeu-Ser(OtBu)-OH

To a separatory funnel, H-Ser(OtBu)-OtBu·HCl (3.76 g, 14.82 mmol) and 2-MeTHF (100 mL) were added. The

mixture was washed twice with 5% aqueous $Na_2CO_3$ solution (50 mL x 2), and the organic layer was concentrated under reduced pressure to obtain H-Ser(OtBu)-OtBu (2.71 g, 12.47 mmol). To the resulting concentrate, Cbz-MeLeu-OH (3.66 g, 13.10 mmol), MeCN (30 mL), and NMM (4.0 mL, 36.38 mmol) were added, and the mixture was cooled while stirring. HATU (6.14 g, 16.15 mmol) was added to the reaction mixture while keeping the reaction mixture at 17°C or lower, and the mixture was stirred continuously at room temperature. The stirring was continued for 1 hour, and the completion of the reaction was confirmed by HPLC analysis. To the reaction mixture, CPME (100 mL), 5% aqueous $K_2CO_3$ solution (50 mL), and NMI (1.0 mL, 12.54 mmol) were added, and the mixture was stirred continuously for 2 hours. All the contents of the flask were transferred to a separatory funnel and the aqueous layer was removed. The organic layer was washed 3 times with 2.5% aqueous ammonia solution (50 mL x 3), 2 times with 5% aqueous $NaHSO_4$ solution (50 mL x 2), and 1 time with 5% aqueous $Na_2CO_3$ solution (50 mL x 1). The obtained organic layer was dried over anhydrous $Na_2SO_4$. The desiccant was filtered off and then the filtrate was concentrated under reduced pressure to obtain Cbz-MeLeu-Ser(OtBu)-OtBu (5.90 g, 12.54 mmol). To the obtained concentrate, 2-MeTHF (60 mL) and HMDS (5.3 mL, 25.07 mmol) were added, and TMSOTf (2.5 mL, 13.79 mmol) was added dropwise. The stirring was continued for 4 hours, and the completion of the reaction was confirmed by HPLC analysis. The flask was cooled, and then 5% aqueous $NaHCO_3$ solution (50 mL) was added dropwise to the reaction mixture while keeping the reaction mixture at 18°C or lower. All the contents of the flask were transferred to a separatory funnel, then 85% $H_3PO_4$ (6 mL) was added gradually. The aqueous layer was removed, and the organic layer was washed with 5% aqueous NaCl solution (50 mL) and the organic layer was concentrated under reduced pressure. The resulting concentrate was purified by silica gel column chromatography to obtain Cbz-MeLeu-Ser(OtBu)-OH (4.30 g).

Yield: 69% (over 2 steps)
Purity: 100%
Measurement method: HPLC Method A, Retention time: 12.8 min
Mass spectrometry: m/z 423.59 ($[M+H]^+$)

(Synthesis of analytical preparation 2) Synthesis of Cbz-MeLeu-D-Ser(OtBu)-OH

[Formula 43]

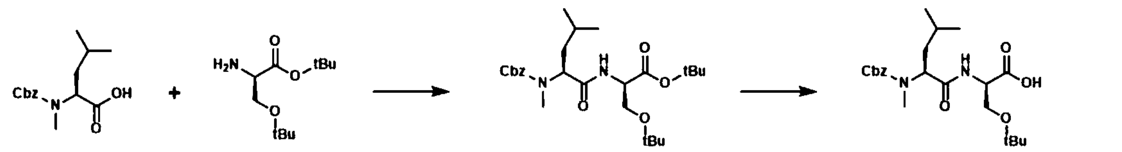

Cbz-MeLeu-OH H-D-Ser(OtBu)-OtBu Cbz-MeLeu-D-Ser(OtBu)-OtBu Cbz-MeLeu-D-Ser(OtBu)-OH

To a separatory funnel, H-D-Ser(OtBu)-OtBu·HCl (380 mg, 1.50 mmol) and 2-MeTHF (25 mL) were added. The mixture was washed twice with 5% aqueous $Na_2CO_3$ solution (10 mL x 2), and the organic layer was concentrated under reduced pressure to obtain H-D-Ser(OtBu)-OtBu (325 mg, 1.50 mmol). To the resulting concentrate, Cbz-MeLeu-OH (463 mg, 1.66 mmol), MeCN (5.0 mL), and NMM (0.33 mL, 2.99 mmol) were added. HATU (854 mg, 2.25 mmol) was added to the reaction mixture, and the mixture was stirred continuously at room temperature. The stirring was continued for 2 hours and 30 minutes, and the completion of the reaction was confirmed by HPLC analysis. To the reaction mixture, CPME (20 mL), 5% aqueous $K_2CO_3$ solution (10 mL), and NMI (120 μL, 1.50 mmol) were added, and the mixture was stirred continuously for 1 hour. All the contents of the flask were transferred to a separatory funnel, and then the aqueous layer was removed. The organic layer was washed 3 times with 2.5% aqueous ammonia solution (10 mL x 3), 2 times with 5% aqueous $NaHSO_4$ solution (10 mL x 2), and 1 time with 5% aqueous $Na_2CO_3$ solution (10 mL x 1). The obtained organic layer was dried over anhydrous $Na_2SO_4$. The desiccant was filtered off and then the filtrate was concentrated under reduced pressure to obtain Cbz-MeLeu-D-Ser(OtBu)-OtBu (0.690 g, 1.44 mmol). To the obtained concentrate, 2-MeTHF (7.0 mL) and HMDS (0.6 mL, 2.88 mmol) were added, and TMSOTf (300 μL, 1.66 mmol) was added dropwise. The stirring was continued for 4 hours, and the completion of the reaction was confirmed by HPLC analysis. The flask was cooled, and then 5% aqueous $NaHCO_3$ solution (6 mL) was added dropwise to the reaction mixture while keeping the reaction mixture at 14°C or lower. All the contents of the flask were transferred to a separatory funnel, and then 85% $H_3PO_4$ (5 mL) was added gradually. The aqueous layer was removed, and the organic layer was washed with 5% aqueous NaCl solution and concentrated under reduced pressure. The resulting concentrate was purified by silica gel column chromatography to obtain Cbz-MeLeu-D-Ser(OtBu)-OH (470 mg).

Yield: 74% (over 2 steps)

Purity: 100%
Measurement method: HPLC Method A, Retention time: 13.6 min
Mass spectrometry: m/z 423.59 ($[M+H]^+$)

(Synthesis of raw material 5) Synthesis of Cbz-MeLeu-Ile-MeLeu-Ile-OH

[Formula 44]

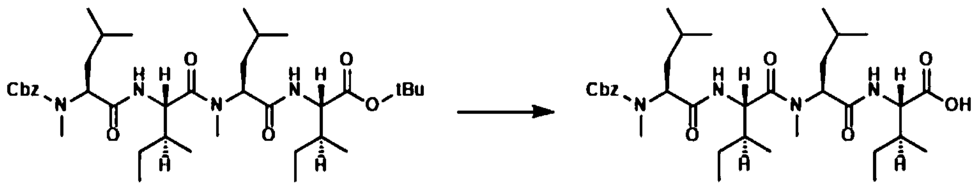

**Cbz-MeLeu-Ile-MeLeu-Ile-OtBu** **Cbz-MeLeu-Ile-MeLeu-Ile-OH**

To Cbz-MeLeu-Ile-MeLeu-Ile-OtBu (2.22 g, 3.22 mmol) synthesized by the method described in Example 8, 2-MeTHF (22.2 mL) and HMDS (3.38 mL, 16.11 mmol) were added. TMSOTf (2.33 mL, 12.89 mmol) was added dropwise to the reaction mixture. The mixture was stirred for 5 hours, then the completion of the reaction was confirmed by HPLC analysis. The flask was cooled, and then 5% aqueous $Na_2CO_3$ solution (20 mL) was added dropwise to the reaction mixture while keeping the reaction mixture at 53°C or lower. All the contents of the flask were transferred to a separatory funnel. The aqueous layer was discharged, and then the organic layer was washed once with 5% aqueous $Na_2CO_3$ solution (20 mL). The aqueous layers were combined, and then 2-MeTHF (30 mL) and 85% $H_3PO_4$ (2 mL) were added gradually. The aqueous layer was removed, and then the organic layers were combined and concentrated under reduced pressure. The resulting concentrate was purified by silica gel column chromatography to obtain Cbz-MeLeu-Ile-MeLeu-Ile-OH (708 mg).

Yield: 35%
HPLC purity: 99.6%
Measurement method: HPLC Method A, Retention time: 15.2 min
Mass spectrometry: m/z 655.52 ($[M+Na]^+$)

(Synthesis of raw material 6) Synthesis of H-MeAla-Aze-EtPhe(4-Me)-Sar-OtBu

[Formula 45]

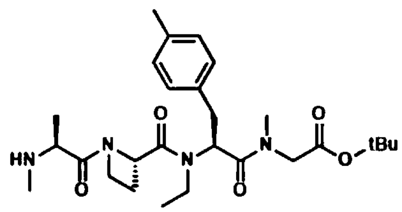

**H-MeAla-Aze-EtPhe(4-Me)-Sar-OtBu**

The compound was synthesized in accordance with the method described in International Publication No. WO 2023/127869.

(Synthesis of raw material 7) Synthesis of H-MeLeu-Ile-MeAla-Aze-EtPhe(4-Me)-Sar-OtBu

[Formula 46]

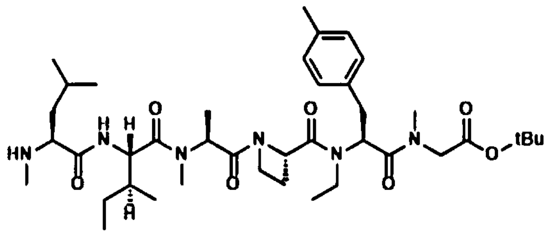

**H-MeLeu-Ile-MeAla-Aze-EtPhe(4-Me)-Sar-OtBu**

The compound was synthesized by desalting an L-tartrate salt of the title compound obtained by the method described in International Publication No. WO 2023/127869.

(Synthesis of raw material 8) Synthesis of H-MeGly(cPent)-MeAsp(OtBu)-$NMe_2$

[Formula 47]

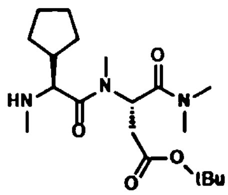

**H-MeGly(cPent)-MeAsp(OtBu)-$NMe_2$**

The compound was synthesized by desalting a hydrochloride salt of the title compound obtained by the method described in International Publication No. WO 2023/127869.

(Synthesis of raw material 9) Synthesis of H-MeLeu-Ile-OtBu

[Formula 48]

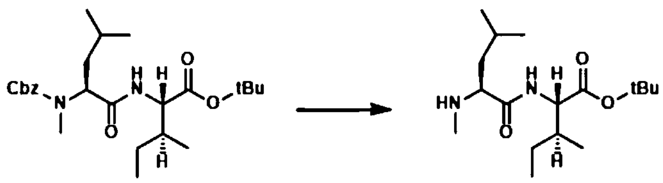

**Cbz-MeLeu-Ile-OtBu** **H-MeLeu-Ile-OtBu**

Cbz-MeLeu-Ile-OtBu (4.00 g, 8.92 mmol) synthesized by the method described in the synthesis of raw material 1 was dissolved in 2-MeTHF (24 mL) and added to a pressurized reaction vessel. 10% Pd/C (0.522 g, 0.223 mmol Pd metal basis) was added, and nitrogen replacement and hydrogen replacement were performed, and then the mixture was stirred under hydrogen pressure (0.3 atm) for 1 hour and 30 minutes. The reaction mixture was filtered and Pd/C was washed with 2-MeTHF (20 mL). The filtrate was concentrated under reduced pressure and the resulting concentrate was purified by silica gel column chromatography to obtain H-MeLeu-Ile-OtBu (1.66 g).

Yield: 59%
HPLC purity: 98.8%
Measurement method: HPLC Method A, Retention time: 8.0 min
Mass spectrometry: m/z 315.38 ($[M+H]^+$)

(Synthesis of raw material 10) Synthesis of H-cLeu-MeGly(cPent)-MeAsp(OtBu)-$NMe_2$

[Formula 49]

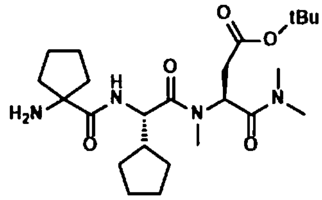

**H-cLeu-MeGly(cPent)-MeAsp(OtBu)-$NMe_2$**

The compound was synthesized in accordance with the method described in International Publication No. WO 2023/127869.

(Synthesis of raw material 11) Synthesis of Cbz-Leu-Ile-OH

[Formula 50]

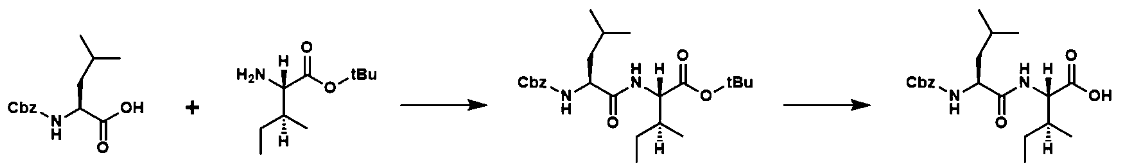

**Cbz-Leu-OH** **H-Ile-OtBu** **Cbz-Leu-Ile-OtBu** **Cbz-Leu-Ile-OH**

To a separatory funnel, H-Ile-OtBu·HCl (2.00 g, 8.94 mmol) and 2-MeTHF (50 mL) were added. The mixture was washed 2 times with 5% aqueous $Na_2CO_3$ solution (25 mL x 2), and the organic layer was concentrated under reduced pressure to obtain H-Ile-OtBu (1.58 g, 8.44 mmol). To the resulting concentrate, Cbz-Leu-OH (2.48 g, 9.35 mmol), MeCN (16 mL), and NMM (1.9 mL, 16.87 mmol) were added, and the reaction mixture was cooled while stirring. HATU (3.87 g, 10.18 mmol) was added to the reaction mixture while keeping the internal temperature of the reaction mixture at 11°C or lower, and the mixture was stirred continuously at room temperature. The stirring of the reaction solution was continued for 2 hours, and the completion of the reaction was confirmed by HPLC analysis. To the reaction mixture, 2-MeTHF (25 mL), 5% aqueous $K_2CO_3$ solution (20 mL), and NMI (0.67 mL, 8.44 mmol) were added, and the mixture was stirred continuously for 2 hours. All the contents of the flask were transferred to a separatory funnel, and the aqueous layer was removed. The organic layer was washed 3 times with 2.5% aqueous ammonia solution (25 mL x 3), 2 times with 5% aqueous $NaHSO_4$ solution (25 mL x 2), and 1 time with 5% aqueous $Na_2CO_3$ solution (25 mL x 1). The resulting organic layer was dried over anhydrous $Na_2SO_4$. The desiccant was filtered off and then the filtrate was concentrated under reduced pressure to obtain Cbz-Leu-Ile-OtBu (3.55 g, 8.17 mmol). To the obtained concentrate, 2-MeTHF (36 mL) and HMDS (7.0 mL, 32.7 mmol) were added, and TMSOTf (4.4 mL, 24.5 mmol) was added dropwise. The stirring of the reaction mixture was continued for 2 hours and 30 minutes, and the completion of the reaction was confirmed by HPLC analysis. The flask was cooled, and then 5% aqueous $NaHCO_3$ solution (36 mL) was added dropwise to the reaction mixture while keeping the reaction mixture at 25°C or lower. All the contents of the flask were transferred to a separatory funnel, and the organic layer was removed. To the resulting aqueous layer, 2-MeTHF (100 mL) was added, and 85% $H_3PO_4$ (4.5 mL) was added. The aqueous layer was removed, and the organic layer was washed with 5% aqueous NaCl solution (36 mL) and concentrated under reduced pressure. The resulting concentrate was purified by silica gel column chromatography to obtain Cbz-Leu-Ile-OH (2.00 g).

Yield: 59% (over 2 steps)
Purity: 98.6%
Measurement method: HPLC Method A, Retention time: 11.8 min
Mass spectrometry: m/z 379.77 ($[M+H]^+$)

(Synthesis of raw material 12) Synthesis of Cbz-Leu-D-allo-Ile-OH

[Formula 51]

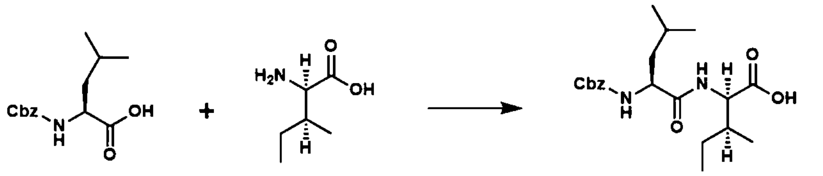

Cbz-Leu-OH H-D-allo-Ile-OH Cbz-Leu-D-allo-Ile-OH

To a 300 mL three-neck flask with a stirrer, Cbz-Leu-OH (1.50 g, 5.65 mmol) and THF (105 mL) were added, and the mixture was cooled to the internal temperature of -3°C while stirring. To the reaction mixture, TEA (2.4 mL, 17.0 mmol) and isobutyl chloroformate (0.70 mL, 5.37 mmol) were sequentially added, and the mixture was stirred for 30 minutes. An aqueous solution prepared by dissolving H-D-allo-Ile-OH (1.11 g, 8.48 mmol) in water (105 mL) was added dropwise, and the reaction mixture was stirred for 2 hours. The reaction mixture was concentrated under reduced pressure, and the resulting concentrate was transferred to a separatory funnel with the addition of 2-MeTHF (225 mL) and 5% aqueous $NaHSO_4$ solution (75 mL). The aqueous layer was removed and the resulting organic layer was concentrated under reduced pressure. The resulting concentrate was purified by silica gel column chromatography to obtain Cbz-Leu-D-allo-Ile-OH (0.645 g).

Yield: 30%
HPLC purity: 100.0%
Measurement method: HPLC Method A, Retention time: 12.2 min
Mass spectrometry: m/z 379.72 ($[M+H]^+$)

(Synthesis of raw material 13) Synthesis of Cbz-D-Leu-Ile-OH

[Formula 52]

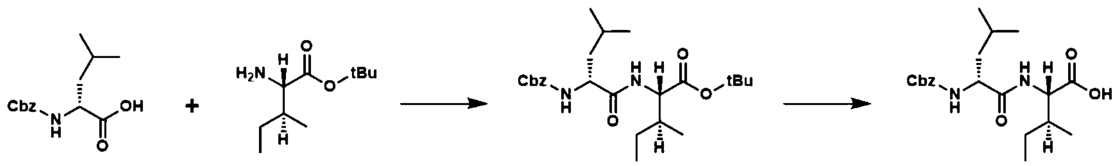

Cbz-D-Leu-OH H-Ile-OtBu Cbz-D-Leu-Ile-OtBu Cbz-D-Leu-Ile-OH

To a separatory funnel, H-Ile-OtBu·HCl (0.65 g, 2.91 mmol) and 2-MeTHF (50 mL) were added. The mixture was washed 2 times with 5% aqueous $Na_2CO_3$ solution (12 mL x 2), and the organic layer was concentrated under reduced pressure to obtain H-Ile-OtBu (0.55 g, 2.90 mmol). To the resulting concentrate, Cbz-D-Leu-OH (0.86 g, 3.2 mmol), MeCN (5.5 mL), and NMM (0.65 mL, 5.9 mmol) were added, and the reaction mixture was cooled while stirring. COMU (1.5 g, 3.5 mmol) was added to the reaction mixture while keeping the internal temperature of the reaction mixture at 11°C or lower, and the mixture was stirred continuously at room temperature. The stirring of the reaction solution was continued for 2 hours, and the completion of the reaction was confirmed by HPLC analysis. To the reaction mixture, 2-MeTHF (10 mL), 5% aqueous $K_2CO_3$ solution (10 mL), and NMI (0.23 mL, 2.9 mmol) were added, and the mixture was stirred continuously for 1 hour. All the contents of the flask were transferred to a separatory funnel, and the aqueous layer was removed. The organic layer was washed 1 time with 5% aqueous $Na_2CO_3$ solution (12 mL x 1), 3 times with 2.5% aqueous ammonia solution (12 mL x 3), 2 times with 5% aqueous $NaHSO_4$ solution (12 mL x 2), and 1 time with 5% aqueous $Na_2CO_3$ solution (12 mL x 1). The resulting organic layer was dried over anhydrous $Na_2SO_4$. The desiccant was filtered off and then the filtrate was concentrated under reduced pressure to obtain Cbz-D-Leu-Ile-OtBu (1.17 g, 2.69 mmol). To the obtained concentrate, 2-MeTHF (12 mL) and HMDS (2.3 mL, 10.75 mmol) were added, and TMSOTf (1.5 mL, 8.06 mmol) was added dropwise. The stirring of the reaction mixture was continued for 3 hours, and the completion of the reaction was confirmed by HPLC analysis. The flask was cooled, and then 5% aqueous $NaHCO_3$ solution (12 mL) was added dropwise to the reaction

mixture while keeping the reaction mixture at 24°C or lower. All the contents of the flask were transferred to a separatory funnel, and the organic layer was removed. To the resulting aqueous layer, 2-MeTHF (20 mL) was added, and 85% $H_3PO_4$ (1.3 mL) was added. The aqueous layer was removed, and the organic layer was washed with 5% aqueous NaCl solution (12 mL) and concentrated under reduced pressure. The resulting concentrate was purified by silica gel column chromatography to obtain Cbz-D-Leu-Ile-OH (0.57 g, 1.51 mmol).

Yield: 52% (over 2 steps)
Purity: 99.3%
Measurement method: HPLC Method A, Retention time: 12.0 min
Mass spectrometry: m/z 379.66 ($[M+H]^+$)

(Synthesis of raw material 14) Synthesis of Cbz-MeLeu-Thr(OtBu)-OH

[Formula 53]

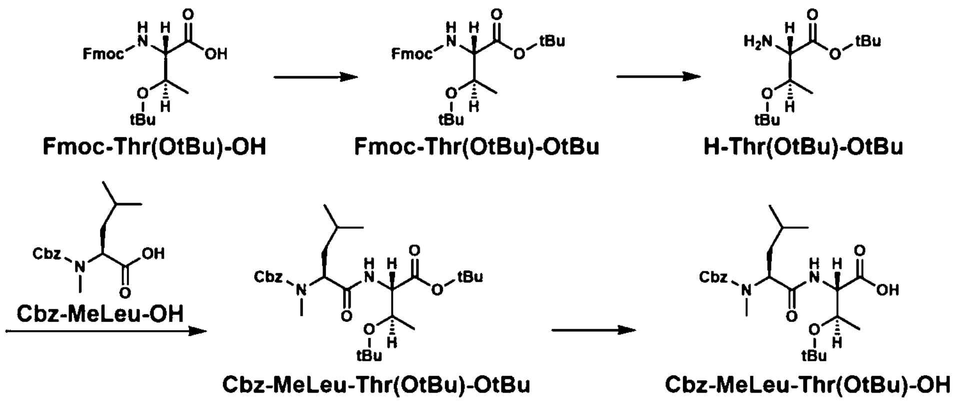

Fmoc-Thr(OtBu)-OH
Fmoc-Thr(OtBu)-OtBu
H-Thr(OtBu)-OtBu
Cbz-MeLeu-OH
Cbz-MeLeu-Thr(OtBu)-OtBu
Cbz-MeLeu-Thr(OtBu)-OH

To a flask with a stirrer, Fmoc-Thr(OtBu)-OH (2.00 g, 5.03 mmol), cyclohexane (16 mL), and DCM (6 mL) were added. To the flask, tert-butyl 2,2,2-trichloroacetimidate (1.8 mL, 10.10 mmol) was added. While cooling the flask in an ice bath, a boron trifluoride diethyl ether complex (0.06 mL, 0.50 mmol) was added dropwise, and then the mixture was stirred continuously at room temperature for 1 hour and 15 minutes. The completion of the reaction was confirmed by HPLC analysis, the reaction mixture was filtered, and the filtered solid was washed with cyclohexane. The filtrate and the washing solution were combined and washed 5 times with a 10% aqueous citric acid solution (16 mL x 5). The resulting organic layer was washed 2 times with 5% aqueous $Na_2CO_3$ solution (16 mL x 2). The resulting organic layer was concentrated under reduced pressure to obtain Fmoc-Thr(OtBu)-OtBu. To the flask, MeCN (18 mL) and DBU (0.75 mL, 5.03 mmol) were added, and the mixture was stirred for 30 minutes. The completion of the reaction was confirmed by HPLC analysis, and TEA (2.8 mL, 20.10 mmol) and water (0.91 mL, 50.3 mmol) were added to the reaction mixture. While cooling the flask in an ice bath, sodium hydrogen sulfite (1.31 g, 12.60 mmol) was added, and the mixture was stirred at room temperature for 1 hour. To the reaction mixture, MTBE (57 mL) and 10% aqueous ammonia solution (32 mL) were added, and all the contents of the flask were transferred to a separatory funnel. The organic layer obtained by removing the aqueous layer was washed 3 times with 10% aqueous ammonia solution (32 mL x 3) and 1 time with 5% aqueous NaCl solution (32 mL x 1). The resulting organic layer was concentrated under reduced pressure to obtain H-Thr(OtBu)-OtBu. To the flask, MeCN (14 mL), Cbz-MeLeu-OH (1.54 g, 5.53 mmol), and NMM (1.7 mL, 15.1 mmol) were added. While cooling the flask in an ice bath, COMU (2.80 g, 6.53 mmol) was added to the reaction mixture, and the mixture was stirred continuously at room temperature for 2 hours. The completion of the reaction was confirmed by HPLC analysis, and then 2-MeTHF (34 mL), 5% aqueous $K_2CO_3$ solution (17 mL), and NMI (0.40 mL, 5.03 mmol) were added to the reaction mixture, and the mixture was stirred continuously for 30 minutes. All the contents of the flask were transferred to a separatory funnel, and the aqueous layer was removed. The resulting organic layer was washed 1 time with 5% aqueous $K_2CO_3$ solution (27 mL x 1), 3 times with 2.5% aqueous ammonia solution (27 mL x 3), 2 times with 5% aqueous $NaHSO_4$ solution (27 mL x 2), 1 time with 5% aqueous $K_2CO_3$ solution (27 mL x 1), and 1 time with 5% aqueous NaCl solution (27 mL x 1). The resulting organic layer was dried over anhydrous $Na_2SO_4$. The desiccant was filtered off and then the filtrate was concentrated under reduced pressure to obtain Cbz-MeLeu-Thr(OtBu)-OtBu (2.35 g, 4.76 mmol). To the flask, 2-MeTHF (23 mL) and HMDS (4.0 mL, 19.00 mmol) were added, and then TMSOTf (2.6 mL, 14.20 mmol) was added dropwise. After continuously stirring for 4 hours, the flask was cooled, and then 5% aqueous $NaHCO_3$ solution (29 mL) was added dropwise to the

reaction mixture while keeping the reaction mixture at 24°C or lower. All the contents of the flask were transferred to a separatory funnel, and the organic layer was removed. To the resulting aqueous layer, 2-MeTHF (56 mL) was added, and 85% $H_3PO_4$ (3.5 mL) was added gradually. The aqueous layer was removed, and the resulting organic layer was washed with 5% aqueous NaCl solution (29 mL) and concentrated under reduced pressure. The resulting concentrate was purified by silica gel column chromatography to obtain Cbz-MeLeu-Thr(OtBu)-OH (0.53 g, 1.20 mmol).

Yield: 24% (over 4 steps starting from Fmoc-Thr(OtBu)-OH)
Purity: 99.5%
Measurement method: HPLC Method A, Retention time: 14.7 min
Mass spectrometry: m/z 437.74 ($[M+H]^+$)

(Synthesis of raw material 15) Synthesis of Cbz-MeLeu-Hph(3,5-$F_2$-4-$CF_3$)-OH

[Formula 54]

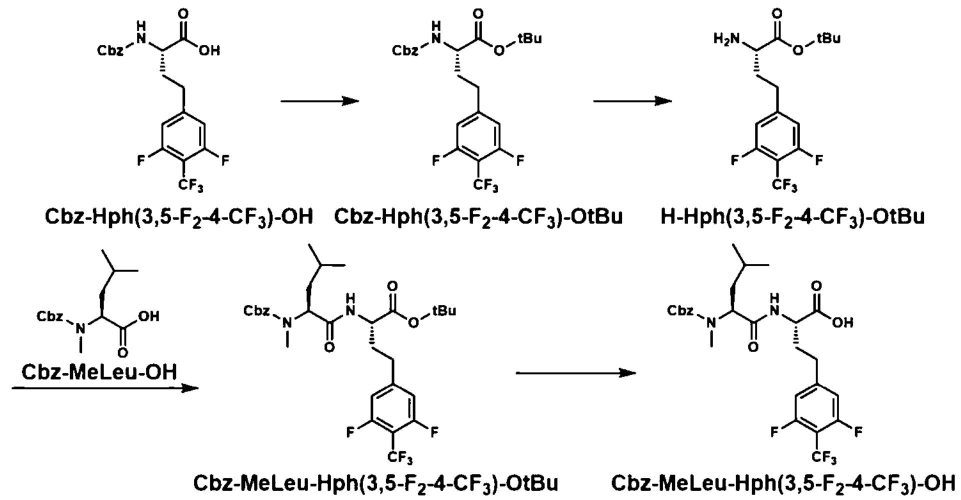


Cbz-Hph(3,5-$F_2$-4-$CF_3$)-OH Cbz-Hph(3,5-$F_2$-4-$CF_3$)-OtBu H-Hph(3,5-$F_2$-4-$CF_3$)-OtBu

Cbz-MeLeu-Hph(3,5-$F_2$-4-$CF_3$)-OtBu Cbz-MeLeu-Hph(3,5-$F_2$-4-$CF_3$)-OH

To a separatory funnel, Cbz-Hph(3,5-$F_2$-4-$CF_3$)-OH·$Cy_2NH$ (5.00 g, 8.35 mmol) and 2-MeTHF (75 mL) were added. The mixture was washed 2 times with 5% aqueous $H_2SO_4$ solution (25 mL x 2), and the organic layer was concentrated under reduced pressure to obtain Cbz-Hph(3,5-$F_2$-4-$CF_3$)-OH. To the obtained concentrate, cyclohexane (40 mL) and DCM (15 mL) were added. To the flask, tert-butyl 2,2,2-trichloroacetimidate (3.0 mL, 16.7 mmol) was added. While cooling the flask in an ice bath, a boron trifluoride diethyl ether complex (0.11 mL, 0.84 mmol) was added dropwise, and then the mixture was stirred continuously at room temperature for 2.5 hours. The completion of the reaction was confirmed by HPLC analysis, the reaction mixture was filtered, and the filtered solid was washed with cyclohexane. The filtrate and the washing solution were combined and washed 5 times with 10% aqueous citric acid solution (20 mL x 5). The resulting organic layer was washed 2 times with 5% aqueous $Na_2CO_3$ solution (20 mL x 2). The resulting organic layer was concentrated under reduced pressure to obtain Cbz-Hph(3,5-$F_2$-4-$CF_3$)-OtBu (3.93 g, 8.30 mmol). To the flask, 5% Pd/C (50% wet, 1.77 g, 0.42 mmol on Pd metal basis) was added, and nitrogen replacement and hydrogen replacement were performed, and then the mixture was stirred under hydrogen pressure (1 atm) for 2 hours and 30 minutes. The reaction mixture was filtered and Pd/C was washed with 2-MeTHF. The filtrate and the washing solution were combined and concentrated under reduced pressure to obtain H-Hph(3,5-$F_2$-4-$CF_3$)-OtBu (2.60 g, 7.66 mmol). To the resulting concentrate, Cbz-MeLeu-OH (2.35 g, 8.43 mmol), MeCN (26 mL), and NMM (1.7 mL, 15.3 mmol) were added, and the reaction mixture was cooled while stirring. COMU (3.94 g, 9.20 mmol) was added to the reaction mixture while keeping the internal temperature of the reaction mixture at 7°C or lower, and the mixture was stirred continuously at room temperature. The stirring of the reaction solution was continued for 2 hours, and the completion of the reaction was confirmed by HPLC analysis. To the reaction mixture, 2-MeTHF (40 mL), 5% aqueous $K_2CO_3$ solution (20 mL), and NMI (0.61 mL, 7.66 mmol) were added, and the mixture was stirred continuously for 30 minutes. All the contents of the flask were transferred to a separatory funnel, and the aqueous layer was removed. The resulting organic layer was washed 1 time with 5% aqueous $Na_2CO_3$ solution (20 mL x 1), 3 times with 2.5% aqueous ammonia solution (20 mL x 3), 2 times with 5% aqueous $NaHSO_4$ solution (20 mL x 2), and 1 time with 5% aqueous $Na_2CO_3$ solution (20 mL x 1). The resulting organic layer was dried over anhydrous $Na_2SO_4$. The desiccant was filtered off and then the filtrate was concentrated under

reduced pressure to obtain Cbz-MeLeu-Hph(3,5-$F_2$-4-$CF_3$)-OtBu (4.26 g, 7.09 mmol). To the flask, 2-MeTHF (44 mL) and HMDS (6.0 mL, 28.4 mmol) were added, and then TMSOTf (3.8 mL, 21.3 mmol) was added dropwise. After continuously stirring for 2 hours, the flask was cooled, and then 5% aqueous $NaHCO_3$ solution (40 mL) was added dropwise to the reaction mixture while keeping the reaction mixture at 22°C or lower. All the contents of the flask were transferred to a separatory funnel, and the aqueous layer was removed. The resulting organic layer was washed with 5% aqueous $NaHSO_4$ solution (40 mL x 1), and the aqueous layer was removed. The resulting organic layer was washed with 5% aqueous NaCl solution (30 mL) and concentrated under reduced pressure. The resulting concentrate was purified by silica gel column chromatography to obtain Cbz-MeLeu-Hph(3,5-$F_2$-4-$CF_3$)-OH (2.64 g, 4.85 mmol).

Yield: 58% (over 4 steps starting from Cbz-Hph(3,5-$F_2$-4-$CF_3$)-OH·$Cy_2$NH
Purity: 100.0%
Measurement method: HPLC Method A, Retention time: 15.2 min
Mass spectrometry: m/z 545.70 ($[M+H]^+$)

(Synthesis of raw material 16) Synthesis of Cbz-MeLeu-Cys(SBn)-OH

[Formula 55]

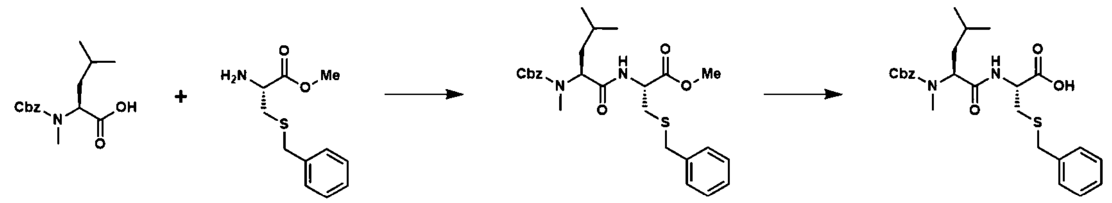

Cbz-MeLeu-OH H-Cys(SBn)-OMe Cbz-MeLeu-Cys(SBn)-OMe Cbz-MeLeu-Cys(SBn)-OH

To a separatory funnel, H-Cys(SBn)-OMe·HCl (1.97 g, 7.53 mmol) and 2-MeTHF (100 mL) were added. The mixture was washed 2 times with 5% aqueous $Na_2CO_3$ solution (40 mL x 2), and the organic layer was concentrated under reduced pressure to obtain H-Cys(SBn)-OMe (1.49 g, 6.61 mmol). To the resulting concentrate, Cbz-MeLeu-OH (2.04 g, 7.27 mmol), MeCN (15 mL), and NMM (1.5 mL, 13.2 mmol) were added, and the reaction mixture was cooled while stirring. COMU (3.40 g, 7.94 mmol) was added to the reaction mixture while keeping the internal temperature of the reaction mixture at 11°C or lower, and the mixture was stirred continuously at room temperature. The stirring of the reaction solution was continued for 2 hours, and the completion of the reaction was confirmed by HPLC analysis. To the reaction mixture, 2-MeTHF (45 mL), 5% aqueous $K_2CO_3$ solution (23 mL), and NMI (0.52 mL, 6.61 mmol) were added, and the mixture was stirred continuously for 1 hour. All the contents of the flask were transferred to a separatory funnel, and the aqueous layer was removed. The organic layer was washed 1 time with 5% aqueous $K_2CO_3$ solution (38 mL x 1), 3 times with 2.5% aqueous ammonia solution (38 mL x 3), 2 times with 5% aqueous $NaHSO_4$ solution (38 mL x 2), 1 time with 5% aqueous $K_2CO_3$ solution (38 mL x 1), and 1 time with 5% aqueous NaCl solution (38 mL x 1). The resulting organic layer was dried over anhydrous $Na_2SO_4$. The desiccant was filtered off and then the filtrate was concentrated under reduced pressure to obtain Cbz-MeLeu-Cys(SBn)-OMe (3.30 g, 6.78 mmol). To the resulting concentrate, THF (30 mL) was added, and the resulting solution was cooled in an ice bath. 2M aqueous NaOH solution (5.0 mL) was added dropwise while keeping the internal temperature of the mixture at 2°C or lower. The stirring of the reaction mixture was continued for 2 hours, and the completion of the reaction was confirmed by HPLC analysis. The contents of the flask were concentrated under reduced pressure, and 2-MeTHF (60 mL) was added to the concentrate. To the resulting solution, 5% aqueous NaHSO4 solution (30 mL) was added, and the mixture was transferred to a separatory funnel, and the aqueous layer was removed. The resulting organic layer was washed with 5% aqueous NaCl solution (45 mL) and concentrated under reduced pressure. The resulting concentrate was purified by silica gel column chromatography to obtain Cbz-MeLeu-Cys(SBn)-OH (0.693 g, 1.467 mmol).

Yield: 22% (over 2 steps)
Purity: 99.2%
Measurement method: HPLC Method A, Retention time: 15.5 min
Mass spectrometry: m/z 473.71 ($[M+H]^+$)

(Synthesis of raw material 17) Synthesis of Cbz-MeAla-Ile-OH

[Formula 56]

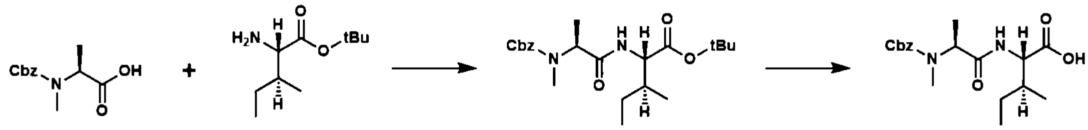

Cbz-MeAla-OH H-Ile-OtBu Cbz-MeAla-Ile-OtBu Cbz-MeAla-Ile-OH

To a separatory funnel, H-Ile-OtBu·HCl (5.05 g, 22.6 mmol) and 2-MeTHF (250 mL) were added. The mixture was washed 2 times with 5% aqueous $Na_2CO_3$ solution (100 mL x 2), and the organic layer was concentrated under reduced pressure to obtain H-Ile-OtBu (4.13 g, 22.1 mmol). To the resulting concentrate, Cbz-MeAla-OH (5.76 g, 24.3 mmol), MeCN (41 mL), and NMM (4.8 mL, 44.1 mmol) were added, and the reaction mixture was cooled while stirring. COMU (11.3 g, 26.5 mmol) was added to the reaction mixture while keeping the internal temperature of the reaction mixture at 11°C or lower, and the mixture was stirred continuously at room temperature. The stirring of the reaction solution was continued for 3 hours, and the completion of the reaction was confirmed by HPLC analysis. To the reaction mixture, 2-MeTHF (83 mL), 5% aqueous $K_2CO_3$ solution (83 mL), and NMI (1.7 mL, 22.1 mmol) were added, and the mixture was stirred continuously for 30 minutes. All the contents of the flask were transferred to a separatory funnel, and the aqueous layer was removed. The organic layer was washed 1 time with 5% aqueous $K_2CO_3$ solution (83 mL x 1), 3 times with 2.5% aqueous ammonia solution (83 mL x 3), 2 times with 5% aqueous $NaHSO_4$ solution (83 mL x 2), and 1 time with 5% aqueous $K_2CO_3$ solution (83 mL x 1). The resulting organic layer was dried over anhydrous $Na_2SO_4$. The desiccant was filtered off and then the filtrate was concentrated under reduced pressure to obtain Cbz-MeAla-Ile-OtBu (8.82 g, 21.7 mmol). The resulting concentrate was dissolved in 2-MeTHF (80 mL), and the resulting solution was divided into 3 solutions. To one of the 3 divided solutions, HMDS (6.0 mL, 28.9 mmol) was added, and TMSOTf (4.0 mL, 21.7 mmol) was added dropwise. The stirring of the reaction mixture was continued for 2 hours, and the completion of the reaction was confirmed by HPLC analysis. The flask was cooled, and then 5% aqueous $NaHCO_3$ solution (36 mL) was added dropwise to the reaction mixture while keeping the reaction mixture at 24°C or lower. All the contents of the flask were transferred to a separatory funnel, and the organic layer was removed. To the resulting aqueous layer, 2-MeTHF (72 mL) was added, and 85% $H_3PO_4$ (5.2 mL) was added. The aqueous layer was removed, and the organic layer was washed with 5% aqueous NaCl solution (36 mL) and concentrated under reduced pressure. The resulting concentrate was purified by silica gel column chromatography to obtain Cbz-MeAla-Ile-OH (2.20 g, 6.28 mmol).

Yield: 85% (over 2 steps)
Purity: 99.8%
Measurement method: HPLC Method A, Retention time: 10.7 min
Mass spectrometry: m/z 351.73 ($[M+H]^+$)

(Synthesis of raw material 18) Synthesis of Cbz-MeLeu-MeAla-Ile-OH

[Formula 57]

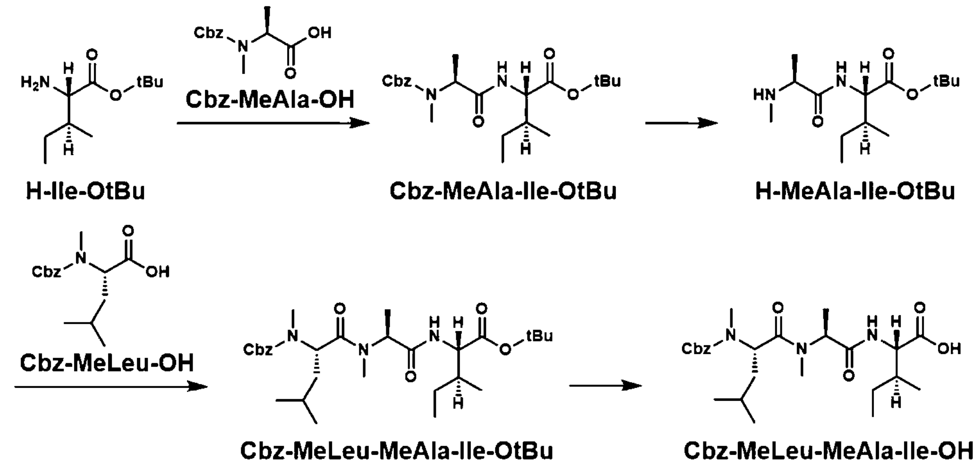


To one of the 3 divided solutions of Cbz-MeAla-Ile-OtBu in 2-MeTHF in the Synthesis of raw material 17, 5% Pd/C (50% wet, 1.53 g, 0.36 mmol on Pd metal basis) was added, and nitrogen replacement and hydrogen replacement were performed, and then the mixture was stirred under hydrogen atmosphere (1 atm) for 2 hours. The reaction mixture was filtered and Pd/C was washed with 2-MeTHF. The filtrate and the washing solution were combined and concentrated under reduced pressure to obtain H-MeAla-Ile-OtBu (1.95 g, 7.17 mmol). To the resulting concentrate, Cbz-MeLeu-OH (2.20 g, 7.87 mmol), MeCN (20 mL), and NMM (1.6 mL, 14.3 mmol) were added, and the reaction mixture was cooled while stirring. COMU (3.69 g, 8.59 mmol) was added to the reaction mixture while keeping the internal temperature of the reaction mixture at 5°C or lower, and the mixture was stirred continuously at room temperature. The stirring of the reaction solution was continued for 1 hour and 30 minutes, and the completion of the reaction was confirmed by HPLC analysis. To the reaction mixture, 2.5% aqueous ammonia solution (60 mL), 2-MeTHF (60 mL), 5% aqueous $K_2CO_3$ solution (30 mL), and NMI (0.57 mL, 7.16 mmol) were added, and the mixture was stirred continuously for 30 minutes. All the contents of the flask were transferred to a separatory funnel, and the aqueous layer was removed. The organic layer was washed 1 time with 5% aqueous $K_2CO_3$ solution (50 mL x 1), 3 times with 2.5% aqueous ammonia solution (50 mL x 3), 2 times with 5% aqueous $NaHSO_4$ solution (50 mL x 2), 1 time with 5% aqueous $K_2CO_3$ solution (50 mL x 1), and 1 time with 5% aqueous NaCl solution (50 mL x 1). The resulting organic layer was dried over anhydrous $Na_2SO_4$. The desiccant was filtered off and then the filtrate was concentrated under reduced pressure, and the resulting concentrate was purified by silica gel chromatography to obtain Cbz-MeLeu-MeAla-Ile-OtBu (3.45 g, 6.46 mmol). To the obtained Cbz-MeLeu-MeAla-Ile-OtBu, MeTHF (35 mL) and HMDS (5.4 mL, 25.9 mmol) were added, and TMSOTf (3.6 mL, 19.4 mmol) was added dropwise. The stirring of the reaction mixture was continued for 3 hours and 30 minutes, and the completion of the reaction was confirmed by HPLC analysis. The flask was cooled, and then 5% aqueous $NaHCO_3$ solution (41 mL) was added dropwise to the reaction mixture while keeping the reaction mixture at 24°C or lower. All the contents of the flask were transferred to a separatory funnel, and the organic layer was removed. To the resulting aqueous layer, 2-MeTHF (83 mL) was added, and 85% $H_3PO_4$ (4.8 mL) was added. The aqueous layer was removed, and the organic layer was washed with 5% aqueous NaCl solution (41 mL) and concentrated under reduced pressure. The resulting concentrate was purified by silica gel column chromatography to obtain Cbz-MeLeu-MeAla-Ile-OH (1.79 g, 3.75 mmol).

Yield: 51% (over 4 steps)
Purity: 100.0%
Measurement method: HPLC Method A, Retention time: 14.03 min
Mass spectrometry: m/z 479.07 ($[M+H]^+$)

(Synthesis of raw material 19) Synthesis of Boc-MeLeu-Phe-OH

[Formula 58]

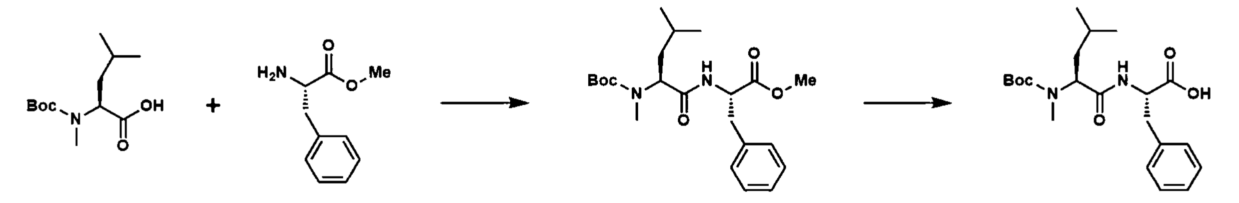

Boc-MeLeu-OH H-Phe-OMe Boc-MeLeu-Phe-OMe Boc-MeLeu-Phe-OH

To a flask, H-Phe-OMe·HCl (2.00 g, 9.27 mmol) and Boc-MeLeu-OH (2.50 g, 10.2 mmol), MeCN (20 mL), and NMM (4.1 mL, 37.1 mmol) were sequentially added, and the reaction mixture was cooled while stirring. COMU (4.77 g, 11.1 mmol) was added to the reaction mixture while keeping the internal temperature of the reaction mixture at 5°C or lower, and the mixture was stirred continuously at room temperature. The stirring of the reaction solution was continued for 2 hours, and the completion of the reaction was confirmed by HPLC analysis. To the reaction mixture, 2-MeTHF (60 mL), 5% aqueous $K_2CO_3$ solution (30 mL), and NMI (0.73 mL, 9.27 mmol) were added, and the mixture was stirred continuously for 1 hour. All the contents of the flask were transferred to a separatory funnel, and the aqueous layer was removed. The organic layer was washed 1 time with 5% aqueous $K_2CO_3$ solution (50 mL x 1), 3 times with 2.5% aqueous ammonia solution (50 mL x 3), 2 times with 5% aqueous $NaHSO_4$ solution (50 mL x 2), 1 time with 5% aqueous $K_2CO_3$ solution (50 mL x 1), and 1 time with 5% aqueous NaCl solution (50 mL). The resulting organic layer was dried over anhydrous $Na_2SO_4$. The desiccant was filtered off and then the filtrate was concentrated under reduced pressure to obtain Boc-MeLeu-Phe-OMe (3.93 g, 9.67 mmol). To the resulting concentrate, THF (38 mL) was added, and the resulting solution was cooled in an ice bath. 2M aqueous NaOH solution (7.0 mL) was added dropwise while keeping the internal temperature of the reaction mixture at 2°C or lower. The stirring of the reaction mixture was continued for 3 hours, and the completion of the reaction was confirmed by HPLC analysis. The contents of the flask were concentrated under reduced pressure, and 2-MeTHF (75 mL) was added to the concentrate. To the resulting solution, 5% aqueous NaHSO4 solution (38 mL) was added, and the mixture was transferred to a separatory funnel, and the aqueous layer was removed. The resulting organic layer was washed with 5% aqueous NaCl solution (57 mL) and concentrated under reduced pressure. The resulting concentrate was purified by silica gel column chromatography to obtain Boc-MeLeu-Phe-OH (3.13 g, 7.97 mmol).

Yield: 86% (over 2 steps)
Purity: 99.6%
Measurement method: HPLC Method A, Retention time: 14.5 min
Mass spectrometry: m/z 393.74 ($[M+H]^+$)

(Synthesis of raw material 20) Synthesis of Fmoc-MeAlGly-Val-OH

[Formula 59]

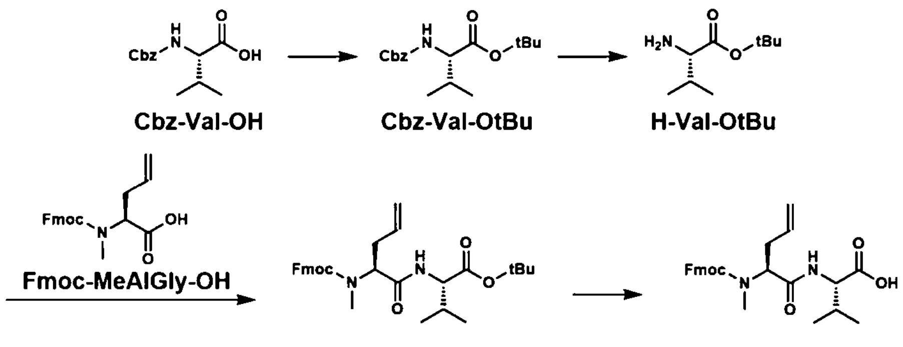

Cbz-Val-OH Cbz-Val-OtBu H-Val-OtBu

Fmoc-MeAlGly-OH

Fmoc-MeAlGly-Val-OtBu Fmoc-MeAlGly-Val-OH

To a flask with a stirrer, Cbz-Val-OH (3.02 g, 11.9 mmol), cyclohexane (24 mL), and DCM (9 mL) were added. To the flask, tert-butyl 2,2,2-trichloroacetimidate (4.3 mL, 23.9 mmol) was added. While cooling the flask in an ice bath, a boron trifluoride diethyl ether complex (0.15 mL, 1.2 mmol) was added dropwise, and then the mixture was stirred continuously at room temperature for 1 hour. The completion of the reaction was confirmed by HPLC analysis, the reaction mixture was filtered, and the filtered solid was washed with cyclohexane. The filtrate and the washing solution were combined and washed 5 times with 10% aqueous citric acid solution (24 mL x 5). The resulting organic layer was washed 2

times with 5% aqueous $Na_2CO_3$ solution (24 mL x 2). The resulting organic layer was concentrated under reduced pressure to obtain Cbz-Val-OtBu (3.67 g, 11.9 mmol). To the resulting concentrate, 2-MeTHF (37 mL) and 5% Pd/C (50% wet, 2.54 g, 0.60 mmol on Pd metal basis) were added, and nitrogen replacement and hydrogen replacement were performed, and then the mixture was stirred under hydrogen atmosphere (1 atm) for 2 hours. The reaction mixture was filtered and Pd/C was washed with 2-MeTHF. The filtrate and the washing solution were combined and concentrated under reduced pressure to obtain H-Val-OtBu (2.06 g, 11.9 mmol). To the flask, MeCN (21 mL), Fmoc-MeAlgly-OH (4.63 g, 13.1 mmol), and NMM (3.9 mL, 35.8 mmol) were added. While cooling the flask in an ice bath, COMU (6.14 g, 14.3 mmol) was added to the reaction mixture, and the mixture was stirred continuously at room temperature for 2 hours and 30 minutes. The completion of the reaction was confirmed by HPLC analysis, and then 2-MeTHF (60 mL) and 5% aqueous $K_2CO_3$ solution (30 mL) were added to the reaction mixture, and the mixture was stirred continuously for 30 minutes. All the contents of the flask were transferred to a separatory funnel, and the aqueous layer was removed. The resulting organic layer was washed 4 times with 5% aqueous $K_2CO_3$ solution (50 mL x 4), 2 times with 5% aqueous $NaHSO_4$ solution (50 mL x 2), 1 time with 5% aqueous $K_2CO_3$ solution (50 mL x 1), and 1 time with 5% aqueous NaCl solution (50 mL x 1). The resulting organic layer was dried over anhydrous $Na_2SO_4$. The desiccant was filtered off and then the filtrate was concentrated under reduced pressure to obtain Fmoc-MeAlGly-Val-OtBu (5.69 g, 11.2 mmol). To the flask, 2-MeTHF (57 mL) and HMDS (9.4 mL, 44.9 mmol) were added, and then TMSOTf (6.2 mL, 33.7 mmol) was added dropwise. After continuously stirring for 2 hours, the flask was cooled, and then 2-MeTHF (57 mL) was added while keeping the reaction mixture at 24°C or lower, and then 5% aqueous $NaHCO_3$ solution (68 mL) was added dropwise. All the contents of the flask were transferred to a separatory funnel, and the aqueous layer was separated. The resulting organic layer was washed with 5% aqueous $NaHCO_3$ solution (20 mL), and the aqueous layer was separated. The two aqueous layers were combined, and then 2-MeTHF (114 mL) was added, and 85% $H_3PO_4$ (8.2 mL) was gradually added. The aqueous layer was removed, and the resulting organic layer was washed with 5% aqueous NaCl solution (68 mL) and concentrated under reduced pressure. The resulting concentrate was purified by silica gel column chromatography to obtain Fmoc-MeAlGly-Val-OH (4.37 g, 9.70 mmol).

Yield: 82% (over 4 steps)
Purity: 99.6%
Measurement method: HPLC Method A, Retention time: 15.0 min
Mass spectrometry: m/z 451.76 ($[M+H]^+$)

(Synthesis of raw material 21) Synthesis of H-MeGly(nPr)-Ile-Pro-OtBu

[Formula 60]

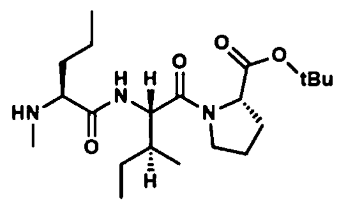

H-MeGly(nPr)-Ile-Pro-OtBu

Step 21-1

Compound 21-1: Synthesis of tert-butyl (2S)-1-[(2S,3S)-2-(benzyloxycarbonylamino)-3-methyl-pentanoyl]pyrrolidine-2-carboxylic acid

[Formula 61]

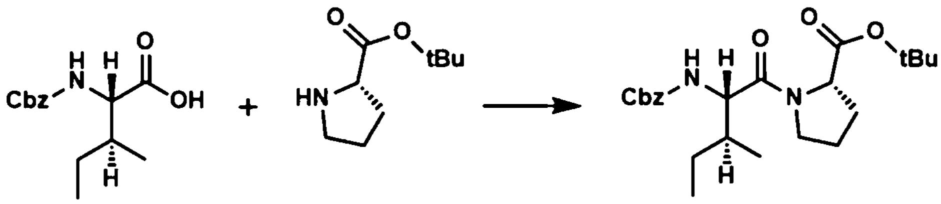

To a reactor after nitrogen replacement, tert-butyl (2S)-pyrrolidine-2-carboxylic acid (18.8 g), (2S,3S)-2-(benzyloxycarbonylamino)-3-methyl-pentanoic acid (20.0 g), and DMF (140 mL) were added at room temperature, and the mixture was stirred. After confirming complete dissolution, the mixture was cooled to 0°C and DIPEA (52.7 mL) was added. To the mixed solution, 50 wt.% solution of propylphosphonic acid anhydride in ethyl acetate (58.3 mL) was added at 0°C over 20 minutes, and the mixture was stirred at 0°C for 1.5 hours. To the mixed solution, water (100 mL) and ethyl acetate (200 mL) were added in this order. The aqueous layer 1 and the organic layer 1 were separated by liquid-liquid separation, and the aqueous layer 1 was taken out. The organic layer 1 was washed with 5% aqueous potassium hydrogen sulfate solution (100 mL), 5% aqueous sodium carbonate solution (100 mL) and 10% saline (100 mL). To the taken aqueous layer 1, water (100 mL) and ethyl acetate (200 mL) were added and the mixture was stirred, and then the organic layer 2 was obtained by liquid-liquid separation. The organic layer 1 and the organic layer 2 were combined and concentrated under reduced pressure to obtain compound 21-1 (33.8 g). LCMS(ESI) for Compound 21-1: Retention time: 2.75 min, m/z = 419 $[M+H]^+$

Step 21-2

Compound 21-2: Synthesis of tert-butyl (2S)-1-[(2S,3S)-2-amino-3-methyl-pentanoyllpyrrolidine-2-carboxylic acid

[Formula 62]

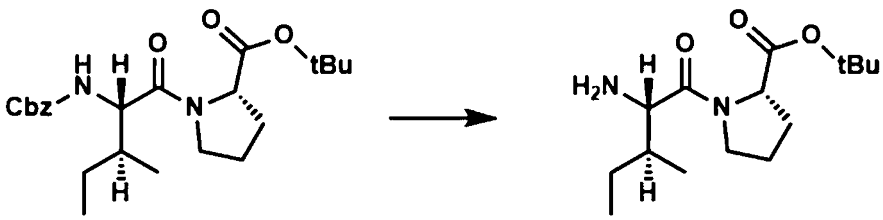

To a reactor after nitrogen replacement, compound 21-1 (31.6 g) obtained in Step 21-1 and 2-MeTHF (221 mL) were added, and the mixture was cooled to 10°C. After adding 5% Pd/C (6.32 g, 50% aqueous product) to the mixed solution, triethylsilane (60.3 mL) was added over 20 minutes. After stirring at the internal temperature of 15°C for 6 hours, the mixture was stirred at room temperature for an additional 17 hours. The mixed solution was filtered, and the filtrate was concentrated under reduced pressure to obtain compound 21-2. Compound 21-2 was used in step 21-3 without further purification.
LCMS(ESI) for Compound 21-2: Retention time: 1.14 min, m/z = 285 $[M+H]^+$

Step 21-3

Compound 21-3: Synthesis of tert-butyl (2S)-1-[(2S,3S)-2-[[(2S)-2-[9H-fluoren-9-ylmethoxycarbonyl(methyl)amino] pentanoyl]amino]-3-methyl-pentanoyl]pyrrolidine-2-carboxylic acid

[Formula 63]

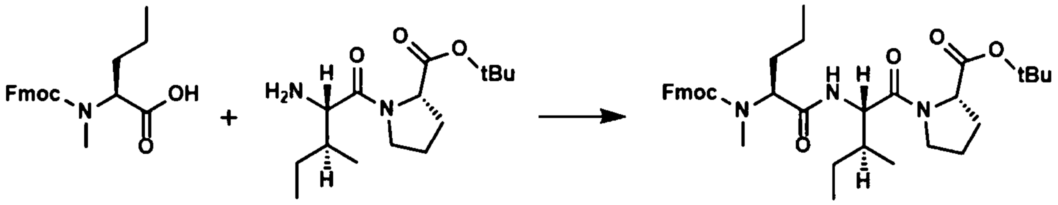

To compound 21-2 obtained in Step 21-2, acetonitrile (221 mL) was added at 5°C, and then (2S)-2-[9H-fluoren-9-ylmethoxycarbonyl(methyl)amino]pentanoic acid (28.0 g) and DIPEA (39.6 mL) were added. To the mixed solution, HATU (34.5 g) was slowly added at 2.5°C. After stirring at 2.5°C for 1 hour, the mixture was stirred at room temperature for 2.5 hours. To the mixed solution, a 5% aqueous sodium carbonate solution (189 mL) and water (150 mL) were added in this order. Toluene (80 mL) and 2-MeTHF (140 mL) were added, and the mixture was stirred, then liquid-liquid separation occurred. The organic layer was washed with 5% aqueous potassium hydrogen sulfate solution (190 mL x 2) and 10% saline (190 mL x 2). The obtained organic layer was concentrated under reduced pressure to obtain compound 21-3. Compound 21-3 was used in step 21-4 without further purification.
LCMS(ESI) for Compound 21-3: Retention time: 3.43 min, m/z = 620 $[M+H]^+$

Step 21-4

Compound 21-4: Synthesis of tert-butyl (2S)-1-[(2S,3S)-3-methyl-2-[[(2S)-2-(methylamino)pentanoyl]amino]pentanoyl] pyrrolidine-2-acetic acid (synthesis of H-MeGly(nPr)-Ile-Pro-OtBu)

[Formula 64]

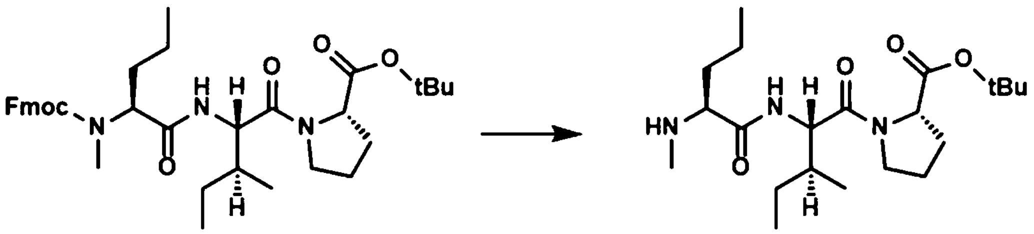

To a reactor after nitrogen replacement, compound 21-3 (296 mg) synthesized by the same method as in Step 21-3 and toluene (2.07 mL) were added, and the mixture was stirred. To the mixed solution, DBU (0.072 mL) was added, and the mixture was stirred for 30 minutes. After adding acetonitrile (1.00 mL) to the mixed solution, the mixture was stirred for 30 minutes, then DBU (0.072 mL) was added, and the mixture was stirred for another 30 minutes. 1N hydrochloric acid (2.00 mL) and n-heptane (1.00 mL) were added, and the mixture was stirred, then the aqueous layer 1 and the organic layer 1 were separated. The organic layer 1 was extracted with 1N hydrochloric acid (1.00 mL) to obtain an aqueous layer 2 containing compound 21-4. The aqueous layer 1 and the aqueous layer 2 were combined and extracted with 5% aqueous potassium carbonate solution (2.00 mL) and toluene (4.00 mL) to separate an organic layer containing compound 21-4. The obtained organic layer was washed with 10% saline (2.00 mL), then concentrated under reduced pressure to obtain compound 21-4 (166 mg).
LCMS(ESI) for Compound 21-4: Retention time: 1.36 min, m/z = 398 $[M+H]^+$

The analysis conditions of compounds 21-1 to 21-4 by LCMS were shown below.

Device: Waters UPLC/SQD
Column: Ascentis Express RP 90A amide, 2.1 mm ID x 50 mm, 2.7 $\mu$m
Mobile Phase: 0.1% FA/water (A), 0.1% FA/MeCN (B)
Elution method: B) 5% (0 min) $\rightarrow$ 100% (4.5 min) $\rightarrow$ 100% (5.0 min) $\rightarrow$ 5% (5.01 min) $\rightarrow$ 5% (7 min)
Flow rate: 0.5 mL/min
Column temperature: 40°C
Detection wavelength: 210-400 nm (PDA)

[Industrial Applicability]

According to the present invention, a peptide compound can be synthesized with high diastereoselectivity and in high yield even in the cases of peptide fragment coupling. Further, according to the present invention, a synthetic method suitable for fragment coupling a peptide compound containing an N-substituted amino acid and/or a peptide compound having a bulky side chain near the reaction point of an amide bond can be provided.

## Claims

1. A method for producing a peptide compound or a salt thereof, comprising a step (linking step) of linking an amino group of a first amino acid or peptide and a carboxy group of a second amino acid or peptide with an amide bond in a two-layer solvent system containing water and one or more organic solvents that are not miscible with water.

2. The method according to claim 1, wherein the linking step is carried out in the presence of an activator.

3. The method according to claim 1 or 2, wherein the activator is an acyl halide.

4. The method according to claim 1, wherein the linking step includes steps of:

   (1) bringing the second amino acid or peptide into contact with an acyl halide in an organic solvent to prepare a mixed acid anhydride; and
   (2) bringing the mixed acid anhydride obtained in step (1) into contact with the first amino acid or peptide in the presence of a base in a mixed solution containing water and one or more organic solvents that are not miscible with water.

5. The method according to any one of claims 1 to 4, wherein the second amino acid or peptide is a peptide containing two or more amino acid residues.

6. The method according to any one of claims 1 to 5, wherein an $\alpha$-position carbon of the carboxy group of the second amino acid or peptide is represented by formula: $-CR^1R^2-$, wherein $R^1$ and $R^2$ are identical or different and each is a hydrogen atom, a linear $C_1$-$C_6$ alkyl, an optionally substituted branched $C_3$-$C_6$ alkyl, an optionally substituted $C_3$-$C_8$ cycloalkyl-$C_1$-$C_6$ alkyl, an optionally substituted $C_3$-$C_8$ cycloalkyl, an optionally substituted phenyl-$C_1$-$C_2$ alkyl, an optionally substituted $C_1$-$C_6$ alkoxy-$C_1$-$C_2$ alkyl, an optionally substituted 5- to 6-membered heteroaryl-$C_1$-$C_2$ alkyl, or an optionally substituted phenyl-$C_1$-$C_2$ alkylthio-$C_1$-$C_2$ alkyl, or $R^1$ and $R^2$ together with a carbon atom to which they are attached form a $C_3$-$C_8$ saturated alicyclic ring.

7. The method according to any one of claims 1 to 6, wherein the first amino acid or peptide is a peptide containing two or more amino acid residues.

8. The method according to any one of claims 3 to 7, wherein the acyl halide is pivaloyl chloride or 2,2-dimethylbutyryl chloride.

9. The method according to any one of claims 4 to 8, wherein step (1) is carried out in the presence of a base.

10. The method according to claim 9, wherein the base used in step (1) is at least one selected from the group consisting of diisopropylethylamine, triethylamine, 2,6-lutidine, and 2,4,6-collidine.

11. The method according to any one of claims 1 to 10, wherein the amino group of the first amino acid or peptide is an amino group represented by formula: $-NR^4R^5$, wherein $R^4$ is a hydrogen atom, and $R^5$ is a hydrogen atom, a linear $C_1$-$C_6$ alkyl, a branched $C_3$-$C_6$ alkyl, or a $C_3$-$C_8$ cycloalkyl.

12. The method according to any one of claims 1 to 11, wherein the peptide compound to be produced or a salt thereof contains 8 to 20 amino acid residues.

13. The method according to any one of claims 1 to 12, wherein the peptide compound to be produced or a salt thereof contains a non-natural amino acid residue, and the non-natural amino acid is an N-methylamino acid residue.

14. The method according to any one of claims 1 to 13, wherein the peptide compound to be produced or a salt thereof contains a cyclic portion composed of 4 to 14 amino acid residues, and wherein an amide bond with which the amino group of the first amino acid or peptide and the carboxy group of the second amino acid or peptide are linked is contained in the cyclic portion at 1 to 7 locations.

15. A peptide compound or a salt thereof produced by the method according to any one of claims 1 to 14.

## INTERNATIONAL SEARCH REPORT

International application No.
**PCT/JP2024/034729**

**A. CLASSIFICATION OF SUBJECT MATTER**

***C07K 1/02***(2006.01)i; ***C07K 1/06***(2006.01)i; ***C07K 1/10***(2006.01)i
FI: C07K1/02; C07K1/06; C07K1/10

According to International Patent Classification (IPC) or to both national classification and IPC

**B. FIELDS SEARCHED**

Minimum documentation searched (classification system followed by classification symbols)

C07K1/02; C07K1/06; C07K1/10

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2021/132336 A1 (OTSUKA CHEMICAL CO., LTD.) 01 July 2021 (2021-07-01)<br>claims, paragraph [0049], examples | 1-7, 9-12, 14, 15 |
| Y | claims, paragraph [0049], examples | 8-15 |
| X | JP 2011-504175 A (SOLVAY SA) 03 February 2011 (2011-02-03)<br>claims | 15 |
| Y | paragraphs [0029], [0030], [0060], [0061], [0073] | 8-15 |
| X | WO 2022/234864 A1 (CHUGAI SEIYAKU KABUSHIKI KAISHA) 10 November 2022 (2022-11-10)<br>claims | 15 |
| Y | paragraph [0138] | 10-15 |
| X | NOZAKI, Sukekatsu et al., RAPID PEPTIDE SYNTHESIS IN LIQUID PHASE. PREPARATION OF ANGIOTENSIN II AS AN EXAMPLE, Chemistry Letters, 1977, pp. 1057-1058<br>table 1 | 1, 2, 6, 7, 11, 12, 15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 December 2024** | **17 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
**PCT/JP2024/034729**

**C. DOCUMENTS CONSIDERED TO BE RELEVANT**

| Category* | Citation of document, with indication, where appropriate, of the relevant passages | Relevant to claim No. |
|---|---|---|
| X | JP 1997-511743 A (MERCK & CO., INC.) 25 November 1997 (1997-11-25)<br>claims | 1, 2, 5-7, 9, 11, 15 |
| X | JP 1990-079993 A (FUAN SHIYUN MOU II U SHIEN KUN TSUU) 20 March 1990 (1990-03-20)<br>claims | 1, 6, 15 |

Form PCT/ISA/210 (second sheet) (July 2022)

**INTERNATIONAL SEARCH REPORT**
**Information on patent family members**

International application No.
**PCT/JP2024/034729**

| Patent document cited in search report | Publication date (day/month/year) | Patent family member(s) | Publication date (day/month/year) |
|---|---|---|---|
| WO 2021/132336 A1 | 01 July 2021 | US 2023/0037643 A1<br>claims, paragraph [0049], examples<br>EP 4095147 A1 | |
| JP 2011-504175 A | 03 February 2011 | US 2010/0280221 A1<br>paragraphs [0029], [0030], [0060], [0061], [0073]<br>WO 2009/065949 A2<br>EP 2062909 A1 | |
| WO 2022/234864 A1 | 10 November 2022 | US 2022/0411462 A1<br>paragraph [0304]<br>EP 4086272 A1 | |
| JP 1997-511743 A | 25 November 1997 | US 5502165 A1<br>claims<br>WO 1995/027727 A2<br>EP 755374 A1 | |
| JP 1990-079993 A | 20 March 1990 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013100132 A
- JP 2023168751 A
- WO 2018225851 A
- WO 2018225864 A
- WO 2019117274 A
- WO 2020111238 A
- WO 2020122182 A
- WO 2021075478 A
- WO 2021090856 A
- WO 2021132545 A
- WO 2021246471 A
- WO 2022097540 A
- WO 2022138891 A
- WO 2022145444 A
- WO 2022234864 A
- WO 2023127869 A
- WO 2023219152 A
- WO 2024096023 A
- WO 2024143514 A


### Non-patent literature cited in the description


- *Acc. Chem. Res.*, 2008, vol. 41, 1331-1342
- *Angew. Chem. Int. Ed.*, 2013, vol. 52, 254-269
- *Chem. Rev.*, 2019, vol. 119, 10360-10391
- *Amino Acids, Peptides and Proteins in Organic Chemistry: Building Blocks, Catalysis and Coupling Chemistry*, 2011, vol. 3
- *J. Org. Chem.*, 1995, vol. 60, 3569-3570
- *Org. Lett.*, 2020, vol. 22, 8039-8043
- *ACS Sustainable Chem. Eng.*, 2022, vol. 10, 5307-5314
- *J. Peptide Res.*, 2005, vol. 65, 153-166
- **R. SRIVASTAVA**. *J. Mol. Catal. A: Chem.*, 2007, vol. 264, 146-152
- **HALL, H.K., JR.** *J. A.m. Chem. Soc.*, 1957, vol. 79, 5441
- *Chemical and Pharmaceutical Bulletin*, 1995, vol. 43, 1872-1877
- **CLARKE, K** ; **ROTHWELL, K.** *J. Chem. Soc.*, 1960, 1885
- **CLARKE, K.** ; **ROTHWELL, K.** *J. Chem. Soc.*, 1960, 1885
- **D.H. RIPIN** ; **D.A. EVANS**. *pKa's of Nitrogen Acids*, 22 September 2023, http://evans.rc.fas.harvard.edu/pdf/evans_pKa_table.pdf
- Greene's Protective Groups in Organic Synthesis. 2014